# EUROPÄISCHE PATENTANMELDUNG

(11) **EP 0 260 551 A2**
(43) Veröffentlichungstag der Anmeldung: **23.03.1988**
(21) Anmeldenummer: 87112977.1
(22) Anmeldetag: 04.09.1987
(51) Int. Cl.: C07C 49/24, C07C 49/258, C07C 47/263, C07C 43/178, C07C 59/76, C07C 33/035, C07D 317/20, C07C 103/133, C07C 131/00, C07F 7/08

(54) **Isopren-Derivate**

(30) Priorität: 10.09.1986 CH 3649/86
(71) Anmelder: F. HOFFMANN-LA ROCHE & CO. Aktiengesellschaft, 4002 Basel (CH)
(72) Erfinder: Fischli, Albert, Prof. Dr., CH-4125 Riehen (CH); Schmid, Max, Dr., CH-4310 Rheinfelden (CH); Schmid, Rudolf, Dr., CH-4142 Münchenstein (CH)
(74) Vertreter: Lederer, Franz, Dr.

(57) **Zusammenfassung**

Verbindungen der Formel 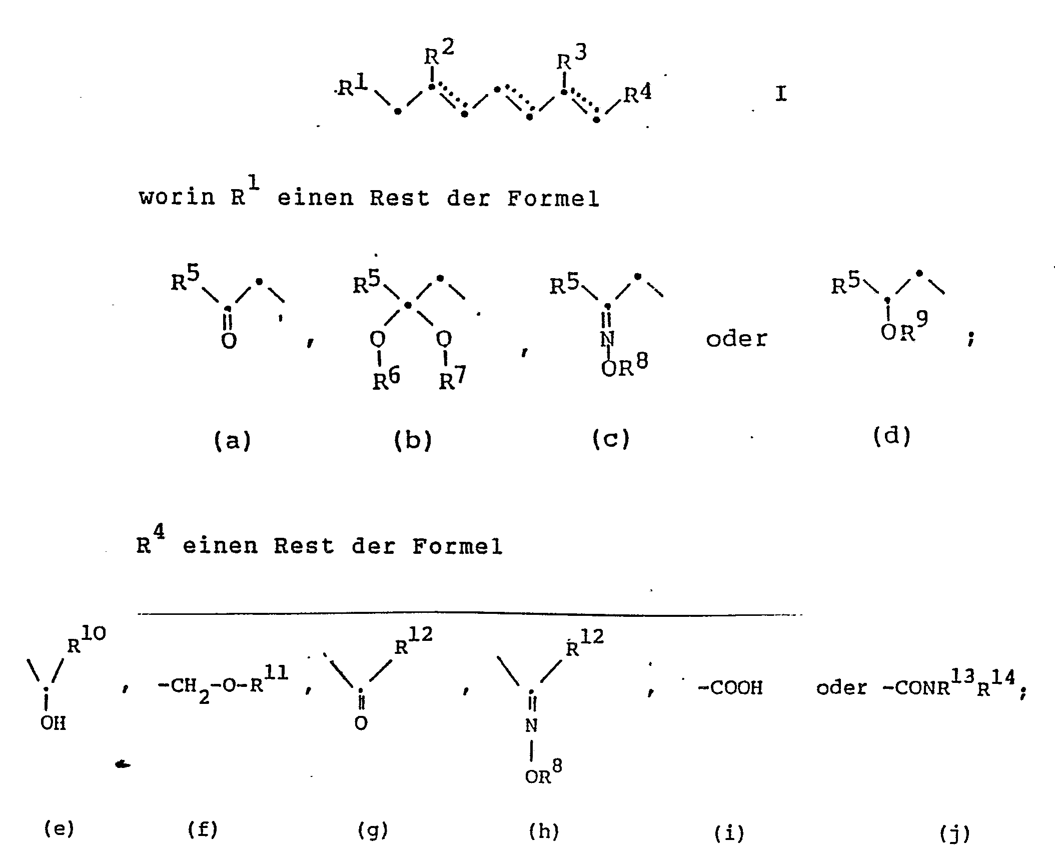 und die punktierten Linien fakultative zusätzliche C-C-Bindungen mit E- oder Z-Konfiguration bedeuten und die übrigen Symbole die in Anspruch 1 angegebene Bedeutung besitzen,
sowie pharmazeutisch verwendbare Salze davon haben mucosa-protektive und magensäuresekretionshemmende Eigenschaften und können zur Behandlung von Ulcus ventriculi und/oder duodeni verwendet werden. Sie sind neu, mit Ausnahme von 10-Hydroxy-3,7,11-trimethyl-2,6,11-dodecatriensäure und 3,7,11-Trimethyl-2,6,11-dodecatrien-1,10-diol. Sie können zu galenischen Darreichungsformen verarbeitet und nach verschiedenen Verfahren hergestellt werden.

## Beschreibung

Die vorliegende Erfindung betrifft Isopren-Derivate. Im speziellen betrifft sie Verbindungen der allgemeinen Formel 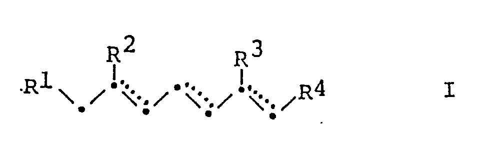 worin
- R¹ einen Rest der Formel 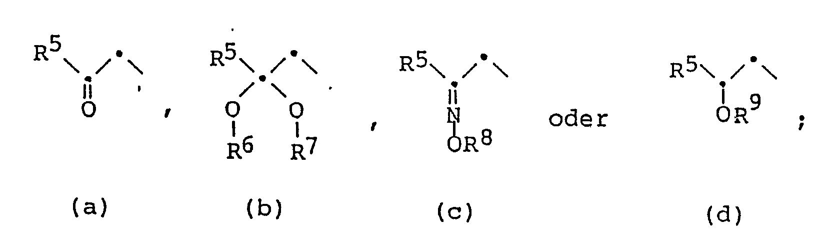 - eines von R² und R³ (C₁-C₈)-Alkyl und das andere Wasserstoff oder (C₁-C₈)-Alkyl;
- R⁴ einen Rest der Formel 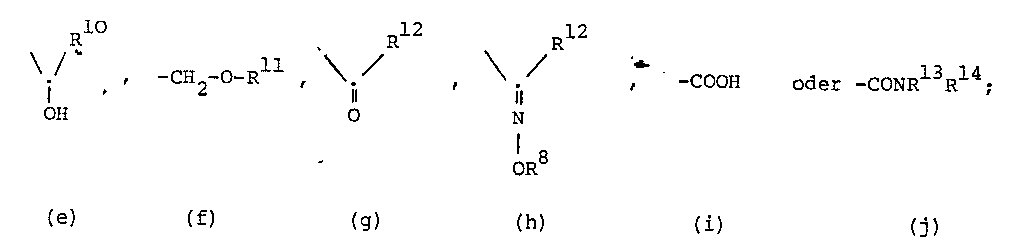 - R⁵ (C₂-C₈)-Alkyl, nicht jedoch Isopropyl, (C₃-C₈)-Cycloalkyl, (C₂-C₈)-Alkenyl, (C₂-C₈)-Alkinyl, Tri-(C₁-C₈)-alkyl-silyäthinyl oder, falls R¹ einen Rest der Formel (a) bedeutet, auch einen Rest der Formel 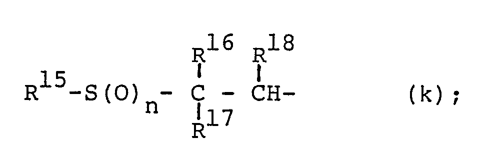 - R⁶ und R⁷ je (C₁-C₈)-Alkyl oder zusammen eine gegebenenfalls durch eine oder zwei (C₁-C₈)-Alkyl-,(C₁-C₈)-Alkoxycarbonylgruppen substituierte Di- oder Trimethylengruppe;
- R⁸ Wasserstoff oder (C₁-C₈)-Alkyl;
- R⁹ Wasserstoff oder (C₂-C₈)-Alkanoyl;
- R¹⁰ (C₁-C₈)-Alkyl;
- R¹¹ Wasserstoff, (C₁-C₈)-Alkyl, (C₂-C₈)-Alkanoyl oder einen Rest der Formel 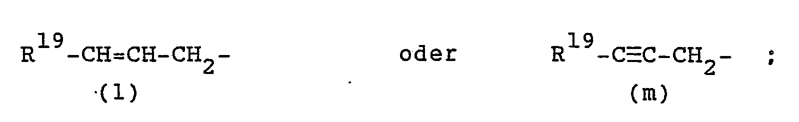 - R¹² Wasserstoff, (C₁-C₈)-Alkyl, (C₂-C₈)-Alkenyl oder (C₂-C₈)-Alkinyl;
- R¹³ und R¹⁴ je Wasserstoff oder (C₁-C₈)-Alkyl oder zusammen mit dem Stickstoffatom 1-Pyrrolidinyl, Piperidino oder Morpholino;
- R¹⁵ (C₁-C₈)-Alkyl, (C₂-C₈)-Alkanoyl, (C₁-C₈)-Alkoxycarbonyl-(C₁-C₈)alkyl, Hydroxy -(C₂-C₈)-alkyl, (C₂-C₈)-Alkanoyloxy-(C₂-C₈)-alkyl, (C₂-C₈)-Alkanoylamino-(C₂-C₈)-alkyl oder durch (C₂-C₈)-Alkanoylamino und (C₁-C₈)-Alkoxycarbonyl oder durch (C₂-C₈)-Alkanoyloxy und (C₁-C₈)-Alkoxycarbonyl disubsituiertes (C₂-C₈)-Alkyl;
- n die Zahl 0, 1 oder 2;
- R¹⁶, R¹⁷ und R¹⁸ je Wasserstoff oder (C₁-C₆)-Alkyl, enthaltend insgesamt höchstens 6 C-Atome;
- R¹⁹ Wasserstoff oder (C₁-C₅)-Alkyl;
- jede der punktierten Linien eine fakultative zusätzliche C-C-Bindung mit E- oder Z-Konfiguration bedeuten; und
- die in den Resten der Formeln (c) und (h) vorhandene Doppelbindung die E- oder Z-Konfiguration aufweist;
sowie pharmazeutisch verwendbare Salze von sauren Verbindungen der Formel I mit Basen.

Diese Verbindungen sind neu, mit Ausnahme von 10-Hydroxy-3,7,11-Trimethyl-2,6,11-dodecatriensäure und 3,7,11-Trimethyl-2,6,11-dodecatrien-1,10-diol, und es hat sich gezeigt, dass sie wertvolle pharmakodynamische Eigenschaften besitzen, nämlich magensäuresekretions-hemmende und/oder mukosa-protektive Eigenschaften, sodass sie zur Bekämpfung oder Verhütung von Krankheiten des Magen-Darm-Trakts verwendet werden können, insbesondere gegen Ulcus ventriculi und duodeni.

Gegenstand der vorliegenden Erfindung sind die eingangs definierten Verbindungen und Salze als therapeutische Wirkstoffe, Arzneimittel, enthaltend eine solche Verbindung oder ein Salz davon, die Herstellung solcher Arzneimittel, die Verwendung der eingangs definierten Verbindungen und Salze bei der Bekämpfung bzw. Verhütung von Krankheiten, insbesondere bei der Bekämpfung bzw. Verhütung von Ulcus ventriculi und duodeni bzw. die Verwendung der eingangs definierten Verbindungen und Salze zur Herstellung von Arzneimitteln für die Bekämpfung bzw. Verhütung von Ulcus ventriculi und duodeni, sowie die neuen unter den eingangs definierten Verbindungen und Salzen als solche und die Herstellung dieser neuen Verbindungen und Salze.

Der Ausdruck "Alkyl", für sich allein genommen oder in Kombinationen, wie "Alkoxyalkyl" oder "Hydroxyalkyl", bezeichnet geradkettige oder verzweigte gesättigte Kohlenwasserstoffreste, wie Methyl, Aethyl, n-Propyl, sec-Butyl, t-Butyl und dgl. Der Ausdruck "Alkoxy" bezeichnet über ein Sauerstoffatom gebundene Alkylgruppen im Sinne der vorstehenden Definition.

Der Ausdruck "Cycloalkyl" umfasst cycloaliphatische Reste, wie Cyclopropyl, Cyclobutyl, Cyclopentyl, Cyclohexyl und dgl. Die Ausdrücke "Alkenyl" und "Alkinyl" bezeichnen Kohlenwasserstoffreste, welche eine Kohlenstoff-Kohlenstoff-Doppel- oder -Dreifachbindung enthalten, wie z.B. Vinyl, 1-Methylvinyl, 1-Propenyl, 2-Methyl-1-propenyl, Aethinyl und dgl.

Der Ausdruck "Alkanoyl" bezeichnet Reste, wie Acetyl, Propionyl und dgl. Die Ausdrücke "Alkoxycarbonyl" und "Alkoxycarbonylalkyl" bezeichnen Reste wie Methoxycarbonyl bzw. Methoxycarbonylmethyl und dgl. Der Ausdruck "Hydroxyalkyl" bezeichnet Reste wie 2-Hydroxyäthyl. Die Ausdrücke "Alkanoyloxyalkyl" und "Alkanoylaminoalkyl" bezeichnen Reste wie Acetoxymethyl bzw. 2-Acetylaminoäthyl und dgl.

Unter den Verbindungen der eingangs definierten Formel I sind diejenigen bevorzugt, worin R¹ einen Rest der Formel (a), (b), (c) oder (d), R² und R³ je (C₁-C₈)-Alkyl, R⁴ einen Rest der Formel (f), (g), (h), (i) oder (j), R⁵ (C₂-C₈)-Alkenyl, (C₂-C₈)-Alkinyl oder Tri-(C₁-C₈)-alkyl-silyl-äthinyl, R⁶ und R⁷ je (C₁-C₈)-Alkyl oder zusammen die Dimethylengruppe, R⁸ Wasserstoff oder (C₁-C₈)-Alkyl, R⁹ Wasserstoff, R¹¹ Wasserstoff oder (C₂-C₈)-Alkanoyl, R¹², R¹³ und R¹⁴ je Wasserstoff und zwei der punktierten Linien nicht-konjugierte zusätzliche C-C-Bindungen bedeuten; speziell bevorzugt sind dabei diejenigen dieser Verbindungen, worin R² und R³ je Methyl, R⁵ Vinyl, 1-Methylvinyl, 1-Propenyl, 2-Methyl-1-propenyl, Aethinyl, 1-Propinyl oder Trimethylsilyläthinyl, R⁶ und R⁷ je Methyl oder Aethyl oder zusammen die Dimethylengruppe, R⁸ Wasserstoff oder Methyl und R¹¹ Wasserstoff oder Acetyl bedeuten, insbesondere diejenigen, worin R¹ einen Rest der Formel (a) und R⁴ einen Rest der Formel (f) bedeuten.

Ganz besonders bevorzugte Verbindungen der Formel I sind:
(all-E)-12-Hydroxy-6,10-dimethyl-6,10-dodecadien-1-in-3-on; (all-E)-12-Hydroxy-6,10-dimethyl-1,6,10-dodecatrien-3-on;
(all-E)-13-Hydroxy-7,11-dimethyl-7,11-tridecadien-2-in-4-on;
(all-E)-13-Hydroxy-7,11-dimethyl-2,7,11-tridecatrien-4-on;
und
(all-E)-10,10-Diäthoxy-3,7-dimethyl-2,6-dodecadien-11-in-1-ol.

Ebenfalls besonders bevorzugte Verbindungen der Formel I sind:
(all-E)-12-Hydroxy-2,6,10-trimethyl-1,6,10-dodecatrien-3-on;
(all-E)-10-Oxo-3,7,11-trimethyl-2,6,11-dodecatrienal; und
(6Z,10Z)-12-Hydroxy-2,6,10-trimethyl-1,6,10-dodecatrien-3-on.

Weitere bevorzugte Verbindungen der Formel I sind:
(all-E)-3,7-Dimethyl-9-[2-(1-methylvinyl)-1,3-dioxolan-2-yl]-2,6-nonadien-1-ol;
(E,E)-3,7,11-Trimethyl-10-oxo-2,6,11-dodecatrienamid;
(all-E)-13-Hydroxy-2,7,11-trimethyl-2,7,11-tridecatrien-4-on;
(6E,10Z)-12-Hydroxy-2,6,10-trimethyl-1,6,10-dodecatrien-3-on; und
(6Z,10E)-12-Hydroxy-2,6,10-trimethyl-1,6,10-dodecatrien-3-on.

Weitere repräsentative Verbindungen der Formel I sind:
(all-E)-10-Oxo-3,7,11-trimethyl-2,6,11-dodecatriensäure;
(all-E)-10,10-Dimethoxy-3,7,11-trimethyl-2,6,11-dodecatrien-1-ol;
(all-E)-10-Hydroxy-3,7,11-trimethyl-2,6,11-dodecatrienal;
(all-E)-12-Hydroxy-6,10-dimethyl-1-(trimethylsilyl)-6,10-dodecadien-1-in-3-on;
(2E,6E)-3,7,11-Trimethyl-10-oxo-2,6,11-dodecatrienal-(E/Z)-oxim;
(6E,10E)-12-Hydroxy-6,10-dimethyl-6,10-dodecadien-1-in-3-on (E/Z)-O-methyloxim; und
(2E,6E,11(E/Z))-3,7-Dimethyl-2,6,11-tridecatrien-1,10-diol.

Die neuen Verbindungen der eingangs definierten Formel I und deren Salze können erfindungsgemäss dadurch hergestellt werden, dass man
a) von einer Verbindung der allgemeinen Formel 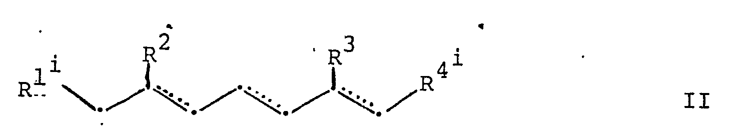 worin R¹ⁱ einen Rest der eingangs definierten Formel (a), (b) oder (c) oder einen Rest der Formel 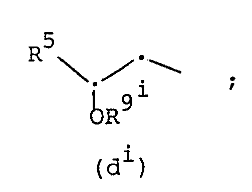 R⁴ⁱ einen Rest der eingangs definierten Formel (g), (h), (i) oder (j) oder einen Rest der Formel 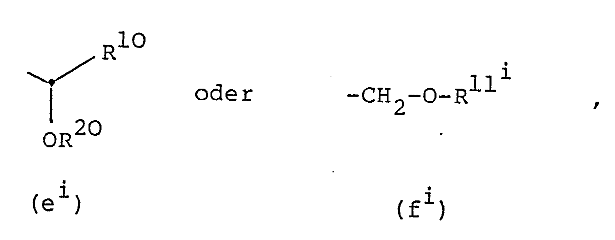 R⁹ⁱ Wasserstoff, (C₂-C₈)-Alkanoyl oder eine Schutzgruppe, R¹¹ⁱ Wasserstoff, (C₁-C₈)-Alkyl, (C₂-C₈)-Alkanoyl, eine Gruppe der eingangs definierten Formel (l) oder (m) oder eine Schutzgruppe und R²⁰ Wasserstoff oder eine Schutzgruppe bedeuten und R², R³, R⁵, R¹⁰ und die punktierten Linien die eingangs angegebene Bedeutung besitzen, wobei das Molekül mindestens eine Schutzgruppe enthält, die Schutzgruppe(n) abspaltet; oder
b) in einer Verbindung der allgemeinen Formel 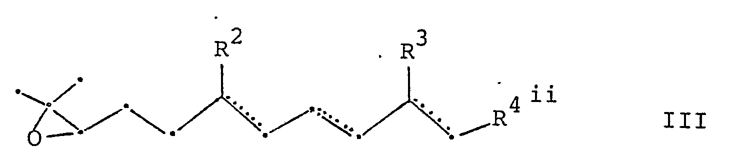 worin R⁴ⁱⁱ einen Rest der eingangs definierten Formeln (f), (h), (i) oder (j) bedeutet und R², R³ und die punktierten Linien die eingangs angegebene Bedeutung besitzen,
   den Epoxidring öffnet; oder
c) einen Carbonsäureester der allgemeinen Formel 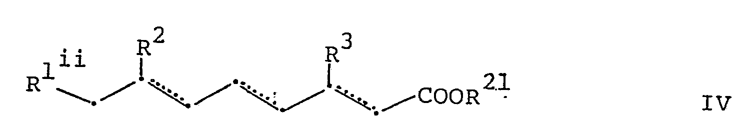 worin R¹ⁱⁱ einen Rest der eingangs definierten Formel (b) oder einen Rest der Formel 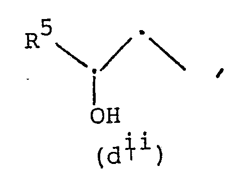 und R²¹ (C₁-C₈)-Alkyl bedeuten und R², R³, R⁵ und die punktierten Linien die eingangs angegebene Bedeutung besitzen,
   zum entsprechenden primären Alkohol reduziert; oder
d) einen Carbonsäureester der allgemeinen Formel 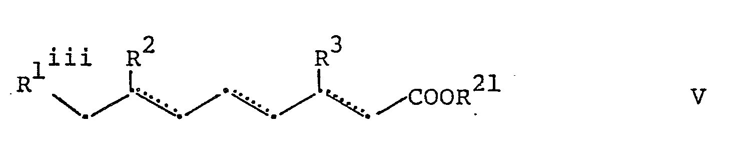 worin R¹ⁱⁱⁱ einen Rest der eingangs definierten Formel (b) oder (c) oder der obigen Formel (dⁱⁱ) oder der Formel 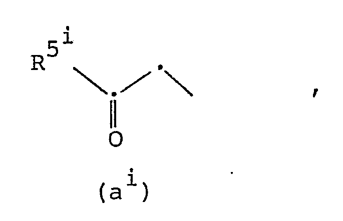 R⁵ⁱ (C₂-C₈)-Alkyl, nicht jedoch Isopropyl, (C₃-C₈)-Cycloalkyl, (C₂-C₈)-Alkenyl, (C₂-C₈)-Alkinyl oder Tri-(C₁-C₈)-alkyl-silyl-äthinyl bedeuten, R²¹ obige Bedeutung besitzt und R², R³ und die punktierten Linien die eingangs angegebene Bedeutung besitzen,
   zur entsprechenden Carbonsäure hydrolysiert; oder
e) in einer Verbindung der eingangs definierten Formel I, worin R¹ einen Rest der eingangs definierten Formel (b), (c) oder (d) oder einen Rest der obigen Formel (aⁱ) bedeutet, wobei das Molekül mindestens eine an ein C-Atom gebun dene Hydroxygruppe enthält, diese Hydroxygruppe(n) oder eine davon zu (einer) Oxo-gruppe(n) oxydiert; oder
f) eine Verbindung der eingangs definierten allgemeinen Formel I, worin R¹ einen Rest der eingangs definierten Formel (b) oder (d) und R⁴ einen Formylgruppe bedeuten, mit einem eine (C₁-C₈)-Alkylgruppe, eine (C₂-C₈)-Alkenylgruppe oder eine (C₂-C₈)-Alkinylgruppe liefernden Grignard-Reagens behandelt; oder
g) eine Verbindung der eingangs definierten Formel I, worin R¹ einen Rest der eingangs definierten Formel (b), (c) oder (d) oder einen Rest der obigen Formel (aⁱ) bedeutet, wobei das Molekül mindestens eine Oxogruppe enthält, mit einer Verbindung der allgemeinen Formel
   H₂N-OR⁸ VI
   worin R⁸ die eingangs angegebene Bedeutung besitzt,
   umsetzt; oder
h) eine Verbindung der eingangs definierten Formel I, worin R¹ einen Rest der Formel 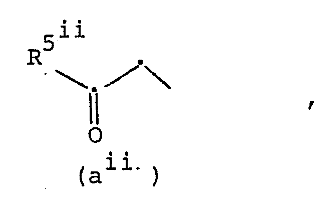 R⁴ einen Rest der eingangs definierten Formel (e), (h), (i) oder (j) oder einen Rest der obigen Formel (fⁱ) und R⁵ⁱⁱ (C₂-C₈)-Alk-1-enyl bedeuten,
   mit einer Verbindung der allgemeinen Formel
   R¹⁵-S-H VII
   worin R¹⁵ die eingangs angegebene Bedeutung besitzt, umsetzt; oder
i) in einer Verbindung der eingangs definierten Formel I, worin R¹ einen Rest der Formel 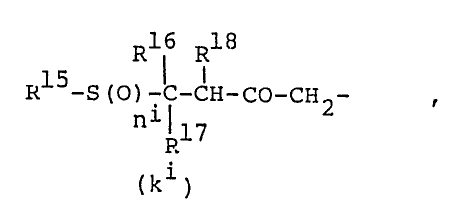 und nⁱ die Zahl 0 oder 1 bedeuten und R¹⁵, R¹⁶, R¹⁷ und R¹⁸ die eingangs angegebene Bedeutung besitzen,
   die Mercaptogruppe zur Sulfinyl- oder Sulfonylgruppe bzw. die Sulfinyl- zur Sulfonylgruppe oxydiert; oder
j) in einer Verbindung der eingangs definierten Formel I, worin R¹ einen Rest der eingangs definierten Formel (a), (b) oder (d) oder einen Rest der Formel 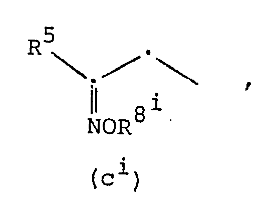 R⁴ einen Rest der eingangs definierten Formel (e), (f), (g), (i) oder (j) oder einen Rest der Formel 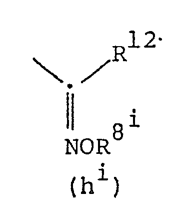 und R⁸ⁱ (C₁-C₈)-Alkyl bedeuten und R⁵ und R¹² die eingangs angegebene Bedeutung besitzen, wobei das Molekül mindestens eine Hydroxygruppe enthält, diese Hydroxygruppe(n) mit einem einen (C₂-C₈)-Alkanoyl rest liefernden Mittel acyliert;
   worauf man erwünschtenfalls eine erhaltene saure Verbindung der eingangs definierten Formel I in ein pharmazeutisch verwendbares Salz mit einer Base überführt.

Als Schutzgruppen in den Verbindungen der allgemeinen Formel II, welche als Ausgangsprodukte in Verfahrensvariante a) verwendet werden, eignen sich selbstverständlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Die Entfernung der Schutzgruppe bzw. der Schutzgruppen aus den Verbindungen der allgemeinen Formel II erfolgt nach an sich bekannten Methoden, wobei natürlich für die Wahl der zur Anwendung gelangenden Methode die Natur der zu entfernenden Schutzgruppe bzw. Schutzgruppen in Betracht gezogen werden muss und zu beachten ist, dass nur die Schutzgruppe bzw. Schutzgruppen selektiv entfernt, andere im Molekül vorhandene Strukturelemente jedoch nicht in Mitleidenschaft gezogen werden sollen.

Als Schutzgruppen eignen sich beispielsweise leicht abspaltbare Acetal- und Ketalschutzgruppen, wie Tetrahydro-2H-pyran-2-yl, Methoxymethyl, Methoxyäthoxymethyl, 1-Methoxy-1-methyläthyl und dgl.; leicht abspaltbare metallorganische Gruppen, insbesondere Trialkylsilylgruppen, wie t-Butyldimethylsilyl, Trimethylsilyl, Triisopropylsilyl und dgl. oder Alkyldiarylsilylgruppen, wie t-Butyldiphenylsilyl und dgl.; usw.

Methoden für die Entfernung der Reste, welche vorstehend als Beispiele für Schutzgruppen erwähnt wurden, sind in der Literatur beschrieben und demnach jedem Fachmann geläufig. Beispielsweise kann eine heterocyclische Gruppe, wie 2-Tetrahydropyranyl, oder eine Alkoxyalkylgruppe, wie Methoxymethyl, leicht abgespalten werden, indem man die Verbindung der Formel II mit einer Säure in Kontakt bringt. Bevorzugte Säuren sind organische Säuren, wie Ameisensäure, Essigsäure, Propionsäure und p-Toluolsulfonsäure, und anorganische Säuren, wie Salzsäure und Schwefelsäure. Die Reaktion wird in Gegenwart oder Abwesenheit eines Lösungsmittels durchgeführt. Vorzugsweise wird jedoch eine Lösungsmittel verwendet, um die Reaktion auf milde Weise durchzuführen. Bevorzugte Lösungsmittel sind Wasser, ein Alkohol, wie Methanol und Aethanol, und eine Mischung von Wasser und einem dieser Alkohole. Es besteht keine spezielle Einschränkung hinsichtlich der Reaktionstemperatur, wobei jedoch Raumtemperatur bevorzugt ist. Die Abspaltung einer Tetrahydro-2H-pyran-2-yl-gruppe erfolgt insbesondere mittels Pyridinium-p-toluolsulfonat, zweckmässigerweise in wässrigem Tetrahydrofuran oder in einem alkoholischen System, z.B. in absolutem Aethanol oder in wässrigem (z.B. 90%igem) Aethanol; bedeutet in Formel II R¹ⁱ einen Rest der obigen Formel (a), so kann beim Arbeiten in einem wasserfreien alkoholischen System teilweise oder vollständige Ketalisierung eintreten, und man erhält, zumindest teilweise, eine entsprechende Verbindung der Formel I, worin R¹ einen Rest der eingangs definierten Formel (b) bedeutet.

Die Abspaltung einer Tetrahydro-2H-pyran-2-yl-gruppe kann aber beispielsweise auch mittels eines sauren Ionenaustauschers erfolgen, z.B. mittels Dowex 50 WX8, wobei man zweckmässigerweise in einem alkoholischen System arbeitet, z.B. in Methanol.

Eine Silylschutzgruppe, wie Trimethylsilyl, kann leicht abgespalten werden, indem man die Verbindung II mit Wasser oder einer wässrigen Lösung einer Säure oder Base in Kontakt bringt. Als Säuren und Basen können organische Säuren z.B. Ameisensäure, Essigsäure und Propionsäure, und anorganische Säuren, z.B. Salzsäure und Schwefelsäure, bzw. anorganische Basen, wie das Hydroxyd eines Alkalimetalls oder eines Erdalkalimetalls, z.B. Kaliumhydroxyd und Calciumhydroxyd, und das Carbonat eines Alkalimetalls oder eines Erdalkalimetalls, z.B. Kaliumcarbonat und Calciumcarbonat, genannt werden. Bezüglich der Reaktionstemperatur besteht keine besondere Einschränkung, jedoch wird im allgemeinen vorzugsweise Raumtemperatur verwendet. Die zur Entfernung der Schutzgruppe erforderliche Zeit variiert in Abhängigkeit von der Art der Schutzgruppe. Die Silylschutzgruppen können auch mit Fluoridhaltigen Reagentien abgespalten werden, beispielsweise mittels Tetrabutylammoniumfluorid in Tetrahydrofuran oder mittels Silberfluorid in Wasser.

Die Oeffnung des Epoxidrings in einer Verbindung der allgemeinen Formel III gemäss Verfahrensvariante b) erfolgt ebenfalls nach an sich bekannten und jedem Fachmann geläufigen Methoden. Beispielsweise kann man die Verbindung der Formel III in einem aromatischen Kohlenwasserstoff, wie Toluol oder dgl., mit Aluminium-isopropylat erhitzen, zweckmässigerweise am Rückfluss. Die Oeffnung des Epoxidringes kann auch mit Amidreagentien, wie z.B. Lithiumdiäthylamid, Lithiumdiisopropylamid und dgl. in Aether oder Hexan oder mit Diäthylaluminium-2,2,6,6-tetramethylpiperidid in Benzol erfolgen.

Die Reduktion eines Carbonsäureesters der allgemeinen Formel IV gemäss Verfahrensvariante c) erfolgt ebenfalls nach an sich bekannten und jedem Fachmann geläufigen Methoden. Zweckmässigerweise verwendet man als Reduktionsmittel ein komplexes Metallhydrid, wie Diisobutylaluminiumhydrid, Lithiumaluminiumhydrid oder Natrium-dihydro-bis[2-methoxyäthoxy]-aluminat, in einem unter den Reaktionsbedingungen inerten organischen Lösungsmittel, beispielsweise in einem Kohlenwasserstoff, wie Hexan, Toluol und dgl., oder in einem Aether, wie Diäthyläther, Tetrahydrofuran und dgl.

Die Hydrolyse eines Carbonsäureesters der allgemeinen Formel V gemäss Verfahrensvariante d) erfolgt ebenfalls nach an sich bekannten und jedem Fachmann geläufigen Methoden. Je nach der Struktur des Ausgangsprodukts, d.h. des Carbonsäure esters der allgemeinen Formel V, wird diese Hydrolyse unter alkalischen oder unter sauren Bedingungen durchgeführt. Eine alkalische Hydrolyse erfolgt zweckmässigerweise mittels einer starken anorganischen Base, beispielsweise eines Alkalimetallhydroxids, wie Natriumhydroxid oder dgl., in einem geeigneten Lösungsmittelsystem, beispielsweise in Wasser oder wässrigem Dioxan oder dgl. Eine saure Hydrolyse erfolgt zweckmässigerweise mit Mineralsäuren, wie Salzsäure oder dgl., in einem geeigneten Lösungsmittelsystem, beispielsweise in wässrigem Tetrahydrofuran oder dgl.

Die Oxidation gemäss Verfahrensvariante e) erfolgt nach an sich bekannten Methoden, welche jedem Fachmann, der sich die Aufgabe stellt, Hydroxygruppen in Oxogruppen überzuführen, geläufig sind. Als Oxidationsmittel verwendet man zweckmässigerweise Mangandioxid (Braunstein) in einem hierfür geeigneten, unter den Reaktionsbedingungen inerten Lösungsmittel, beispielsweise in einem Kohlenwasserstoff, wie Hexan oder dgl., in einem halogenierten Kohlenwasserstoff, wie Methylenchlorid oder dgl., usw. Sind im Ausgangsprodukt zwei Hydroxygruppen vorhanden, wovon die eine primär und die andere sekundär ist, so kann man erwünschtenfalls durch geeignete Wahl der Reaktionsbedingungen die primäre Hydroxygruppe selektiv oxidieren, ohne dass dabei die sekundäre Hydroxygruppe in Mitleidenschaft gezogen wird. Anstelle von Mangandioxid können auch Chrom-haltige Oxidationsmittel, wie z.B. Pyridiniumdichromat oder Pyridiniumchlorochromat und dgl., eingesetzt werden. Als Lösungsmittel werden dabei gebräuchlicherweise halogenierte Kohlenwasserstoffe, wie Methylenchlorid und dgl., für Pyridiniumdichromat auch Dimethylformamid, verwendet. Ein weiteres gängiges Oxidationsmittel ist auch Chromtrioxid in Schwefelsäure (Jones-Reagens).

Auch die Grignard-Reaktion gemäss Verfahrensvariante f) erfolgt nach an sich bekannten Methoden, welche jedem Fachmann geläufig sind. Als Grignard-Reagens verwendet man zweck mässigerweise Verbindungen wie Methylmagnesiumjodid, Vinylmagnesiumbromid, n-Pentylmagnesiumbromid, Cyclopropyllithium oder dgl.; als Lösungsmittel eignen sich beispielsweise Aether, wie Diäthyläther oder Tetrahydrofuran und dgl. Wenn im Ausgangsprodukt R¹ einen Rest der Formel (d) und R⁹ (C₂-C₈)-Alkanoyl bedeutet, so wird diese Alkanoylgruppe im Verlaufe der Grignard-Reaktion abgespalten, und man erhält eine entsprechende Verbindung der Formel I, worin R¹ einen Rest der Formel (d) und R⁹ Wasserstoff bedeuten.

Auch die Oximbildung gemäss Verfahrensvariante g) erfolgt nach an sich bekannten und jedem Fachmann geläufigen Methoden. Zweckmässigerweise wird die Verbindung der Formel VI (z.B. Hydroxylamin, O-Methylhydroxylamin oder dgl.) in Form eines Säureadditionssalzes eingesetzt, beispielsweise als Hydrochlorid; dabei erfolgt die Reaktion in Gegenwart einer Base, z.B. eines Alkalimetallhydroxids, wie Natriumhydroxid und dgl., eines Alkalimetallsalzes einer schwachen Säure, wie Natriumacetat und dgl., usw. Als Lösungsmittel verwendet man zweckmässigerweise alkoholische oder alkoholisch/wässrige Systeme, beispielsweise absolutes Methanol, wässriges Methanol oder dgl.

Für die Umsetzung gemäss Verfahrensvariante h) verwendet man als schwefelhaltige Komponente Verbindungen der Formel VII, wie 2-Mercaptoäthanol, Thioglykolsäuremethylester, Thioessigsäure oder dgl. oder geschützte Derivate von Verbindungen der Formel VII, wie N-(2-Acetylthioäthyl)acetamid, S-[(2-Acetamido-2-methoxycarbonyl)äthyl]thioacetat oder dgl.; aus solchen geschützten Derivaten wird die Schutzgruppe unter den Reaktionsbedingungen abgespalten, wobei die entsprechende Verbindung der Formel VII freigesetzt wird und in situ reagiert. Die Umsetzung erfolgt zweckmässigerweise in Gegenwart einer Base, beispielsweise einer tertiären organischen Base, wie Triäthylamin oder dgl., und in einem geeigneten, unter den Reaktionsbedingungen inerten organischen Lösungsmittel, beispielsweise in einem Alkohol, wie Methanol oder dgl., in einem halogenierten Kohlenwasserstoff, wie Methylenchlorid oder dgl., usw.

Für die Oxidation gemäss Verfahrensvariante i) bei welcher eine Mercaptogruppe zur Sulfinyl- oder Sulfonylgruppe bzw. eine Sulfinylgruppe zur Sulfonylgruppe oxidiert wird, verwendet man Oxidationsmittel, welche für derartige Ueberführungen gebräuchlich sind, beispielsweise Persäuren, wie m-Chlorperbenzoesäure, Wasserstoffperoxid, Perester, Natriummetaperjodat, usw. Die Oxidation erfolgt zweckmässigerweise in einem unter den Reaktionsbedingungen inerten organischen Lösungsmittel, beispielsweise in einem halogenierten Kohlenwasserstoff, wie Methylenchlorid, Chloroform, Dichloräthan und dgl., oder in einem Kohlenwasserstoff, wie Benzol und dgl.; bei Verwendung von Wasserstoffperoxid als Oxidationsmittel kann auch in Essigsäure, wässriger Essigsäure und dgl. gearbeitet werden. Es ist von Vorteil, dass Oxidatonsmittel in leichtem Ueberschuss bezüglich des zu oxidierenden Produktes einzusetzen. Zweckmässigerweise wird bei Raumtemperatur oder darunter gearbeitet, vorzugsweise bei Temperaturen von etwa -50 bis etwa 0°C.

Als O-Acylierungsmittel verwendet man in Verfahrensvariante j) reaktionsfähige funktionelle derivate von (C₂-C₈)-Alkancarbonsäuren, zweckmässigerweise entsprechende Säurechloride oder Säureanhydride, wie Essigsäureanhydrid oder dgl. Die Acylierung erfolgt in Gegenwart einer Base, zweckmässigerweise einer tertiären organischen Base, wie Pyridin, Triäthylamin, N-Methylpiperidin, 4-Dimethylaminopyridin oder dgl. Als Lösungsmittel eignen sich in erster Linie halogenierte Kohlenwasserstoffe, wie Methylenchlorid oder dgl.; falls als Base Pyridin verwendet wird, so kann dieses gleichzeitig auch als Lösungsmittel dienen.

Die Ueberführung einer erhaltenen sauren Verbindung der Formel I in ein pharmazeutisch verwendbares Salz kann durch Behandeln mit einer pharmazeutisch annehmbaren Base in an sich bekannter Weise bewerkstelligt werden. Als derartige Salze eignen sich sowohl solche mit von einer anorganischen Base abgeleiteten Kationen, z.B. also Kaliumsalze, Natriumsalze, Calciumsalze und dgl., als auch Salze mit organischen Basen, wie Aethylendiamin, Monoäthanolamin, Diäthanolamin und dgl.

Die Herstellung der Ausgangsprodukte der allgemeinen Formel II, III, IV und V kann ausgehend von bekannten Verbindungen nach an sich bekannten und jedem Fachmann geläufigen Methoden erfolgen. In den nachfolgenden Schemata 1 bis 5 wird die Herstellung derartiger Verbindungen anhand ausgewählter spezifischer Fälle illustriert. Die in diesen Schemata vorkommenden Abkürzungen haben folgende Bedeutungen:
DHP = 3,4-Dihydro-2H-pyran
DIBAH = Diisobutylaluminiumhydrid
OTHP = (Tetrahydro-2H-pyran-2-yl)oxy
PTS = Pyridinium-p-toluolsulfonat
Schemata 1 bis 3 beziehen sich auf die Herstellung von Verbindungen der Formel II, Schema 4 auf die Herstellung von Verbindungen der Formel III und Schema 5 auf die Herstellung von Verbindungen der Formeln IV und V.

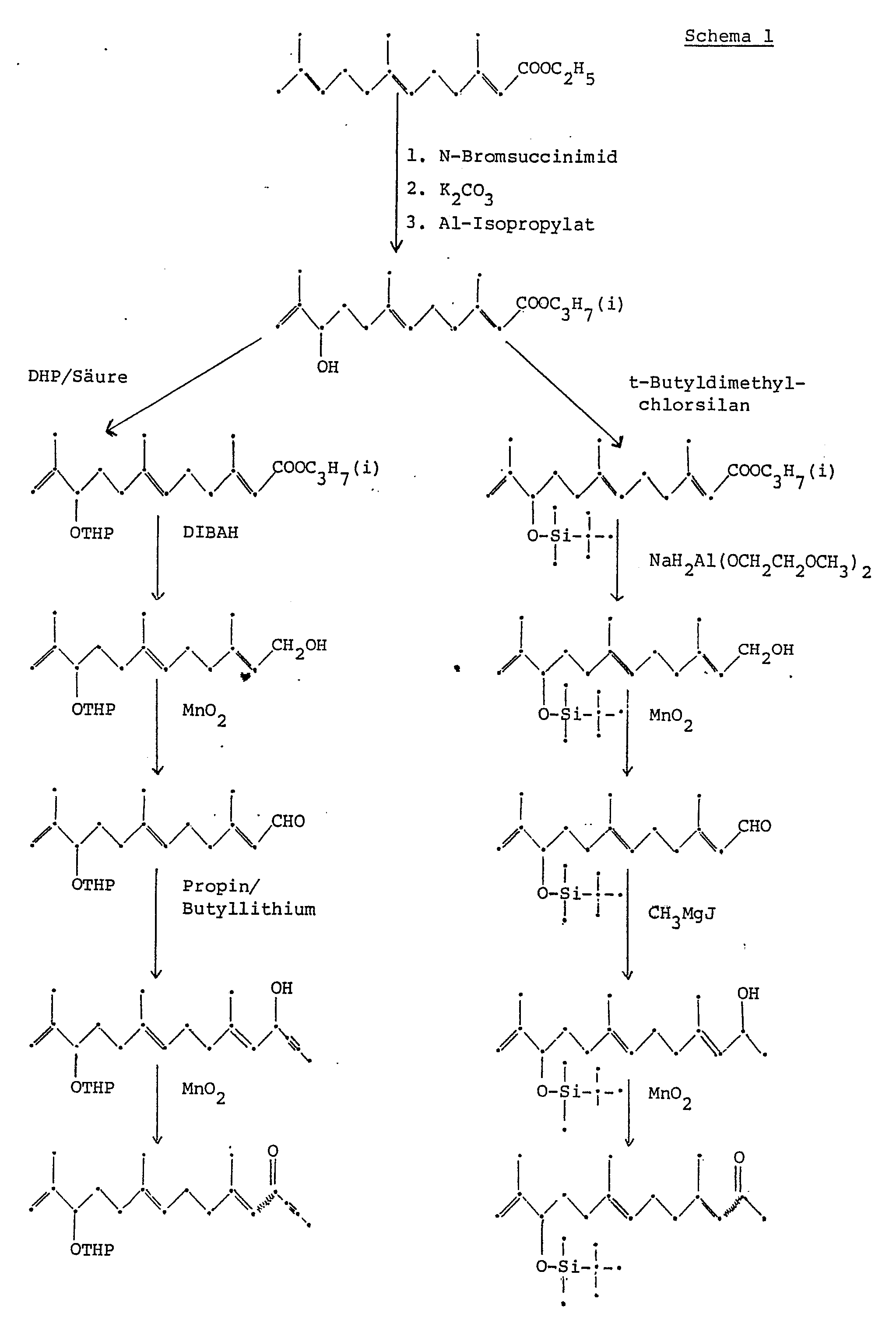

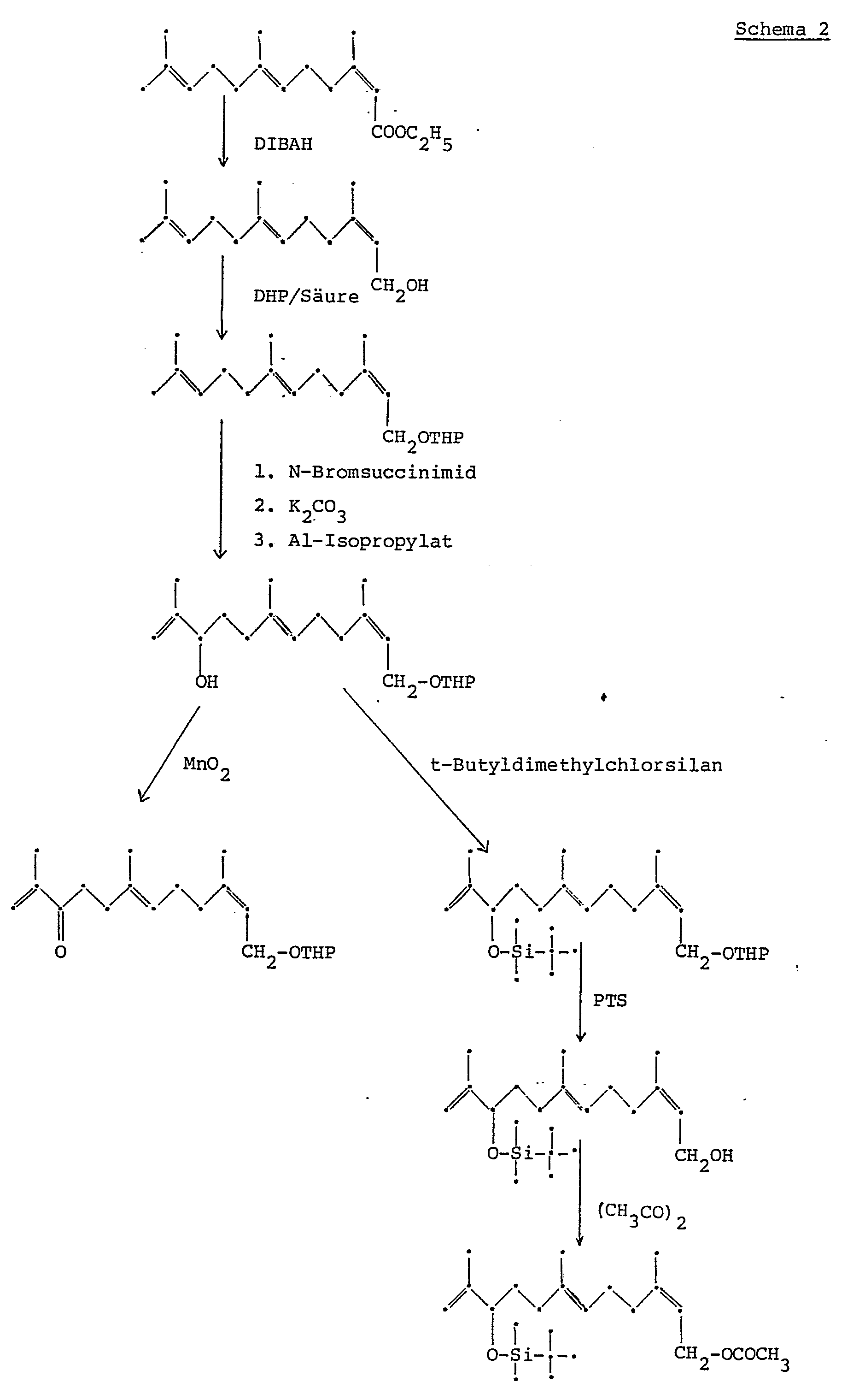

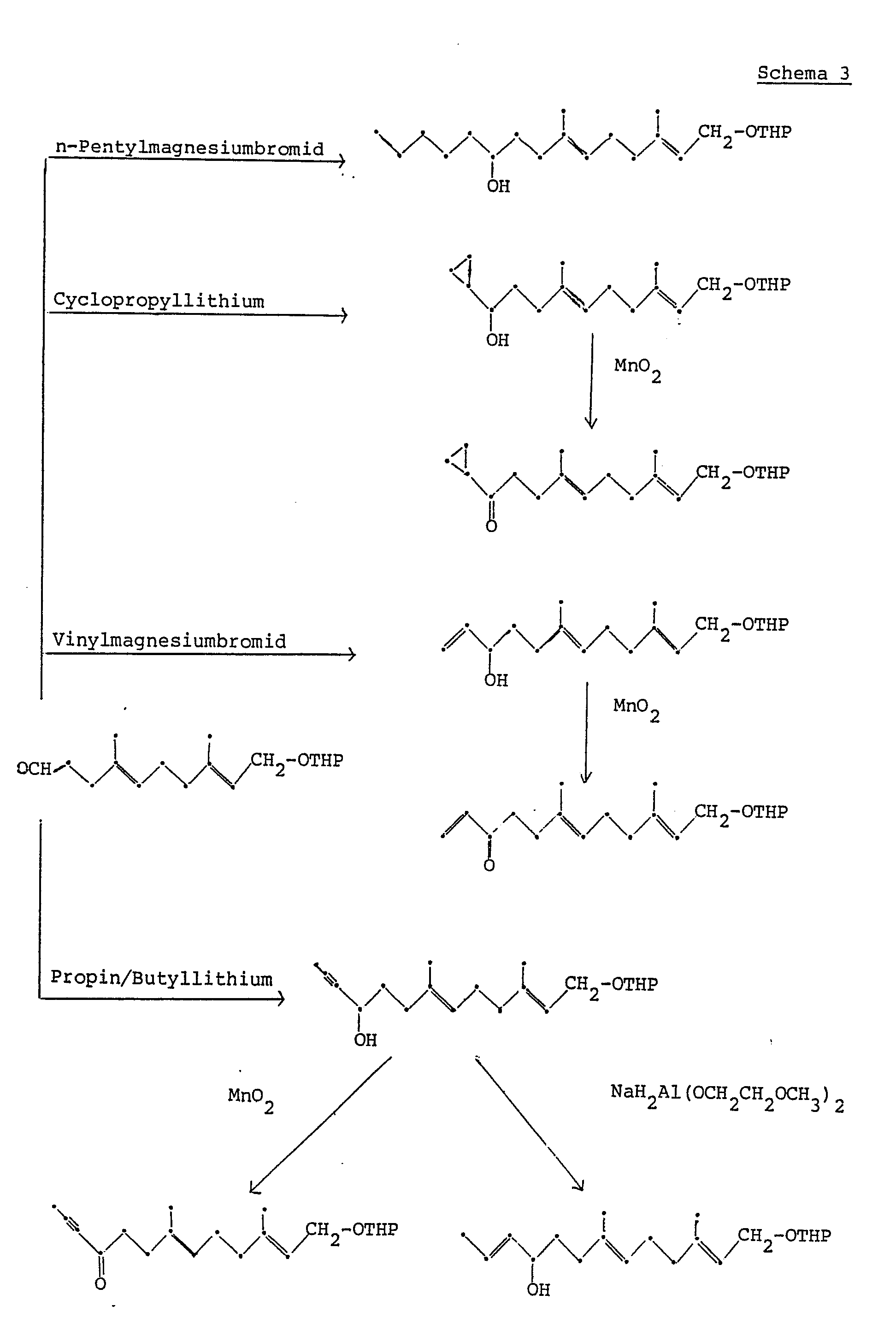

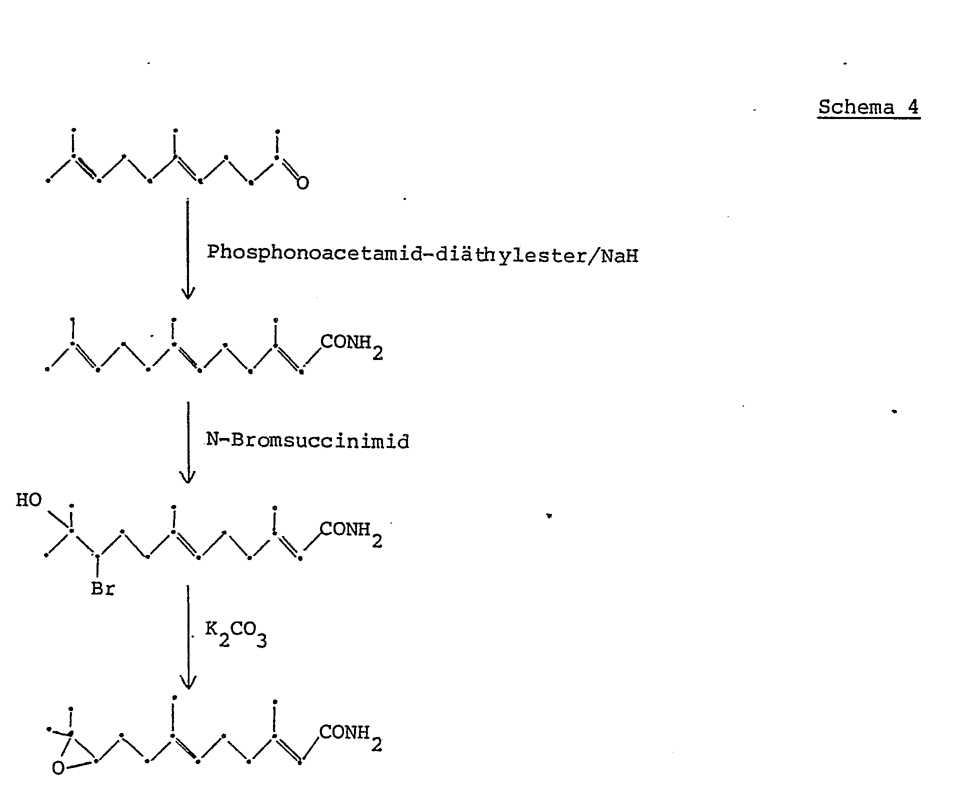

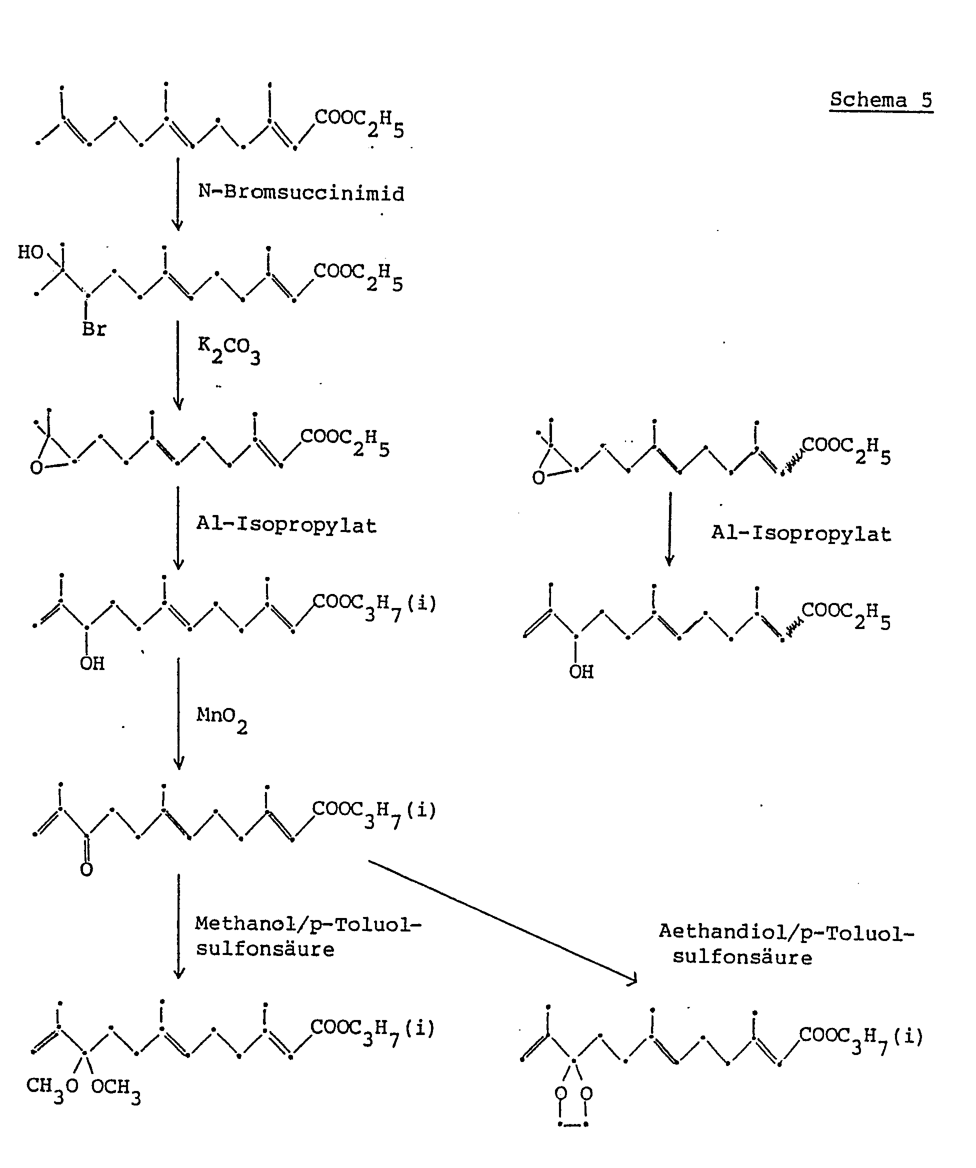

Darüberhinaus enthalten zahlreiche der nachfolgenden Beispiele ausführliche Angaben betreffend die Herstellung von spezifischen Verbindungen, welche unter eine der Formeln II bis V fallen.

Die Ausgangsprodukte der Formeln VI und VII sowie geschützte Derivate von Verbindungen der Formel VII sind bekannt oder nach an sich bekannten und jedem Fachmann geläufigen Methoden leicht herstellbar.

Wie eingangs erwähnt, besitzen die Verbindungen der allgemeinen Formel I sowie pharmazeutisch verwendbare Salze von sauren Verbindungen der Formel I mit Basen wertvolle pharmakodynamische Eigenschaften.

Repräsentative Verbindungen der Formel I wurden auf ihre mucosaprotektiven und magensäuresekretionshemmenden Eigenschaften sowie auf ihre Toxizität untersucht.

Zur Bestimmung der mucosa-protektiven Eigenschaft wurde die nachstehend beschriebene Versuchsanordnung verwendet.

Orale Verabreichung von absolutem Aethanol an männliche Ratten in einer Dosis von 1 ml pro Ratte führt innerhalb 1 Stunde zu blutigen Läsionen der Magenschleimhaut. Verschiedene Dosen der zu prüfenden Substanzen (suspendiert in 0.125% Carboxymethylcellulose) oder das Vehikel allein (Kontrolle) werden den Ratten oral (1 ml pro Ratte) 30 Minuten vor der Behandlung mit Aethanol verabreicht. Eine Stunde nach der Verabreichung des Aethanols tötet man die Tiere, untersucht deren Mägen auf das Vorhandensein von Läsionen und ermittelt die Anzahl und die gesamte Ausdehnung solcher Läsionen. Als ID₅₀ bezeichnet man diejenige Dosis einer Testsubstanz, welche die Anzahl der Läsionen im Vergleich zur Kontrollgruppe um 50% reduziert.

Zur Bestimmung der magensäuresekretionshemmenden Wirkung wurde die nachstehend beschriebene Versuchsanordnung verwendet:

Männlichen Ratten wird unter leichter Aethernarkose gemäss Shay et al. [Gastroenterology 5, 43 (1945)] der Pylorus ligiert. Die zu prüfenden Substanzen, suspendiert in 0,5% Carboxymethylcellulose, werden intraduodenal verabreicht; Kontrolltiere werden lediglich mit dem Vehikel behandelt. Fünf Stunden nach der Ligation tötet man die Tiere, bestimmt das Volumen und die Azidität ihres Magensaftes und vergleicht die erhaltenen Werte mit denjenigen von Kontrolltieren. Als ID₅₀ bezeichnet man diejenige Dosis einer Testsubstanz, welche bei den behandelten Tieren im Vergleich zu den Kontrolltieren eine 50%ige Verminderung der Sekretion bewirkt.

In der nachfolgenden Tabelle werden für eine Reihe repräsentativer Verbindungen der Formel I die Resultate der Prüfung auf ihre mucosa-protektive Wirkung ("Aethanol-Test") und auf ihre magensäuresekretionshemmende Wirkung wiedergegeben. Ausserdem enthält diese Tabelle Angaben über die akute Toxizität (DL₅₀) bei einmaliger oraler Verabreichung an Mäuse).

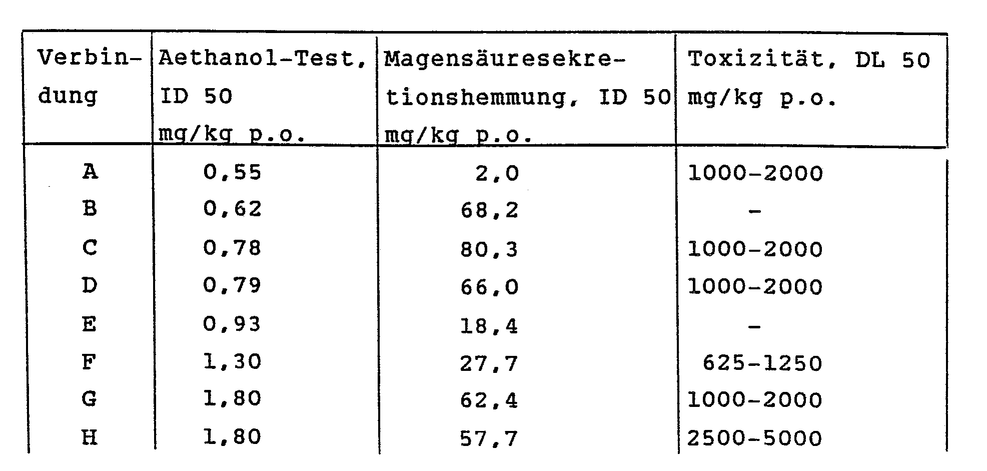

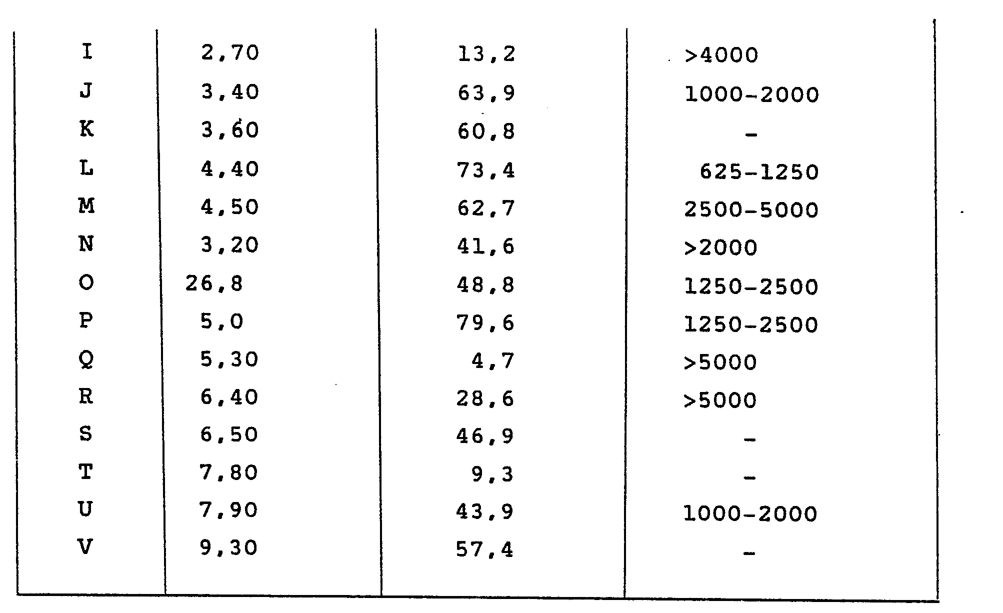

A = (all-E)-12-Hydroxy-6,10-dimethyl-6,10-dodecadien-1-in-3-on
B = (all-E)-12-Hydroxy-6,10-dimethyl-1,6,10-dodecatrien-3-on
C = (all-E)-13-Hydroxy-7,11-dimethyl-7,11-tridecadien-2-in-4-on
D = (all-E)-13-Hydroxy-7,11-dimethyl-2,7,11-tridecatrien-4-on
E = (all-E)-10,10-Diäthoxy-3,7-dimethyl-2,6-dodecadien-11-in-1-ol
F = (all-E)-12-Hydroxy-2,6,10-trimethyl-1,6,10-dodecatrien-3-on
G = (all-E)-10-Oxo-3,7,11-trimethyl-2,6,11-dodecatrienal
H = (6Z,10Z)-12-Hydroxy-2,6,10-trimethyl-1,6,10-dodecatrien-3-on
I = (all-E)-3,7-Dimethyl-9-[2-(1-methylvinyl)-1,3-dioxolan-2-yl]-2,6-nonadien-1-ol
J = (E,E)-3,7,11-Trimethyl-10-oxo-2,6,11-dodecatrienamid
K = (all-E)-13-Hydroxy-2,7,11-trimethyl-2,7,11-tridecatrien-4-on
L = (6E,10Z)-12-Hydroxy-2,6,10-trimethyl-1,6,10-dodecatrien-3-on
M = (6Z,10E)-12-Hydroxy-2,6,10-trimethyl-1,6,10-dodecatrien-3-on
N = (all-E)-3,7,11-Trimethyl-2,6,11-dodecatrien-1,10-diol
O = (all-E)-10-Hydroxy-3,7,11-trimethyl-2,6,11-dodecatriensäure
P = (all-E)-10-Oxo-3,7,11-trimethyl-2,6,11-dodecatriensäure
Q = (all-E)-10,10-Dimethoxy-3,7,11-trimethyl-2,6,11-dodecatrien-1-ol
R = (all-E)-10-Hydroxy-3,7,11-trimethyl-2,6,11-dodecatrienal
S = (all-E)-12-Hydroxy-6,10-dimethyl-1-(trimethylsilyl)-6,10-dodecadien-1-in-3-on
T = (2E,6E)-3,7,11-Trimethyl-10-oxo-2,6,11-dodecatrienal-(E/Z)-oxim
U = (6E,10E)-12-Hydroxy-6,10-dimethyl-6,10-dodecadien-1-in-3-on-(E/Z)-O-methyloxim
V = (2E,6E,11(E/Z))-3,7-Dimethyl-2,6,11-tridecatrien-1,10-diol

Die eingangs definierten Verbindungen und Salze können als Heilmittel, z.B. in Form pharmazeutischer Präparate Verwendung finden. In erster Linie kommt orale Verabreichung in Form von festen pharmazeutischen Präparaten, wie Tabletten, Lacktabletten, Dragées, Hartgelatinekapseln und Weichgelatinekapseln in Frage. Orale Verabreichung in Form flüssiger pharmazeutischer Präparate, wie Lösungen, Emulsionen und Suspensionen, rektale Verabreichung, z.B. in Form von Suppositorien, oder parenterale Verabreichung, z.B. in Form von Injektionslösungen, sind zwar weniger in Betracht zu ziehen aber auch nicht auszuschliessen.

Arzneimittel, enthaltend eine der eingangs definierten Verbindungen und Salze, sind ebenfalls Gegenstand der vorliegenden Erfindung. Die Herstellung derartiger Arzneimittel kann dadurch erfolgen, dass man eine oder mehrere der eingangs definierten Verbindungen und Salze und erwünschtenfalls einen oder mehrere andere therapeutische Wirkstoffe zusammen mit einem oder mehreren therapeutisch inerten Excipientien in eine galenische Darreichungsform bringt.

Zur Herstellung von Tabletten, Lacktabletten, Dragées und Hartgelatinekapseln können die eingangs definierten Verbindungen und Salze mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden. Zur Herstellung von magensaftresistenten pharmazeutischen Präparaten ist noch ein magensaftresistenter Lack aufzubringen, welcher z.B. aus Hydroxypropylmethylcellulosephthalat bestehen kann.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glucose und dgl.

Für Suppositorien eignen sich als Excipientien z.B. natürliche oder gehärtete Oele, Wachse, Fette, halbflüssige oder flüssige Polyole und dgl.

Für Injektionslösungen eignen sich als Excipientien z.B. Wasser, Alkohole, Polyole, Glycerin, vegetabile Oele etc.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die eingangs definierten Verbindungen und Salze bei der Bekämpfung bzw. Verhütung von Krankheiten verwenden, beispielsweise bei der Bekämpfung bzw. Verhütung von Ulcus ventriculi und duodeni. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 30-400 mg und bei intravenöser Verabreichung eine Tagesdosis von etwa 1-50 mg angemessen sein.

Gegenstand der Erfindung ist auch die Verwendung der eingangs definierten Verbindungen und Salze zur Herstellung von Arzneimitteln für die Bekämpfung bzw. Verhütung von Ulcus ventriculi und duodeni.

In den nachfolgenden Beispielen, welche die vorliegende Erfindung illustrieren, ihren Umfang aber in keiner Weise beschränken sollen, sind sämtliche Temperaturen in Celsiusgraden angegeben.

### Beispiel 1

a) Eine Lösung von 1 g (0,0038 Mol) Aethyl (2Z,6E)-3,7,11-trimethyl-2,6,10-dodecatrienoat in 8 ml Toluol wird tropfenweise bei -5° bis -10° mit 9,45 ml einer Lösung von Diisobutylaluminiumhydrid in Toluol (1,2 Mol/l) versetzt. Die Lösung wird bei gleicher Temperatur 1 Stunde unter Argon weitergerührt. Das Reaktionsgemisch wird auf ein Eis-Wassergemisch gegossen und mit Aether extrahiert. Nach dem Trocknen und Entfernen der Lösungsmittel wird der Rückstand mit Aether-Hexan 1:1 an Silicagel chromatographiert. Man erhält (2Z,6E)-3,7,11-Trimethyl-2,6,10-dodecatrien-1-ol, das im IR-Spektrum folgende Banden aufweist; 3326 cm⁻¹, 1669 cm⁻¹, 1446 cm⁻¹, 1378 cm⁻¹, 1000 cm⁻¹.
   Eine Lösung von 1 g (0,0045 Mol) (2Z,6E)-3,7,11-Trimethyl-2,6,11-dodecatrien-1-ol in 32 ml 3,4-Dihydro-2H-pyran wird bei 0° mit 91 mg p-Toluolsulfonsäure versetzt. Die Lösung wird 2 Stunden bei gleicher Temperatur unter Argon gerührt. Nach Zugabe von 100 ml Wasser wird mit Aether extrahiert. Die organische Phase wird mit Natriumbicarbonatlösung gewaschen. Nach dem Trocknen und Entfernen der Lösungsmittel wird mit Aether-Hexan 1:1 an Silicagel chromatographiert. Man erhält Tetrahydro-2-[[(2Z,6E)-3,7,11-trimethyl-2,6,10 -dodecatrienyl]oxy]-2H-pyran, das im IR-Spektrum folgende Banden aufweist: 1666 cm⁻¹, 1444 cm⁻¹, 1379 cm⁻¹, 1116 cm⁻¹, 1075 cm⁻¹, 1020 cm⁻¹.
b) Eine Lösung von 2 g (0,0065 Mol) Tetrahydro-2-[[(2Z,6E)-3,7,11-trimethyl-2,6,10 -dodecatrienyl]oxy]-2H-pyran, gelöst in 13 ml Monoglym und 2,6 ml Wasser, wird bei -10° portionenweise mit 1,3 g N-Bromsuccinimid versetzt. Anschliessend wird 1/4 Stunde bei 0° und 2 Stunden bei Raumtemperatur gerührt. Die Reaktionslösung wird auf 100 ml Wasser gegossen und mit Essigester extrahiert. Die organische Phase wird mit gesättigter Kochsalzlösung gewaschen. Nach dem Trocknen und Entfernen der Lösungsmittel wird der Rückstand, enthaltend Tetrahydro-2-[[(2Z,6E)-10-brom-11-hydroxy-3,7,11 -trimethyl-2,6,10-dodecatrienyl]oxy]-2H-pyran, in 18 ml Methanol gelöst und mit 600 mg Kaliumcarbonat versezt. Die Suspension wird 1,5 Stunden bei Raumtemperatur unter Argon gerührt. Nach dem Entfernen des Methanols am Rotationsverdampfer wird mit 500 ml Aether und 200 ml Wasser versetzt. Die organische Phase wird mit gesättigter Kochsalzlösung gewaschen. Nach dem Trocknen und Entfernen der Lösungsmittel wird der Rückstand, enthaltend (6E,10Z)-2,3-Epoxy-2,6,10-trimethyl-12-[(tetrahydro -2H-pyran-2-yl)oxy]-6,10-dodecadien, in 30 ml Toluol gelöst und mit 1,35 g Aluminiumisopropylat versetzt. Das Reaktionsgemisch wird während 17 Stunden am Rückfluss gekocht und nach Abkühlen auf Raumtemperatur auf 200 ml Eis-Wassergemisch gegossen. Nach Ansäuern mit 1N-Salzsäure wird mit 3 × 150 ml Hexan extrahiert. Die organische Phase wird mit 500 ml gesättigter Kochsalzlösung gewaschen. Nach dem Trocknen und Entfernen der Lösungsmittel wird der Rückstand mit einem 1:2 Aether-Hexangemisch, das zusätzlich 0,1% Triäthylamin enthält, an Silicagel chromatographiert. Man erhält (6E,10Z)-2,6,10-Trimethyl-12-[(tetrahydro-2H-pyran -2-yl)oxy]-1,6,10-dodecatrien-3-ol, das im IR-Spektrum folgende Banden aufweist: 3445 cm⁻¹, 1648 cm⁻¹, 1441 cm⁻¹, 1375 cm⁻¹, 901 cm⁻¹.
c) Eine Lösung von 0,5 g (0,0016 Mol) (6E,10Z)-2,6,10-Trimethyl-12-[(tetrahydro-2H-pyran -2-yl)oxy]-1,6,10-dodecatrien-3-ol, gelöst in 14,5 ml Tetrahydrofuran und 3,7 ml Wasser, wird mit 167,2 mg (0,4 Moläquivalenten)Pyridinium-p-toluolsulfonat versetzt. Die Lösung wird während 17 Stunden am Rückfluss gekocht. Nach dem Abkühlen werden 100 ml Wasser und 300 ml Aether zugegeben. Die organische Phase wird mit Natriumbicarbonatlösung neutralisiert. Nach dem Trocknen und Entfernen der Lösungsmittel wird der Rückstand mit Aether-Hexan 1:1 an Silicagel chromatographiert. Man erhält (6E,10Z)-2,6,10-Trimethyl-1,6,10-dodecatrien-3,12-diol, das im IR-Spektrum folgende Banden aufweist: 3344 cm⁻¹, 1647 cm⁻¹, 1449 cm⁻¹, 1375⁻¹, 997 cm⁻¹.

### Beispiel 2

Eine Lösung von 0,5 g (0,0016 Mol) (6E,10Z)-2,6,10-Trimethyl-12-[(tetrahydro-2H-pyran -2-yl)oxy]-1,6,10-dodecatrien-3-ol, gelöst in 15 ml Methylenchlorid, wird mit 10 g Mangandioxid versetzt. Die Suspension wird 18 Stunden bei Raumtemperatur unter Argon gerührt. Nach dem Filtrieren und Entfernen des Lösungsmittels wird der Rückstand mit Aether-Hexan 10:1 an Silicagel chromatographiert. Das Produkt, enthaltend (6E,10Z)-2,6,10-Trimethyl-12-[(tetrahydro-2H- pyran -2-yl)oxy]-1,6,10-dodecatrien-3-on, wird in 10 ml Tetrahydrofuran und 2, 6 ml Wasser gelöst und mit 118 mg Pyridinium-p-toluolsulfonat versetzt. Die Lösung wird während 17 Stunden am Rückfluss gekocht. Nach dem Abkühlen werden 100 ml Wasser und 200 ml Aether zugegeben. Die organische Phase wird mit Natriumbicarbonatlösung neutralisiert. Nach dem Trocknen und Entfernen der Lösungsmittel wird der Rückstand mit Aether-Hexan 1:1 an Silicagel chromatographiert. Man erhält (6E,10Z)-12-Hydroxy-2,6,10-trimethyl-1,6,10-dodecatrien-3-on, das folgende Absorptionsbanden im IR-Spektrum zeigt: 3390 cm⁻¹, 1678 cm⁻¹, 1625 cm⁻¹, 1415 cm⁻¹, 1380 cm⁻¹, 1001 cm⁻¹, 945 cm⁻¹.

### Beispiel 3

Eine Lösung von 0,5 g (0,002 Mol) (6E,10Z)-12-Hydroxy-2,6,10-trimethyl-1,6,10-dodecatrien-3-on, gelöst in 5 ml Methylenchlorid und 1.3 ml Pyridin, wird mit 1 ml Essigsäureanhydrid versetzt. Die Lösung wird während 18 Stunden bei Raumtemperatur unter Argon gerührt. Nach dem Entfernen der Lösungsmittel wird der Rückstand dreimal in Toluol aufgenommen und am Rotationverdampfer vom Lösungsmittel befreit. Der Rückstand wird mit Aether-Hexan 1:5 an Silicagel chromatographiert. Man erhält (2Z,6E)-3,7,11-Trimethyl-10-oxo-2,6,11-dodecatrienylacetat, das im IR-Spektrum folgende Banden aufweist: 1735 cm⁻¹, 1677 cm⁻¹, 1630 cm⁻¹, 1448 cm⁻¹, 1368 cm⁻¹, 950 cm⁻¹.

### Beispiel 4

a) Eine Lösung von 1 g (0,0038 Mol) Aethyl (2E,6Z)-3,7,11-trimethyl-2,6,10-dodecatrienoat in 8 ml Toluol wird tropfenweise bei -5° bis -10° mit 9,45 ml einer Lösung von Diisobutylaluminiumhydrid in Toluol (1,2, Mol/l, d.h. 3,0 Moläquivalente) versetzt. Die Lösung wird bei gleicher Temperatur 1 Stunde unter Argon weitergerührt. Das Reaktionsgemisch wird auf ein Eis-Wassergemisch gegossen und mit Aether extrahiert. Nach dem Trocknen und Entfernen der Lösungsmittel wird der Rückstand mit Aether-Hexan 1:1 an Silicagel chromatographiert. Man erhält (2E,6Z)-3,7,11-Trimethyl-2,6,10-dodecatrien-1-ol, das im IR-Spektrum folgende Banden aufweist: 3323 cm⁻¹, 1668 cm⁻¹, 1446 cm⁻¹, 1377 cm⁻¹, 1001 cm⁻¹.

   Eine Lösung von 1 g (0,0045 Mol) (2E,6Z)-3,7,11-Trimethyl-2,6,11-dodecatrien-1-ol in 32 ml 3,4-Dihydro-2H-pyran wird bei 0° mit 91 mg p-Toluolsulfonsäure versetzt. Die Lösung wird 2 Stunden bei gleicher Temperatur unter Argon gerührt. Nach Zugabe von 100 ml Wasser wird Aether extrahiert. Die organische Phase wird mit Natriumbicarbonatlösung gewaschen. Nach dem Trocknen und Entfernen der Lösungsmittel wird Aether-Hexan 1:1 an Silicagel chromatographiert. Man erhält Tetrahydro-2-[[2E,6Z)-3,7,11-trimethyl-2,6,10 -dodecatrienyl]oxy]-2H-pyran, das im IR-Spektrum folgende Banden aufweist: 1669 cm⁻¹, 1448 cm⁻¹, 1377 cm⁻¹, 1115 cm⁻¹, 1078 cm⁻¹, 1019 cm⁻¹.

   Eine Lösung von 2 g (0,0065 Mol) Tetrahydro-2-[[(2E,6Z)-3,7,11-trimethyl-2,6,10 -dodecatrienyl]oxy]-2H-pyran, gelöst in 13 ml Monoglym und 2,6 ml Wasser, wird bei -10° portionenweise mit 1,3, g N-Bromsuccinimid versetzt. Anschliessend wird 1/4 Stunde bei 0° und 2 Stunden bei Raumtemperatur gerührt. Die Reaktionslösung wird auf 100 ml Wasser gegossen und mit Essigester extrahiert. Die organische Phase wird mit gesättigter Kochsalzlösung gewaschen. Nach dem Trocknen und Entfernen der Lösungsmittel wird der Rückstand, enthaltend Tetrahydro-2-[[(2E,6Z)-10-brom-11-hydroxy-3,7,11-trimethyl -2,6,10-dodecatrienyl]oxy]-2H-pyran, in 18 ml Methanol gelöst und mit 600 mg Kaliumcarbonat versetzt. Die Suspension wird 1,5 Stunden bei Raumtemperatur unter Argon gerührt. Nach dem Entfernen des Methanols am Rotationsverdampfer wird mit 500 ml Aether und 200 ml Wasser versetzt. Die organische Phase wird mit gesättigter Kochsalzlösung gewaschen. Nach dem Trocknen und Entfernen der Lösungsmittel wird der Rückstand, enthaltend (6Z,10E)-2,3-Epoxy-2,6,10-trimethyl-12-[(tetrahydro-2H-pyran -2-yl)oxy]-6,10-dodecadien, in 30 ml Toluol gelöst und mit 1,35 g Aluminiumisopropylat versetzt. Das Reaktionsgemisch wird während 17 Stunden am Rückfluss gekocht. Nach Abkühlen auf Raumtemperatur wird das Reaktionsgemisch auf 200 ml Eis-Wassergemisch gegossen. Nach dem Ansäuern mit 1N-Salzsäure wird mit 3 × 150 ml extrahiert. Die organische Phase wird mit 500 ml gesättigter Kochsalzlösung gewaschen. Nach dem Trocknen und Entfernen der Lösungsmittel wird der Rückstand mit einem 1:2 Aether-Hexangemisch, das zusätzlich 0,1% Triäthylamin enthält, an Silicagel chromatographiert. Man erhält (6Z,10E)-2,6,10-Trimethyl-12-[tetrahydro-2H-pyran -2-yl)oxy]-1,6,10-dodecatrien-3-ol, das im IR-Spektrum folgende Banden aufweist: 3447 cm⁻¹, 1644 cm⁻¹, 1443 cm⁻¹, 1379 cm⁻¹, 909 cm⁻¹.
b) Eine Lösung von 0,5 g (0,0016 Mol) (6Z,10E)-2,6,10-Trimethyl-12-[(tetrahydro-2H-pyran -2-yl)oxy]-1,10-dodecatrien-3-o1, gelöst in 14,5 ml Tetrahydrofuran und 3,7 ml Wasser, wird mit 167,2 mg (0,4 Moläquivalente) Pyridinium-p-toluolsulfonat versetzt. Die Lösung wird während 17 Stunden am Rückfluss gekocht. Nach dem Abkühlen werden 100 ml Wasser und 300 ml Aether zugegeben. Die organische Phase wird mit Natriumbicarbonatlösung neutralisiert. Nach dem Trocknen und Entfernen der Lösungsmittel wird der Rückstand mit Aether-Hexan 1:1 an Silicagel chromatographiert. Man erhält (6E,10Z)-2,6,10-Trimethyl-1,6,10-dodecatrien-3,12-diol, das im IR-Spektrum folgende Banden aufweist: 3346 cm⁻¹, 1649 cm⁻¹, 1446 cm⁻¹, 1377 cm⁻¹, 1001 cm⁻¹.

### Beispiel 5

Eine Lösung von 0,5 g (0,0016 Mol) (6Z,10E)-2,6,10-Trimethyl-12-[(tetrahydro-2H-pyran -2-yl)oxy]-1,6,10-dodecatrien-3-ol, gelöst in 15 ml Methylenchlorid, wird mit 10 g Mangandioxid versetzt. Die Suspension wird 18 Stunden bei Raumtemperatur unter Argon gerührt. Nach dem Filtrieren und Entfernen des Lösungsmittels wird der Rückstand mit Aether-Hexan 10:1 an Silicagel chromatographiert. Das Produkt, enthaltend (6Z,10E)-2,6,10-Trimethyl-12-[(tetrahydro-2H-pyran -2-yl)oxy]-1,6,10-dodecatrien-3-on, wird in 10 ml Tetrahydrofuran und 2,6 ml Wasser gelöst und mit 118 mg Pyridinium-p-toluolsulfonat versetzt. Die Lösung wird während 17 Stunden am Rückfluss gekocht. Nach dem Abkühlen werden 100 ml Wasser und 200 ml Aether zugegeben. Die organische Phase wird mit Natriumbicarbonatlösung neutralisiert. Nach dem Trocknen und Entfernen der Lösungsmittel wird der Rückstand mit Aether-Hexan 1:1 an Silicagel chromatographiert. Man erhält (6Z,10E)-12-Hydroxy-2,6,10-trimethyl-1,6,10-dodecatrien-3-on, das folgende Absorptionsbanden im IR-Spektrum zeigt: 3389 cm⁻¹, 1675 cm⁻¹, 1626 cm⁻¹, 1413 cm⁻¹, 1382 cm⁻¹, 1000 cm⁻¹, 948 cm⁻¹.

### Beispiel 6

Eine Lösung von 0,5 g (0,0021 Mol) (6Z,10E)-12-Hydroxy-2,6,10-trimethyl-1,6,10-dodecatrien-3-on, gelöst in 5 ml Methylenchlorid und 1,3 ml Pyridin, wird mit 1 ml Essigsäureanhydrid versetzt. Die Lösung wird während 18 Stunden bei Raumtemperatur unter Argon gerührt. Nach dem Entfernen der Lösungsmittel wird der Rückstand dreimal in Toluol aufgenommen und am Rotationsverdampfer vom Lösungsmittel befreit. Der Rückstand wird mit Aether-Hexan 1:5 an Silicagel chromatographiert. Man erhält (2E,6Z)-3,7,11-Trimethyl-10-oxo-2,6,11-dodecatrienylacetat, das im IR-Spektrum folgende Banden aufweist: 1739 cm⁻¹, 1675 cm⁻¹, 1629 cm⁻¹, 1449 cm⁻¹, 1370 cm⁻¹, 948 cm⁻¹.

### Beispiel 7

a) Eine Lösung von 1 g (0,0038 Mol) Aethyl (2Z,6Z)-3,7,11-trimethyl-2,6,10-dodecatrienoat in 8 ml Toluol wird tropfenweise bei -5° bis -10° mit 9,45 ml einer Lösung von Diisobutylalumiumhydrid in Toluol (1,2, Mol,l, d.h. 3,0 Moläquivalente) versetzt. Die Lösung wird bei gleicher Temperatur 1 Stunde unter Argon weitergerührt. Das Reaktionsgemisch wird auf ein Eis-Wassergemisch gegossen und mit Aether extrahiert. Nach dem Trocknen und Entfernen der Lösungsmittel wird der Rückstand mit Aether-Hexan 1:1 an Silicagel chromatographiert. Man erhält (2Z,6Z)-3,7,11-Trimethyl-2,6,10-dodecatrien-1-ol, das im IR-Spektrum folgende Banden aufweist: 3327 cm⁻¹, 1666 cm⁻¹, 1448 cm⁻¹, 1379 cm⁻¹, 999 cm⁻¹.

   Eine Lösung von 1 g (0,0045 Mol) (2Z,6Z)-3,7,11-Trimethyl-2,6,11-dodecatrien-1-ol in 32 ml 3,4-Dihydro-2H-pyran wird bei 0° mit 91 mg p-Toluolsulfonsäure versetzt. Die Lösung wird 2 Stunden bei gleicher Temperatur unter Argon gerührt. Nach Zugabe von 100 ml Wasser wird mit Aether extrahiert. Die organische Phase wird mit Natriumbicarbonatlösung gewaschen. Nach dem Trocknen und Entfernen der Lösungsmittel wird mit Aether-Hexan 1:1 an Silicagel chromatographiert. Man erhält Tetrahydro-2-[[(2Z,6Z)-3,7,11-trimethyl-2,6,10 -dodecatrienyl]oxy]-2H-pyran, das im IR-Spektrum folgende Banden aufweist: 1668 cm⁻¹, 1444 cm⁻¹, 1378 cm⁻¹, 1118 cm⁻¹, 1075 cm⁻¹, 1023 cm⁻¹.

   Eine Lösung von 2 g (0,0065 Mol) Tetrahydro-2-[[(2Z,6Z)-3,7,11-trimethyl-2,6,10 -dodecatrienyl]oxy]-2H-pyran, gelöst in 13 ml Monoglym und 2,6 ml Wasser, wird bei -10° portionenweise mit 1,3 g N-Bromsuccinimid versetzt. Anschliessend wird 1/4 Stunde bei 0° und Stunden bei Raumtemperatur gerührt. Die Reaktionslösung wird auf 100 ml Wasser gegossen und mit Essigester extrahiert. Die organische Phase wird mit gesättigter Kochsalzlösung gewaschen. Nach dem Trocknen und Entfernen der Lösungsmittel wird der Rückstand, enthaltend Tetrahydro-2-[[(2Z,6Z)-10-brom-11-hydroxy-3,7,11-trimethyl-2,6,10-dodecatrienyl]oxy]-2H-pyran, in 18 ml Methanol gelöst und mit 600 mg Kaliumcarbonat versetzt. Die Suspension wird 1,5 Stunden bei Raumtempe ratur unter Argon gerührt. Nach dem Entfernen des Methanols am Rotationsverdampfer wird mit 500 ml Aether und 200 ml Wasser versetzt. Die organische Phase wird mit gesättigter Kochsalzlösung gewaschen. Nach dem Trocknen und Entfernen der Lösungsmittel wird der Rückstand, enthaltend (6Z,10Z)-2,3-Epoxy-2,6,10-trimethyl-12-[(tetrahydro-2H-pyran -2-yl)-oxy]-6,10-dodecadien, in 30 ml Toluol gelöst und mit 1,35 g Aluminiumisopropylat versetzt. Das Reaktionsgemisch wird während 17 Stunden am Rückfluss gekocht. Nach Abkühlen auf Raumtemperatur wird das Reaktionsgemisch auf 200 ml Eis-Wassergemisch gegossen. Nach dem Ansäuren mit 1N-Salzsäure wird mit 3 × 150 ml Hexan extrahiert. Die organische Phase wird mit 500 ml gesättigter Kochsalzlösung gewaschen. Nach dem Trocknen und Entfernen der Lösungsmittel wird der Rückstand mit einem 1:2 Aether-Hexangemisch, das zusätzlich 0,1% Triäthylamin enthält, an Silicagel chromatographiert. Man erhält (6Z,10Z)-2,6,10-Trimethyl-12-[(tetrahydro-2H-pyran -2-yl)oxy]-1,6,10-dodecatrien-3-ol, das im IR-Spektrum folgende Banden aufweist: 3449 cm⁻¹, 1647 cm⁻¹, 1449 cm⁻¹, 1377 cm⁻¹, 901 cm⁻¹.
b) Eine Lösung von 0,5 g (0,0016 Mol) (6Z,10Z)-2,6,10-Trimethyl-12-[(tetrahydro-2H-pyran -2-yl)oxy]-1,6,10-dodecatrien-3-ol, gelöst in 14,5 ml Tetrahydrofuran und 3,7 ml Wasser, wird mit 167,2 mg (0,4 Moläquivalente) Pyridinium-p-toluolsulfonat versetzt. Die Lösung wird während 17 Stunden am Rückfluss gekocht. Nach dem Abkühlen werden 100 ml Wasser und 300 ml Aether zugegeben. Die organische Phase wird mit Natriumbicarbonatlösung neutralisiert. Nach dem Trocknen und Entfernen der Lösungsmittel wird der Rückstand mit Aether-Hexan 1:1 an Silicagel chromatographiert. Man erhält (6Z,10Z)-2,6,10-Trimethyl-1,6,10-dodecatrien-3, 12-diol, das im IR-Spektrum folgende Banden aufweist: 3348 cm⁻¹, 1646 cm⁻¹, 1443 cm⁻¹, 1375 cm⁻¹, 998 cm⁻¹.

### Beispiel 8

Eine Lösung von 0,5 g (0,0016 Mol) (6Z,10Z)-2,6,10-Trimethyl-12-[(tetrahydro-2H-pyran -2-yl)oxy]-1,6,10-dodecatrien-3-ol, gelöst in 15 ml Methylenchlorid, wird mit 10 g Mangandioxid versetzt. Die Suspension wird 18 Stunden bei Raumtemperatur unter Argon gerührt. Nach dem Filtrieren und Entfernen des Lösungsmittels wird der Rückstand mit Aether-Hexan 10:1 an Silicagel chromatographiert. Das Produkt, enthaltend (6Z,10Z)-2,6,10-Trimethyl-12-[(tetrahydro-2H-pyran -2-yl)oxy]-1,6,10-dodecatrien-3-on, wird in 10 ml Tetrahydrofuran und 2,6 ml Wasser gelöst und mit 118 mg Pyridinium-p-toluolsulfonat versetzt. Die Lösung wird während 17 Stunden Rückfluss gekocht. Nach dem Abkühlen werden 100 ml Wasser und 200 ml Aether zugegeben. Die organische Phase wird mit Natriumbicarbonatlösung neutralisiert. Nach dem Trocknen und Entfernen der Lösungsmittel wird der Rückstand mit Aether-Hexan 1:1 an Silicagel chromatographiert. Man erhält (6Z,10Z)-12-Hydroxy-2,6,10-trimethyl-1,6,10-dodecatrien-3-on, das folgende Absorptionsbanden im IR-Spektrum zeigt: 3387 cm⁻¹, 1677 cm⁻¹, 1629 cm⁻¹, 1417 cm⁻¹, 1389 cm⁻¹, 997 cm⁻¹, 950 cm⁻¹.

### Beispiel 9

Eine Lösung von 0,5 g (0,0021 Mol) (6Z,10Z)-12-Hydroxy-2,6,10-trimethyl-1,6,10-dodecatrien-3-on, gelöst in 5 ml Methylenchlorid und 1,3 ml Pyridin, wird mit 1 ml Essigsäureanhydrid versetzt. Die Lösung wird während 18 Stunden bei Raumtemperatur unter Argon gerührt. Nach dem Entfernen der Lösungsmittel wird der Rückstand dreimal in Toluol aufgenommen und jeweils am Rotationsverdampfer vom Lösungsmittel befreit. Der Rückstand wird mit Aether-Hexan 1:5 an Silicagel chromatographiert. Man erhält (2Z,6Z)-3,7,11-Trimethyl-10-oxo-2,6,11-dodecatrienylacetat, das im IR-Spektrum folgende Banden aufweist: 1742 cm⁻¹, 1671 cm⁻¹, 1634 cm⁻¹, 1442 cm⁻¹, 1367 cm⁻¹, 951 cm⁻¹.

### Beispiel 10

a) Eine Lösung von 2,0 g (6E,10Z)-2,6,10-Trimethyl-12-[(tetrahydro-2H-pyran -2-yl)oxy]-1,6,10-dodecatrien-3-ol und 4,23 g Imidazol in 30 ml Dimethylformamid wird mit 4,7 g (5 Moläquivalente) tert.-Butyl-dimethylchlorsilan versetzt. Das Reaktionsgemisch wird während 6 Stunden bei Raumtemperatur unter Argon gerührt. Nach wässeriger Aufarbeitung wird die organische Phase getrocknet und von den Lösungsmitteln befreit. Der Rückstand wird mit Aether-Hexan 1:10 an Silicagel chromatographiert.

   Das Produkt, enthaltend (6E,10Z)-3-(tert.-Butyldimethylsilyloxy)-2,6,10 -trimethyl-12-[(tetrahydro-2H-pyran-2-yl)oxy] -1,6,10-dodecatrien, wird in 50 ml Isoproponal gelöst und mit 350 mg Pyridinium-p-toluolsulfonat versetzt. Die Reaktionslösung wird während 6 Stunden bei Raumtemperatur unter Argon gerührt. Nach wässeriger Aufarbeitung wird die organische Phase getrocknet und von den Lösungsmitteln befreit. Der Rückstand wird mit Aether-Hexan 2:1 an Silicagel chromatographiert. Man erhält (2Z,6E)-10-(tert.-Butyldimethylsilyloxy)-3,7,11-trimethyl -2,6,11-dodecatrien-1-ol, das folgende Absorptionsbanden im IR-Spektrum zeigt: 3339 cm⁻¹, 3071 cm⁻¹, 1666 cm⁻¹, 1651 cm⁻¹, 1448 cm⁻¹, 1251 cm⁻¹, 1078 cm⁻¹, 1022 cm⁻¹, 1004 cm⁻¹.
b) Eine Lösung von 200 mg (2Z,6E)-10-(tert.-Butyldimethylsilyloxy)-3,7,11-trimethyl -2,6,11-dodecatrien-1-ol in 5 ml Methylenchlorid wird mit 0,4 ml Triäthylamin und 0,2 ml Essigsäureanhydrid versetzt. Die Lösung wird während 6 Stunden bei Raumtemperatur gerührt und anschliessend am Rotationsverdampfer von den Lösungsmitteln befreit. Der Rückstand wird dreimal in Toluol aufgenommen und jeweils am Rotationsverdampfer von flüchtigen Anteilen befreit. Das Rohprodukt wird mit Aether-Hexan 1:5 an Silicagel chromato graphiert.

   Das erhaltene gereinigte Produkt, (2Z,6E)-10-(tert.-Butyldimethylsilyloxy)-3,7,11-trimethyl -2,6,11-dodecatrienylacetat, wird in 5 ml Tetrahydrofuran gelöst und mit 320 mg Tetrabutylammoniumfluorid versetzt. Die Lösung wird während 16 Stunden bei Raumtemperatur gerührt. Nach dem Entfernen des Lösungsmittels am Rotationsverdampfer wird der Rückstand mit Aether-Hexan 1:1 an Silicagel chromatographiert. Man erhält (2Z,6E)-10-Hydroxy-3,7,11-trimethyl-2,6,11 -dodecatrienylacetat, das folgende Banden im IR-Spektrum zeigt: 3454 cm⁻¹, 1739 cm⁻¹, 1651 cm⁻¹, 1446 cm⁻¹, 1236 cm⁻¹, 1023 cm⁻¹, 955⁻¹, 898 cm⁻¹.

### Beispiel 11

Eine Lösung von 3,5 g (0,0148 Mol) (6E,10Z)-12-Hydroxy-2,6,10-trimethyl-1,6,10-dodecatrien-3-on in 110 ml Methanol wird mit 7,15 g g N-(2-Acetylthioäthyl)acetamid und 6,2 ml Triäthylamin versetzt. Die Lösung wird während 24 Stunden bei Raumtemperatur unter Argon gerührt. Die Lösungsmittel werden am Rotationsverdampfer entfernt. Der Rückstand wird dreimal mit Toluol aufgenommen und am Rotationsverdampfer vom Lösungsmittel befreit. Das Rohprodukt kann direkt für die in Beispiel 12 beschriebene Acetylierung eingesetzt oder mit Aether-Methanol 50:1 an Silicagel chromatographiert werden. Nach der chromatographischen Reinigung erhält man (2Z,6E)-12-[(Acetamidoäthyl)thio]-3,7,11-trimethyl-10-oxo -2,6-dodecadien-1-ol, das folgende Banden im IR-Spektrum zeigt: 1737 cm⁻¹, 1715 cm⁻¹, 1437 cm⁻¹, 1366 cm⁻¹, 1277 cm⁻¹, 1235 cm⁻¹, 1157 cm⁻¹, 1136 cm⁻¹, 1022 cm⁻¹.

### Beispiel 12

Das gemäss Beispiel 11 erhaltene rohe (2Z,6E)-12-[(2 -Acetamidoäthyl)thio]-3,7,11-trimethyl-10-oxo -2,6-dodecadien-1-ol wird bei 0° in 160 ml Methylenchlorid gelöst und mit 32 ml Essigsäureanhydrid und 42 ml Pyridin versetzt. Die Lösung wird während 20 Stunden bei Raumtemperatur unter Argon gerührt. Die Lösungsmittel werden am Wasserstrahlvakuum entfernt. Der Rückstand wird dreimal mit Toluol aufgenommen und jeweils am Rotationsverdampfer vom Lösungsmittel befreit. Das Rohprodukt wird mit Aether-Methanol 50:1 an Silicagel chromatographiert. Man erhält (2Z,6E)-12-[(2-Acetamidoäthyl)thio]-3,7,11-trimethyl -10-oxo-2,6-dodecadienylacetat, das im IR-Spektrum folgende Absorptionsbanden aufweist: 1736 cm⁻¹, 1712 cm⁻¹, 1658 cm⁻¹, 1545 cm⁻¹, 1375 cm⁻¹, 1235 cm⁻¹, 1023 cm⁻¹, 992 cm⁻¹.

### Beispiel 13

Eine Lösung von 300 mg (0,0013 Mol) (6E,10Z)-12-Hydroxy-2,6,10-trimethyl-1,6,10-dodecatrien-3-on in 10 ml Methanol wird mit 0,27 ml 2-Mercapto-äthanol und 0,53 ml Triäthylamin versetzt. Die Lösung wird während 18 Stunden bei Raumtemperatur unter Argon gerührt. Nach dem Entfernen des Lösungsmittels am Rotationsverdampfer wird der Rückstand mit Wasser und Methylenchlorid versetzt. Die organische Phase wird nach dem Ausschütteln getrocknet und am Rotationsverdampfer vom Lösungsmittel befreit. Der Rückstand wird mit Aether-Methanol 50:1 an Silicagel chromatographiert. Man erhält (6E,10Z)-12-Hydroxy-1-[(2-hydroxyäthyl)thio]-2,6,10 -trimethyl-6,10-dodecadien-3-on, das im IR-Spektrum folgende Absorptionsbanden aufweist: 3369 cm⁻¹, 1708 cm⁻¹, 1667 cm⁻¹, 1450 cm⁻¹, 1044 cm⁻¹, 1005 cm⁻¹.

### Beispiel 14

Eine Lösung von 300 mg (0,00096 Mol) (6E,10Z)-12-Hydroxy-1-[(2-hydroxyäthyl)thio]-2,6,10-trimethyl -6,10-dodecadien-3-on in 10 ml Methylenchlorid wird mit 1,1 ml Pyridin und 0,9 ml Essigsäureanhydrid versetzt. Die Lösung wird 18 Stunden bei Raumtemperatur unter Argon gerührt. Nach dem Entfernen der Lösungsmittel wird der Rückstand dreimal in Toluol aufgenommen und am Rotationsverdampfer vom Lösungsmittel befreit. Der Rückstand wird mit Aether an Silicagel chromatographiert. Man erhält (2Z,6E)-12-[2-Acetoxyäthyl)thio]-3,7,11-trimethyl -2,6-dodecadienylacetat, das folgende Absorptionsbanden im IR-Spektrum aufweist: 1740 cm⁻¹, 1712 cm⁻¹, 1450 cm⁻¹, 1379 cm⁻¹, 1235 cm⁻¹, 1025 cm⁻¹, 957 cm⁻¹.

### Beispiel 15

Eine Lösung von 4 g (0,0017 Mol) (6E,10Z)-12-Hydroxy-2,6,10-trimethyl-1,6,10-dodecatrien-3-on in 130 ml Methanol wird mit 4,7 ml Thioglykolsäuremethylester und 7,1 ml Triäthylamin versetzt. Die Lösung wird während 18 Stunden bei Raumtemperatur unter Argon gerührt. Nach dem Entfernen des Lösungsmittels wird der Rückstand mit Wasser und Methylenchlorid aufgearbeitet. Die organische Phase wird getrocknet und vom Lösungsmittel befreit. Der Rückstand wird mit Aether an Silicagel chromatographiert. Man erhält Methyl [[(6E,10Z)-12-hydroxy-2,6,10-trimethyl-3-oxo -6,10-dodecadienyl]thio]acetat, das im IR-Spektrum folgende Banden aufweist: 1736 cm⁻¹, 1711 cm⁻¹, 1437 cm⁻¹, 1408 cm⁻¹, 1281 cm⁻¹, 1137 cm⁻¹, 1007 cm⁻¹.

### Beispiel 16

Eine Lösung von 300 mg (0,00088 Mol) Methyl [[(6E,10Z)-12-hydroxy-2,6¸10-trimethyl-3-oxo -6,10-dodecadienyl]thio]-acetat in 5 ml Methylenchlorid wird mit 0,5 ml Pyridin und 0,4 Essigsäureanhydrid versetzt. Die Lösung wird während 18 Stunden bei Raumtemperatur unter Argon gerührt. Nach dem Entfernen der Lösungsmittel wird der Rückstand dreimal in Toluol aufgenommen und jeweils am Rotationsverdampfer vom Lösungsmittel befreit. Der Rückstand wird mit Aether an Silicagel chromatographiert. Man erthält (2Z,6E)-12 -[[(Methoxycarbonyl)methyl]thio]-3,7,11-trimethyl -10-oxo-2,6-dodecadienylacetat, das folgende Banden im IR-Spektrum zeigt: 1710 cm⁻¹, 1657 cm⁻¹, 1552 cm⁻¹, 1445 cm⁻¹, 1375 cm⁻¹, 1290 cm⁻¹, 1235 cm⁻¹.

### Beispiel 17

Eine Lösung von 4 g(0,017 Mol) (6Z,10E)-12-Hydroxy-2,6,10-trimethyl-1,6,10-dodecatrien-3-on in 100 ml Methylenchlorid wird mit 6 ml Thiossigsäure und 4,7 ml Triäthylamin versetzt. Die Lösung wird 21 Stunden bei Raumtemperatur unter Argon gerührt. Nach dem Entfernen des Lösungsmittels wird mit Wasser und Methylenchlorid versetzt. Nach dem Ausschütteln wird die organische Phase getrocknet und vom Lösungsmittel befreit. Der Rückstand wird mit Aether an Silicagel chromatographiert. Man erhält S-[(6Z,10E)-12-Hydroxy-2,6,10-trimethyl-3-oxo, -6,10-dodecadienyl]thioacetat, das im IR-Spektrum folgende Banden aufweist: 3414 cm⁻¹- 1694 cm⁻¹, 1454 cm⁻¹, 1135 cm⁻¹, 1107 cm⁻¹.

### Beispiel 18

Eine Lösung von 3,5 g (0,0015 Mol) (6Z,10Z)-12-Hydroxy-2,6,10-trimethyl-1,6,10-dodecatrien-3-on in 110 ml Methanol wird mit 9,7 g S-[(2-Acetamido-2-methoxycarbonyl)äthyl]thioacetat und 6,2 ml Triäthylamin versetzt. Die Lösung wird während 24 Stunden bei Raumtemperatur gerührt. Nach dem Entfernen des Lösungsmittels wird der Rückstand dreimal in Toluol aufgenommen und jeweils am Rotationsverdampfer vom Lösungsmittel befreit. Der Rückstand wird mit Aether-Methanol 50:1 an Silicagel chromatographiert. Man erhält N-Acetyl-3-[[(6Z,10Z)-12-hydroxy-2,6,10-trimethyl-3-oxo-6,10-dodecadienyl]thio]alaninmethylester, der im IR-Spektrum folgende Banden aufweist: 3289 cm⁻¹, 1746 cm⁻¹, 1711 cm⁻¹, 1661 cm⁻¹, 1541 cm⁻¹, 1438 cm⁻¹, 1374 cm⁻¹, 1214 cm⁻¹.

### Beispiel 19

Eine Lösung von 3,5 g N-Acetyl-3-[[6Z,10Z)-12-hydroxy-2,6,10-trimethyl-3-oxo -6,10-dodecadienyl]thio]alaninmethylester, gelöst in 150 ml Methylenchlorid, wird bei 0° mit 39 ml Pyridin und 30 ml Essigsäureanhydrid versetzt. Die Lösung wird während 20 Stunden bei Raumtemperatur gerührt. Nach dem Entfernen des Lösungsmittels wird der Rückstand dreimal in Toluol aufgenommen und jeweils am Rotationsverdampfer vom Lösungsmittel befreit. Das Rohprodukt wird mit Aether-Methanol 50:1 an Silicagel chromatographiert. Man erhält N-Acetyl-3-[[(6Z,10Z)-12-acetoxy-2,6,10-trimethyl-3--oxo -6,10-dodecadienyl]thio]alaninmethylester, der folgende Absorptionsbanden im IR-Spektrum zeigt: 1738 cm⁻¹, 1713 cm⁻¹, 1664 cm⁻¹, 1438 cm⁻¹, 1412 cm⁻¹, 1236 cm⁻¹, 1177 cm⁻¹.

### Beispiel 20

a) Eine Lösung von 5 g E-Geranylaceton in 90 ml Dimethoxyäthan wird bei -20° mit 25,1 g Phosphonoacetamid-diäthylester und 1,86 g 50%iger Natriumhydriddispersion in Mineralöl versetzt. Das Reaktionsgemisch wird während 3,5 Stunden bei Raumtemperatur unter Argon gerührt. Nach Wasserzugabe wird mit Essigester extrahiert. Die organische Phase wird getrocknet und vom Lösungsmittel befreit. Kristallisation des als Rückstand verbleibenden (2E/Z,6E)-3,7,11-Trimethyl-2,6,10-dodecatrien-amids (4:1 2E/Z-Gemisch) aus Hexan gibt weisse Kristalle, Smp. 51-52,5°. Dieses Material wird noch zweimal aus Hexan umkristallisiert, wobei reines (E,E)-3,7,11-Trimethyl-2,6,10-dodecatrienamid als weisses kristallines Material vom Smp. 53-53.5° erhalten werden.
b) Zu einer Mischung von 9,4 g (E,E)-3,7,11-Trimethyl-2,6,10-dodecatrienamid in 80 ml 1,2-Dimethoxyäthan und 20 ml Wasser werden bei 0° innerhalb von 20 Minuten portionenweise 8,0 g N-Bromsuccinimid zugegeben. Die Reaktionsmi schung wird 1 Stunde bei 0° und 2 Stunden bei Raumtemperatur gerührt und dann auf eine Mischung von 500 ml gesättigter Kochsalzlösung und Eis gegossen, worauf man mit 2 × 300 ml Aether extrahiert. Die vereinigten Extrakte werden mit gesättigter Kochsalzlösung und Wasser gewaschen, über Natriumsulfat getrocknet, filtriert und eingeengt. Man erhält rohes (E,E)-10-Brom-11-hydroxy-3,7,11-trimethyl-2,6-dodecadienamid als gelbes Oel.

   Dieses Oel wird in 50 ml Methanol gelöst, worauf man mit 5,66 g Kaliumcarbonat versetzt und 3 Stunden bei Raumtemperatur rührt. Nach Zugabe von 500 ml gesättigter Kochsalzlösung wird mit 2 × 300 ml extrahiert, worauf die vereinigten Extrakte mit gesättigter Kochsalzlösung und Wasser gewaschen, über Natriumsulfat getrocknet, filtriert und eingedampft werden. Das erhaltene gelbe Oel wird an 500 g Kieselgel mit Hexan-Aether (25% bis 100%)/0.1% Triäthylamin als Elutionsmittel chromatographiert. Man erhält (E,E)-10,11-Epoxy-3,7,11-trimethyl-2,6-dodecadienamid als gelbliches Oel. IR (Film): 3398, 3338, 3192 (NH₂); 1672 (Amid-C=O). MS (CI mit NH₃): 252 (M+H)⁺.
c) Eine Lösung von 4,8 g (E,E)-10,11-Epoxy-3,7,11-trimethyl-2,6-dodecadienamid in 70 ml Toluol wird mit 4,48 g Aluminiumisopropylat versetzt, worauf die Mischung 4,5 Stunden unter Rückfluss gekocht wird. Das abgekühlte Reaktionsgemisch wird auf ein Gemisch von 2N Salzsäure und Eis gegossen, worauf man mit 2 × 100 ml Aether extrahiert. Die vereinigten organischen Auszüge werden mit Wasser und mit gesättigter Natriumbicarbonatlösung gewaschen, über Natriumsulfat getrocknet, filtriert und eingedampft. Der Rückstand wird an 230 g Aluminiumoxid (basisch, Aktivität III) chromatographiert, wobei mit Hexan-Essigsäureäthylester (20% bis 100%) und mit Essigsäureäthylester-Methanol (10%) eluiert wird. Man erhält (E,E)-10-Hydroxy-3,7,11-trimethyl-2,6,11-dodecatrienamid als gelbliches, viskoses Oel. IR (Film): 3335, 3195 (OH und NH₂); 1668 (Amid-C=O). MS (CI mit NH₃): 252 (M+H)⁺; 251 (M+NH₄-H₂O)⁺.

### Beispiel 21

Eine Lösung von 1,9 g (E,E)-10-Hydroxy-3,7,11-trimethyl-2,6,11-dodecatrienamid in 50 ml Methylenchlorid wird mit 19,0 Mangandioxid ("gefällt", aktiviert durch Erhitzen auf 110° während 15 Minuten versetzt, und die resultierende Suspension wird 16 Stunden bei Raumtemperatur gerührt. Nach Filtration über Hyflo wird eingeengt, und das erhaltene gelbe Oel wird an 100 g Aluminiumoxid (neutral, Aktivität III) mit Hexan-Essigsäureäthylester (50% bis 100%) chromatographiert. Es wird (E,E)-3,7,11-Trimethyl-10-oxo-2,6,11-dodecatrienamid als farbloses Oel gewonnen, welches beim Stehenlassen kristallisiert, Smp. 42-44°. IR (Film): 3428, 3347, 3194 (NH₂); 1671 (Amid-C=O, Keton konj. C=O). MS (CI mit NH₃): 250 (M+H)⁺.

### Beispiel 22

Eine Lösung von 4,1 g (0,016 Mol) (E,E)-3,7,11-Trimethyl-10-oxo-2,6,11-dodecatrienamid in 125 ml Methanol wird mit 3,5 ml 2-Mercaptoäthanol und 7 ml Triäthylamin versetzt. Die Lösung wird während 18 Stunden bei Raumtemperatur gerührt. Nach dem Entfernen des Lösungsmittels wird der Rückstand dreimal in Toluol aufgenommen und am Rotationsverdampfer vom Lösungsmittel befreit. Der Rückstand wird mit Aether-Methanol 10:1 an Silicagel chromatographiert. Man erhält (2E,6E)-12-(2-Hydroxyäthyl)-3,7,11-trimethyl-10-oxo-2,6-dodecadienamid, das im IR-Spektrum folgende Absorptionsbanden zeigt: 3340 cm⁻¹, 3201 cm⁻¹, 2924 cm⁻¹, 1707 cm⁻¹, 1669 cm⁻¹, 1608 cm⁻¹, 1409 cm⁻¹, 1369 cm⁻¹, 1305 cm⁻¹, 1046 cm⁻¹, 1012 cm⁻¹.

### Beispiel 23

Eine Lösung von 1,75 g (2E,6E)-12-(2-Hydroxyäthyl) -3,7,11-trimethyl-10-oxo -2,6-dodecadienamid, gelöst in 87 ml Methylenchlorid, wird bei 0° mit 1,02 g m-Chlorperbenzoesäure versetzt. Die Lösung wird während 1,5 Stunden bei 0° unter Argon gerührt. Die Reaktionslösung wird mit wässeriger Kaliumcarbonatlösung gewaschen. Nach dem Trocknen und Entfernen des Lösungsmittels wird Rückstand mit Aether-Methanol 1:1 an Silicagel chromatographiert. Man erhält (2E,6E)-12-[(2-Hydroxyäthyl)sulfinyl]-3,7,11-trimethyl -10-oxo-2,6-dodecadienamid, das im IR-Spektrum folgende Banden zeigt: 3335 cm⁻¹, 3201 cm⁻¹, 3201 cm⁻¹, 1710 cm⁻¹, 1669 cm⁻¹, 1639 cm⁻¹, 1438 cm⁻¹, 1407 cm⁻¹, 1369 cm⁻¹, 1020 cm⁻¹, 995 cm⁻¹.

### Beispiel 24

Eine Lösung von 2,0 g (0,008 Mol) (E,E)-3,7,11-Trimethyl-10-oxo-2,6,11-dodecatrienamid in 60 ml Methanol wird mit 2,3 ml Thioglykolsäuremethylester und 3,4 ml Triäthylamin versetzt. Die Lösung wird während 8 Stunden bei Raumtemperatur unter Argon gerührt. Nach dem Entfernen des Lösungsmittels wird der Rückstand in Wasser und Methylenchlorid aufgenommen. Nach dem Ausschütteln wird die organische Phase getrocknet und vom Lösungsmittel befreit. Der Rückstand wird mit Aether-Methanol 20:1 an Silicagel chromatographiert. Man erhält Methyl [[(6E,10E)-11-carbamoyl-2,6,10-trimethyl-3-oxo -6,10-undecadienyl]thio]acetat, das im IR-Spektrum folgende Absorptionsbanden zeigt: 3447 cm⁻¹, 3352 cm⁻¹, 3195 cm⁻¹, 1735 cm⁻¹, 1712 cm⁻¹, 1669 cm⁻¹, 1638 cm⁻¹, 1609 cm⁻¹, 1436 cm⁻¹, 1300 cm⁻¹, 1137 cm⁻¹, 1008 cm⁻¹.

### Beispiel 25

Eine Lösung von 500 mg (0,0014 Mol) Methyl [[(6E,10E)-11-carbamoyl-2,6,10-trimethyl-3-oxo -6,10-undecadienyl]thio]acetat in 30 ml Methylenchlorid wird bei 0° mit 270 mg m-Chlorperbenzoesäure versetzt. Die Lösung wird während 1 Stunde bei 0° unter Argon gerührt. Die Reaktionslösung wird mit wässeriger Kaliumcarbonatlösung gewaschen. Nach dem Trocknen und Entfernen des Lösungsmittels wird der Rückstand mit Aether-Methanol 3:1 an Silicagel chromatographiert. Man erhält Methyl [[(6E,10E)-11-carbamoyl-2,6,10-trimethyl-3-oxo -6,10-undecadienyl]sulfinyl]acetat, das im IR-Spektrum folgende Absorptionsbanden zeigt: 3329 cm⁻¹, 3197 cm⁻¹, 1734 cm⁻¹, 1671 cm⁻¹, 1639 cm⁻¹, 1610 cm⁻¹, 1439 cm⁻¹, 1307 cm⁻¹, 1218 cm⁻¹, 1193 cm⁻¹, 1148 cm⁻¹, 1016 cm⁻¹.

### Beispiel 26

a) Zu einer Mischung aus 32 g (0,156 Mol) Aluminiumisopropylat und 300 ml Toluol werden bei 110° 43, 6 g (0,155 Mol) (all-E)-10,11-Epoxy-3,7,11-trimethyl -2,6-dodecadiensäureäthylester getropft. Die Reaktionsmischung wird noch 12 Stunden unter Rückfluss gekocht und dann in Eiswasser gegossen, worauf man mit 2N Salzsäure neutralisiert und mit Hexan extrahiert. Die vereinigten organischen Phasen werden zuerst mit gesättigter Kochsalzlösung gewaschen, dann über Natriumsulfat getrocknet und eingedampft. Zur Reinigung wird der Rückstand mit Hexan-Essigester 4:1 an Kieselgel chromatographiert, wobei (all-E)-10-Hydroxy-3,7,11-trimethyl-2,6,11-dodecatriensäureisopropylester erhalten wird. CI-MS (NH₃): 295 (M+H)⁺, 277 (M+H-H₂O)⁺, 235 (M+H-<)⁺.
b) 4,8 g (0,016 Mol) (all-E)-10-Hydroxy-3,7,11-trimethyl-2,6,11 -dodecatriensäureisopropylester und 45 ml 1N Natronlauge werden in 35 ml Dioxan während 2,5 Stunden unter Rückfluss gekocht. Anschliessend lässt man die Reaktionsmischung erkalten und giesst sie in 250 ml Eiswasser. Die wässrige Phase wird zweimal mit Aether gewaschen, mit 25%iger Salzsäure angesäuert und mit Aether extrahiert. Die erhaltene Aetherphase wird mit gesättigter Kochsalzlösung gewaschen über Natriumsulfat getrocknet und eingedampft. Durch Chromatographieren des Rückstands an Kieselgel unter Eluieren mit einer Elutionsmischung aus Hexan-Essigester 2:1 welcher 0,1% Triäthylamin zugesetzt wird, erhält man (all-E)-10-Hydroxy-3,7,11-trimethyl-2,6,11-dodecatriensäure. IR (Film): 3360 (OH); 2670, 2576, 1249 (CO₂H); 1691 (Säure-C=O); 1642 (C=C, konjugiert); 900 (c=CH₂).

### Beispiel 27

5,0 g (0,02 Mol) (all-E)-10-Hydroxy-3,7,11-trimethyl-2,6,11-dodecatriensäure werden zusammen mit 10,0 g Braunstein (gefällt aktiv) in 100 ml Methylenchlorid während 6 Stunden bei Raumtemperatur gerührt, worauf man durch Hyflo abnutscht und das Filtrat eindampft. Der Rückstand wird an Kieselgel mit Hexan-Essigester 1:1 unter Zusatz von 0,1% Triäthylamin chromatographiert. Man erhält (all-E)-10-Oxo-3,7,11-trimethyl-2,6,11-dodecatriensäure. IR (Film) : 2559, 2482, 1254 (CO₂H), 1676 (Säure C=O; C=O, konjugiert), 1641 (C=C, konjugiert).

### Beispiel 28

Zu 360 ml einer 20%igen Lösung von Diisobutylaluminiumhydrid (ca. 0,36 Mol) in Hexan werden bei 0° innerhalb von 30 Minuten 31,55 g (all-E)-10-Hydroxy-3,7,11-trimethyl-2,6,11-dodecatriensäureisopropylester (0,107 Mol) zugetropft. Anschliessend wird noch während 1 Stunde bei 0° gerührt. Dann werden bei derselben Temperatur langsam 50 ml Methanol zugegeben, worauf die Mischung auf 1,5 l Eiswasser gegossen wird. Anschliessend wird mittels 2N Schwefelsäure schwach angesäuert, worauf man die organische Phase abtrennt und die wässrige Phase dreimal mit je 300 ml Aether extrahiert. Die vereinigten Hexan- und Aetherphasen werden mit 500 ml gesättigter Kochsalzlösung gewaschen, über 50 g Natriumsulfat getrocknet und eingedampft. Das als Rückstand verbleibende Rohprodukt wird über 250 g Kieselgel mit ca. 2 l Essigester-Hexan 4:1 filtriert. Nach Eindampfen und Trocknen am Hochvakuum erhält man (all-E)-3,7,11-Trimethyl- 2,6,11-dodecatrien-1,10-diol als farbloses Oel, welches beim Aufbewahren im Tiefkühlschrank kristallisiert (Smp. <30°).

### Beispiel 29

Eine Mischung von 13 g (all-E)-3,7,11-Trimethyl-2,6,11-dodecatrien-1,10-diol und 140 g Braunstein wird in 1 l n-Hexan während 1,5 Stunden bei Raumtemperatur gerührt. Nach Filtration und Eindampfen erhält man 11,53 g Rohprodukt, welches an Kieselgel zuerst mit Hexan-Essigester 2:1, dann mit Hexan-Essigester 1:1 unter Zusatz von 0,1% Triäthylamin chromatographiert wird. Man erhält (all-E)-10-Hydroxy-3,7,11-trimethyl-2,6,11-dodecatrienal. IR (Film): 3440 (OH), 1670 (Aldehyd, konjugiert), 897 (C=CH₂). MS: 218 (M-H₂O)+.

### Beispiel 30

Zu einer Suspension von 260 g Braunstein (aktiv gefällt) und 0,8 l Methylenchlorid werden bei Raumtemperatur 26,0 g (all-E)-3,7,11-Trimethyl-2,6,11-dodecatrien-1,10-diol zugetropft. Nach 3-stündigem Rühren filtriert man durch Natriumsulfat und engt ein. Der Rückstand wird unter Rühren zu einer Suspension von 260 g Braunstein (aktiv gefällt) und 0,8 l Methylenchlorid getropft. Anschliessend wird nochmals 2 Stunden bei Raumtemperatur gerührt. Nach Filtration und Einengen wird ein Rohprodukt erhalten, welches an Kieselgel mit Hexan-Essigester 2:1 unter Zusatz von 0,1% Triäthylamin chromatographiert wird. Man erhält (all-E)-10-Oxo-3;7,11-trimethyl-2,6,11-dodecatrienal. MS: 205 (M-CHO)⁺.

### Beispiel 31

Zu einer Grignardlösung, hergestellt aus 1,8 g (74 mMol) Magnesium und 10,24 (72,6 mMol) Methyljodid in 50 ml absolutem Aether, werden bei 0° 4,33 g (18,3 mMol) (all-E) -10-Hydroxy-3,7,11-trimethyl-2,6,11-dodecatrienal getropft. Nach beendeter Zugabe wird über Nacht bei Raumtemperatur gerührt. Dann gibt man 40 ml einer gesättigten Lösung von Ammoniumchlorid zu und rührt heftig, wobei eine klare Lösung entsteht. Diese Lösung wird in 500 ml einer gesättigten Lösung von Ammoniumchlorid gegossen, worauf man dreimal mit Aether extrahiert. Nach Waschen der vereinigten organischen Phasen mit gesättigter Kochsalzlösung und Filtration durch Natriumsulfat wird eingedampft. Der Rückstand wird an Kieselgel mit Hexan-Essigester 4:1 unter Zusatz von 0,1% Triäthylamin chromatographiert. Man erhält (all-E)-4,8,12-Trimethyl-3,7,12-tridecatrien-2,11,diol. MS: 234 (M-H₂O)⁺ 216 (M-2H₂O)⁺.

### Beispiel 32

Zu einer Mischung von 0,81 g (11,7 mMol) Hydroxylaminhydrochlorid, 0,98 (12 mMol) Natriumacetat, 10 ml Wasser und 50 ml Methanol wird bei 0° eine Lösung von 2,64 g (11,2 mMol) (all-E)-10-Oxo-3,7,11-trimethyl-2,6,11-dodecatrienal getropft. Anschliessend wird noch 30 Minuten bei 0° gerührt. Dann wird Reaktionsmischung eingeengt, worauf man den Rückstand in 100 ml Aether aufnimmt und mit 200 ml entionisiertem Wasser wäscht. Die organische Phase wird zuerst mit gesättigter Kochsalzlösung gewaschen und dann durch Natriumsulfat filtriert. Nach Eindampfen wird der Rückstand an Kieselgel mit Hexan-Essigester 4:1 unter Zusatz von 0,1% Triäthylamin chromatographiert, wonach (2E,6E)-3,7,11-Trimethyl-10-oxo-2,6,11-dodecatrienal -(E/Z)-oxim erhalten wird. MS: 232 (M-OH)⁺.

### Beispiel 33

a) Eine Mischung von 50 g Braunstein (aktiv gefällt) und 5,0 g (all-E)-10-Hydroxy-3,7,11-trimethyl-2,6,11 -diodecatriensäureisopropylester in 100 ml Methylenchlorid wird während 4 Stunden bei Raumtemperatur gerührt. Dann wird durch Hyflo filtriert, worauf man das Filtrat eindampft und den Rückstand erneut mit 100 ml Methylenchlorid und 50 g Braunstein versetzt. Nach 4-stündigem Rühren wird das Gemisch filtriert und das Filtrat eingedampft, wonach ein farbloses Oel Rückstand verbleibt. 3,1 g dieses Rückstands werden an Kieselgel mit Hexan-Essigester 4:1 unter Zusatz von 0,1% Triäthylamin chromatographiert, wonach (all-E)-10-Oxo-3,7,11-trimethyl-2,6,11 -dodecatriensäureisopropylester erhalten wird. IR (Film): 1711 (Ester-C=O), 1679 (Keton konjugiert -CO), 1224, 1146 (Ester).
b) Zu einer Mischung von 20 g (68 mMol) (all-E)-10-Oxo-3,7,11-trimethyl-2,6,11 -dodecatriensäureisopropylester, 10 ml Methanol und 100 mg p-Toluolsulfonsäure werden bei Raumtemperatur innerhalb von 20 Minuten 10,0 g (94 mMol) Orthoameisensäuretrimethylester getropft. Anschliessend wird noch 4 Stunden bei Raumtemperatur gerührt, und dann wird die Reaktionsmischung in 500 ml einer gesättigten Lösung von Natriumhydrogencarbonat gegossen. Nach dreimaliger Extraktion mit Aether werden die vereinigten organischen Phasen zuerst mit gesättigter Kochsalzlösung gewaschen, dann über Natriumsulfat getrocknet und eingedampft. Als Rückstand verbleibt (all-E)-10,10-Dimethoxy-3,7,11-trimethyl-2,6,11 -dodecatriensäureisopropylester als farbloses Oel.

10,2 g dieses Oels werden in 30 ml absolutem Aether gelöst. Dann werden bei ca. 15° innerhalb von 20 Minuten 72 ml einer 20%igen Lösung von Diisobutylaluminiumhydrid in Toluol zugetropft. Anschliessend wird noch 3 Stunden bei Raumtemperatur weitergerührt. Dann wird die Reaktionsmischung auf 0° abgekühlt und vorsichtig zuerst mit 10 ml Essigester, dann mit 5 ml Methanol und schliesslich mit 50 ml Wasser versetzt und auf eine Mischung von Eis und gesättigter Kochsalzlösung gegossen. Nach zweimaliger Extraktion mit Aether werden die vereinigten organischen Phasen zuerst mit gesättigter Natriumhydrogencarbonatlösung und dann mit gesättigter Kochsalzlösung gewaschen. Nach Trocknen über Natriumsulfat und Eindampfen wird der Rückstand an Kieselgel mit Hexan-Essigester 2:1 unter Zusatz von 0,1% Triäthylamin chromatographiert. Man erhält (all-E)-10,10-Dimethoxy-3,7,11-trimethyl-2,6,11 -dodecatrien-1-ol. MS: 251 (M-OCH₃)⁺.

### Beispiel 34

Eine Mischung von 19,4 g (66 mMol) (all-E)-10-Oxo-3,7,11-trimethyl-2,6,11 -dodecatriensäureisopropylester, 5,0 g (82 mMol) 1,2-Aethandiol, 100 mg p-Toluolsulfonsäure und 50 ml Toluol wird während 5 Stunden unter Rückfluss gekocht. Dann wird die Reaktionsmischung in 500 ml einer gesättigten Lösung von Natriumhydrogencarbonat gegossen, worauf man dreimal mit Aether extrahiert. Die vereinigten organischen Phasen werden mit gesättigter Kochsalzlösung gewaschen, über Natriumsulfat getrocknet, filtriert und eingedampft, wonach (all-E)-3,7-Dimethyl-9-[2-(1-methylvinyl)-1,3-dioxolan-2-yl] -2,6-nonadiensäureisopropylester in Form eines gelblichen Oels erhalten wird.

8,5 g dieses Oels werden in 25 ml absolutem Aether gelöst. Dann werden bei ca. 15° innerhalb von 20 Minuten 60 ml einer 20%igen von Diisobutylaluminiumhydrid in Toluol zugetropft. Nach beendeter Zugabe noch 2,5 Stunden bei Raumtemperatur gerührt. Dann wird die Reaktionsmischung auf 0° abgekühlt und langsam zuerst mit 10 ml Essigester, dann mit 5 ml Methanol und schliesslich mit 50 ml Wasser versetzt und anschliessend auf eine Mischung von Eis und gesättigter Kochsalzlösung gegossen. Nach zweimaliger Extraktion mit Aether werden die vereinigten organischen Phasen zuerst mit gesättigter Natriumhydrogencarbonatlösung und dann mit gesättigter Kochsalzlösung gewaschen. Nach Trocknen über Natriumsulfate, Filtration und Eindampfen wird der Rückstand an Kieselgel mit Hexan-Essigester 2:1 unter Zusatz von 0,1% Triäthylamin chromatographiert, wobei (all-E)-3,7-Dimethyl-9-[2-(1-methylvinyl)-1,3 -dioxolan-2-yl]-2,6-nonadien-1-ol erhalten wird. MS: 239 (M-CH₂=CH-CH₂-)⁺.

### Beispiel 35

Eine Mischung von 38,9 g (0,19 Mol) Aluminiumisopropylat, 50 ml Toluol und 37,4 g (0,15 Mol) 10,11-Epoxy-1-methoxy-3,7,11-trimethyl-(2E,6E)-dodecadien wird während 15 Stunden unter Rückfluss (Badtemperatur 140°) gekocht. Dann wird die Reaktionsmischung in 1,2l einer Eis-Wasser-Mischung gegossen, mit 25%iger Salzsäure angesäuert und mit Hexan extrahiert. Die organische Phase wird zuerst mit gesättigter Natriumbicarbonatlösung und dann mit gesättigter Kochsalzlösung gewaschen, anschliessend über Natriumsulfat getrocknet, filtriert und eingedampft. Zur Reinigung wird der Rückstand mit Hexan-Diäthyläther 7:3 an Kieselgel chromatographiert, wonach (all-E)-12-Methoxy-2,6,10-trimethyl-1,6,10-dodecatrien-3-ol erhalten wird. CI-MS (NH₃): 253 (M+H)⁺, 203 (M+H-(CH₃OH+H₂O))⁺.

### Beispiel 36

Eine Mischung von 5 g (15,8 mMol) (all-E)-12-Methoxy-2,6,10-trimethyl-1,6,10-dodecatrien-3-ol, 20 g Braunstein (aktiv gefällt) und 100 ml Methylenchlorid wird bei Raumtemperatur über Nacht gerührt. Dann filtriert man, versetzt das Filtrat mit 10 g frischem Braunstein, gibt nach 3-stündigem Rühren sowie nach weiteren 1,5 Stunden bzw. 3,5 Stunden nochmals je 10 g Braunstein zu und rührt anschliessend über Nacht weiter. Nach Filtration wird das Filtrat im Rotationsverdampfer eingedampft. Nach Trocknen im Hochvakuum wird (all-E)-12-Methoxy-2,6,10-trimethyl-1,6,10-dodecatrien-3-on erhalten. MS: 218 (M-CH₃OH)⁺.

### Beispiel 37

Eine Mischung von 3,0 g (11 mMol) (all-E/Z)-10,11-Epoxy -3,7,11-trimethyl-1-(2-propynyloxy) -2,6-dodecadien, 3,5 g (18 mMol) Aluminiumisopropylat und 40 ml Toluol wird während 18 Stunden unter Rückfluss gekocht. Dann wird das Reaktionsgemisch in eine Mischung von Eis und 2N Salzsäure gegossen, worauf man zweimal mit Aether extrahiert. Die vereinigten organischen Phasen zuerst mit gesättigter Natriumhydrogencarbonatlösung und dann mit gesättigter Kochsalzlösung gewaschen. Nach Trocknen über Natriumsulfat, Filtration und Einengen wird Rohprodukt erhalten, welches an Kieselgel mit Hexan-Essigester 4:1 unter Zusatz von 0,1% Triäthylamin chromatographiert wird. Man erhält (all-E/Z)-2,6,10-Trimethyl-12-(2-propynyloxy) -1,6,10-dodecatrien-3-ol. CI-MS (NH₃): 277 (M+H)⁺, 259 (M+H-H₂O)⁺, 221 (M+H-HO-CH₂-C≡CH)⁺.

### Beispiel 38

a) Eine Mischung von 5,7 g (20 mMol) (E/Z)-10,11-Epoxy-3,7,11-trimethyl-2-dodecensäureäthylester, 6,7 g (34 mMol) Aluminiumisopropylat und 70 ml Toluol wird während 18 Stunden unter Rückfluss gekocht. Dann wird das Reaktionsgemisch in eine Mischung von Eis und 2N Salzsäure gegossen, worauf man zweimal mit Aether extrahiert. Die vereinigten organischen Phasen werden zuerst mit gesättigter Natriumhydrogencarbonatlösung und dann mit gesättigter Kochsalzlösung gewaschen. Nach Trocknen über Natriumsulfat, Filtration und Eindampfen wird (E/Z)-10-Hydroxy-3,7,11-trimethyl-2,11 -dodecadiensäureäthylester als farbloses Oel erhalten.
b) Zu einer Lösung von 5,9 g (E/Z)-10-Hydroxy-3,7,11-trimethyl-2,11 -dodecadiensäureäthylester in 25 ml absolutem Aether werden bei 10° innerhalb von 30 Minuten 60 ml einer 20% Lösung von Diisobutylaluminiumhydrid getropft. Anschliessend wird noch 2 Stunden bei Raumtemperatur weitergerührt. Dann wird die Reaktionsmischung auf 0° abgekühlt und vorsichtig zuerst mit 6 ml Essigester, dann mit 3 ml Methanol und dann mit 25 ml 25% Salzsäure versetzt und anschliessend auf eine Mischung von Eis und gesättigter Kochsalzlösung gegossen. Nach zweimaliger Extraktion mit Aether werden die vereinigten organischen Phasen zuerst mit gesättigter Natriumhydrogencarbonatlösung und dann mit gesättigter Kochsalzlösung gewaschen, über Natriumsulfat getrocknet, filtriert und eingedampft. Der Rückstand wird an Kieselgel mit Hexan-Essigester 1:1 unter Zusatz von 0,1% Triäthylamin chromatographiert, wonach (E/Z)-3,7,11-Trimethyl-2,11-dodecadien-1,10-diol als farbloses Oel erhalten wird. MS: 222 (M-H₂O)⁺, 189 (M-H₂O)⁺, 189 (M-(2 H₂O+CH₃))⁺.

### Beispiel 39

Eine Mischung von 22,0 g (71 mMol) Tetrahydro-2-[(3,7,11-trimethyl-6,10-dodecadienyl)oxy] -2H-pyran, 80 ml 1,2-Dimethoxyäthan und 16 ml Wasser wird bei -5° innerhalb von 30 Minuten mit 13,5 g (76 mMol) N-Bromsuccinimid versetzt. Anschliessend wird noch während 2 Stunden bei 0° und 30 Minuten bei Raumtemperatur gerührt. Dann wird die Reaktionsmischung auf Eiswasser gegossen, worauf man dreimal mit Essigester extrahiert. Die vereinigten organischen Phasen werden zuerst mit gesättigter Natriumhydrogencarbonatlösung und dann mit gesättigter Kochsalzlösung gewaschen, über Natriumsulfat getrocknet, filtriert und eingedampft. Man erhält rohes (E)-3-Brom-2,6,10-trimethyl-12-[(tetrahydro-2H-pyran -2-yl)oxy]-6-dodecen-2-ol, welches man anschliessend in 100 ml Methanol löst und bei 0° mit 14,0 g (0,1 Mol) Kaliumcarbonat versetzt. Nach 15-stündigem Rühren bei 0° wird über Hyflo filtriert, worauf man das Filtrat eindampft, den Rückstand in 600 ml Eiswasser aufnimmt und dreimal mit Aether extrahiert. Die vereinigten organischen Phasen werden mit Wasser gewaschen, über Natriumsulfat getrocknet, filtriert und eingedampft. Der Rückstand wird an Kieselgel mit Hexan-Essigester 4:1 chromatographiert. Man erhält (E)-2-[(10,11-Epoxy-3,7,11-trimethyl-6-dodecen -1-yl)oxy]tetrahydro-2H-pyran, welches anschliessend zusammen mit 8,0 g (40 mMol) Aluminiumisopropylat in 80 ml Toluol während 18 Stun den unter Rückfluss gekocht wird. Dann wird das Reaktionsgemisch eingedampft und der Rückstand in 300 ml 2N Salzsäure aufgenommen. Nach zweimaliger Extraktion mit Aether werden die vereinigten organischen Phasen zuerst mit gesättigter Natriumhydrogencarbonatlösung und dann mit gesättigter Kochsalzlösung gewaschen, über Natriumsulfat getrocknet, filtriert und eingedampft. Man erhält (E)-2,6,10-Trimethyl-12-[(tetrahydro-2H-pyran-2-yl)oxy] -1,6-dodecadien-3-ol, welches in 130 ml Methanol gelöst und zusammen mit 13 g stark saurem Ionenaustauscher (Dowex 50 W X 8) während 4 Stunden bei Raumtemperatur gerührt wird. Nach Filtration und Entfernen des Lösungsmittels im Rotationsverdampfer verbleibt ein gelbliches Oel, welches an Kieselgel mit Hexan-Essigester 2:1 unter Zusatz von 0,1% Triäthylamin chromatographiert wird. Man erhält (E)-3,7,11-Trimethyl-6,10-dodecadien-1,10-diol in Form eines farblosen Oels. MS:240 (M)⁺, 222 (M-H₂O)⁺.

### Beispiel 40

a) Zu einer Mischung von 11,2 g (38 mMol) (all-E)-10-Hydroxy-3,7,11-trimethyl-2,6,11 -dodecatriensäureisopropylester, 100 ml Methylenchlorid und 1 g Pyridinium-toluol-4-sulfonat werden bei 0° innerhalb von 30 Minuten 9,54 g (113,4 mMol) 3,4-Dihydro-2H-pyran getropft, worauf noch 2 Stunden bei Raumtemperatur gerührt wird. Nach Einengen im Rotationsverdampfer nimmt man den Rückstand in Wasser auf und extrahiert dreimal mit Hexan. Die vereinigten organischen Phasen werden mit gesättigter Kochsalzlösung gewaschen, über Natriumsulfat getrocknet, filtriert und eingedampft. Der Rückstand wird an Kieselgel mit Hexan-Essigester 9:1 unter Zusatz von 0,1% konz. Ammoniaklösung chromatographiert, wonach (all-E)-3,7,11-Trimethyl-10[(tetrahydro-2H-pyran-2-yl)oxy] -2,6,11-dodecatriensäureisopropylester erhalten wird. MS: 294 (M-Dihydropyran, C₅H₈O)⁺, 276 (M-(2-Hydroxy-tetrahydropyran, C₅H₁₀O₂))⁺.
b) Zu einer Lösung von 66,2 g (0,17 Mol) rohem (all-E)-3,7,11-Trimethyl-10-[(tetrahydro-2H-pyran-2-yl)oxy] -2,6,11-dodecatriensäureisopropylester in 250 ml Diäthyläther werden bei 10° innerhalb von 1 Stunde 600 ml einer 20% Lösung von Diisobutylaluminiumhydrid getropft. Anschliessend wird noch 2 Stunden bei Raumtemperatur gerührt. Dann wird die Reaktionsmischung auf 0° abgekühlt und vorsichtig zuerst mit 60 ml Essigester und dann mit 30 ml Methanol versetzt. Diese Mischung wird nun in eine gesättigte Lösung von Kaliumnatriumtartrat gegossen. Dann wird zweimal mit Aether extrahiert, worauf die vereinigten organischen Phasen zuerst mit gesättigter Natriumhydrogencarbonatlösung und dann mit gesättigter Kochsalzlösung gewaschen, über Natriumsulfat getrocknet, filtriert und eingedampft werden. Das als Rückstand verbleibende farblose Oel wird an Kieselgel mit Hexan-Essigester 4:1 unter Zusatz von 0,1% Triäthylamin chromatographiert. Man erhält gemäss Gaschromatogramm zu 98,6% reines (all-E)-3,7,11-Trimethyl-10-[(tetrahydro-2H-pyran-2-yl)oxy] -2,6,11-dodecatrien-1-ol.
c) Zu einer Mischung von 5 g (15,5 mMol) (all-E)-3,7,11-Trimethyl-10-[(tetrahydro-2H-pyran-2-yl)oxy] -2,6,11-dodecatrien-1-ol und 2,24 g (18,6 mMol) Pivalinsäurechlorid wird bei 0° eine Lösung von 1,84 g (23,25 mMol) absolutem Pyridin in 10 ml absolutem Tetrahydrofuran getropft. Anschliessend wird noch 1 Stunde bei Raumtemperatur gerührt. Dann wird die Reaktionsmischung in 250 ml einer Eis-Wasser-Mischung gegossen, worauf man dreimal mit Hexan extrahiert. Die vereinigten organischen Phasen werden mit gesättigter Kochsalzlösung gewaschen, über Natriumsulfat getrocknet, filtriert und eingedampft. Das als Rückstand verbleibende farblose Oel wird an Kieselgel mit Hexan-Essigester 9:1 unter Zusatz von 0,1% Triäthylamin chromatographiert, wonach (all-E)-3,7,11-Trimethyl-10-[(tetrahydro-2H-pyran-2-yl)oxy] -2,6,11-dodecatrienylpivalat erhalten wird.
d) Eine Suspension von 5,5 g stark saurem Ionenaustauscher (Dowex 50 W 8 X) in 35 ml Methanol bei Raumtemperatur mit 4,7 g (all-E)-3,7,11-Trimethyl-10-[(tetrahydro-2H-pyran-2-yl)oxy] -2,6,11-dodecatrienylpivalat versetzt. Anschliessend wird noch 20 Stunden bei Raumtemperatur gerührt. Nach Filtration und Eindampfen wird der Rückstand an Kieselgel mit Hexan-Essigester 4:1 unter Zusatz von 0,1% Triäthylamin chromatographiert, wonach (all-E)-3,7,11-Trimethyl-10-hydroxy-2,6,11 -dodecatrienylpivalat erhalten wird. MS (CI mit NH₃): 305 ((M+H)-H₂O)⁺, 221 ((M+H)-(CH₃)₃CCO₂H)⁺.

### Beispiel 41

a) Eine Mischung von 26 g (0,1 Mol) (all-E)-3,7,11-Trimethyl-2,4,6,10-dodecatetraensäureäthylester, 180 ml 1,2-Dimethoxyäthan und 40 ml Wasser wird bei -5° innerhalb von 20 Minuten mit 20 g (0,112 Mol) N-Bromsuccinimid versetzt. Nach 3-stündigem Rühren bei -5° wird das Gemisch mit 5 g Natriumsulfat und 10 g Kaliumcarbonat versetzt und anschliessend noch während 10 Minuten gerührt. Nach Entfernung der Feststoffe durch Filtration wird das Filtrat mit 18 g (0,13 Mol) Kaliumcarbonat versetzt und während 12 Stunden bei 0° gerührt. Dann wird die Reaktionsmischung eingeengt, mit 200 ml Eiswasser versetzt und zweimal mit je 100 ml Aether extrahiert. Die vereinigten organischen Phasen werden über Natriumsulfat getrocknet, filtriert und eingedampft, und das erhaltene gelbe Oel wird mit Hexan-Essigester 95:5 an Kieselgel chromatographiert. Man erhält (all-E)-10,11-Epoxy-3,7,11-trimethyl-2,4,6 -dodecatriensäureäthylester, welcher anschliessend zusammen mit 8,4 g (40,8 mMol) Aluminiumisopropylat in 170 ml Toluol während 15 Stunden unter Rückfluss gekocht wird. Dann wird die Reaktionsmischung auf 150 ml Eiswasser gegossen, mit 25%iger Salzsäurelösung auf pH 4 gebracht und mit Aether extrahiert. Die Aetherphase wird mit Eiswasser und dann mit gesättigter Kochsalzlösung gewaschen, über Natriumsulfat getrocknet, filtriert und eingeengt. Das verbleibende gelbe Oel wird an Kieselgel mit Hexan-Essigester 8:2 unter Zusatz von 0,1% Triäthylamin chromatographiert, wonach (all-E)-10-Hydroxy-3,7,11-trimethyl-2,4,6,11 -dodecatetraensäureisopropylester erhalten wird. MS: 292 (M)⁺, 232 (M-(CH₃)₂CH-OH)⁺.
b) Zu einer Lösung von 5 g (17, 1 mMol) (all-E)-10-Hydroxy-3,7,11-trimethyl-2,4,6,11 -dodecatetraensäureisopropylester in 20 ml Hexan werden bei -5° innerhalb von 30 Minuten 57 ml (85 mMol) einer 20%igen Lösung von Diisobutylaluminiumhydrid in Hexan getropft. Danach wird die Reaktionsmischung mit 250 ml 20%iger Natronlauge versetzt, worauf man mit Aether extrahiert. Die Aetherphase wird zuerst mit gesättigter Natriumhydrogencarbonatlösung und dann mit Wasser gewaschen, über Natriumsulfat getrocknet und eingeengt. Der Rückstand wird an Kieselgel mit Hexan-Essigester 1:1 unter Zusatz von 0,1% Triäthylamin chromatographiert, wonach man weisses, wachsartiges (all-E)-3,7,11-2,4,6,11-dodecatetraen-1,10-diol vom Smp. 53-54° erhält.

### Beispiel 42

a) Zu einer Mischung von 90,4 g (0,295 Mol) (all-E)-Farnesyl-tetrahydropyranyläther, 600 ml 1,2-Dimethoxyäthan und 120 ml Wasser werden bei -5° innerhalb von 30 Minuten 58,8 g (0,33 Mol) N-Bromsuccinimid zugefügt. Anschliessend wird noch 1 Stunde bei Raumtemperatur gerührt. Dann wird die Mischung in 2 l Wasser gegossen, worauf man dreimal mit je 650 ml Essigester extrahiert. Die vereinigten organischen Phasen werden mit gesättigter Kochsalzlösung gewaschen, über Natriumsulfat getrocknet und eingedampft. Man erhält rohes (all-E)-3-Brom-2,6,10-trimethyl-12-[(tetrahydro-2H-pyran -2-yl)oxy]-6,10-dodecadien-2-ol.

   Das obige Rohprodukt wird innerhalb von 5 Minuten zu einer Mischung von 30 g Kaliumcarbonat und 250 ml Methanol getropft, worauf man noch während 1 Stunde bei Raumtempera tur rührt und dann einengt. Der Rückstand wird in Aether aufgenommen und zuerst mit Wasser und dann mit gesättigter Kochsalzlösung gewaschen. Nach Trocknen über Natriumsulfat und Einengen wird (2E,6E)-10,11-Epoxy-farnesyltetrahydropyranyläther erhalten. MS (CI mit NH₃): 221 (M+H- (2-hydroxy-tetrahydropyran, C₅H₁₀O))⁺; IR (Film): 1668 (-C=C-), 1378, 1359 (gem. Dimethyl), 870 (Epoxyd).
b) Zu einer Mischung von 60 g (0,294 Mol) Aluminiumisopropylat und 600 ml Toluol werden bei 110° innerhalb von 5 Minuten 95 g (0,295 Mol) (2E,6E)-10,11-Epoxy-farnesyltetrahydropyranyläther gegeben. Dann wird während 20 Stunden unter Rückfluss gekocht. Anschliessend wird die Reaktionsmischung in 2 l Eiswasser gegossen und durch Zugabe von eiskalter 2N Salzsäure auf pH 5 gebracht. Nach dreimaliger Extraktion mit Hexan werden die vereinigten organischen Phasen mit gesättigter Kochsalzlösung gewaschen, über Natriumsulfat filtriert und dann eingedampft. Das anfallende hellgelbe Oel wird an Kieselgel mit Hexan-Essigester 4:1 unter Zusatz von 0,1% Triäthylamin chromatographiert. Nach Einengen und Trocknen im Hochvakuum erhält man (all-E)-2,6,10-Trimethyl-12-[(tetrahydro-2H-pyran-2-yl)oxy] -1,6,10-dodecatrien-3-ol.
c) Zu einer Mischung von 500 g Mangandioxid und 1,5 l Methylenchlorid werden innerhalb von 10 Minuten 50,4 g (0,156 Mol) (all-E)-2,6,10-Trimethyl-12-[(tetrahydro-2H-pyran-2-yl)oxy] -1,6,10-dodecatrien-3-ol getropft. Man rührt noch während 20 Stunden bei Raumtemperatur weiter und filtriert dann über eine Schicht von Natriumsulfat. Nach Eindampfen des Filtrates wird (all-E)-2,6,10-Trimethyl-12-[(tetrahydro-2H-pyran-2-yl)oxy] -1,6,10-dodecatrien-3-on erhalten.

Das obige Produkt wird innerhalb von 10 Minuten zu einer Mischung von g Dowex 50 W X 8 und 500 ml Methanol getropft. Man rührt während 2 Stunden bei Raumtemperatur, filtriert dann die Mischung durch eine Nutsche und dampft das Filtrat anschliessend ein. Der Rückstand wird an Kieselgel mit Hexan-Essigester 1:1 unter Zusatz von 0.1% Triäthylamin chromatographiert. Nach Einengen der Eluate und Trocknen im Hochvakuum erhält man reines (all-E)-12-Hydroxy-2,6,10-trimethyl-1,6,10-dodecatrien-3-on. IR (Film): 3398, 1003 (OH), 3097 (C=CH₂), 1675 (Keton konj.), 1630 (-C=C-, konj.), 1368 (gem. Dimethyl).

### Beispiel 43

Zu einer Mischung von 1,5 g (6, 34 mMol) (all-E)-12-Hydroxy-2,6,10-trimethyl-1,6,10-dodecatrien-3-on und 776 mg (7,6 mMol) Essigsäureanhydrid werden bei 0° 752 mg (9,5 mMol) absolutes Pyridin getropft. Anschliessend wird noch 30 Minuten bei 0° weitergerührt, worauf die Reaktionsmischung unter starkem Rühren in 250 ml Eiswasser gegossen wird. Nach dreimaliger Extraktion mit Hexan werden die vereinigten organischen Phasen mit gesättigter Kochsalzlösung gewaschen, über Natriumsulfat getrocknet und dann eingeengt. Der Rückstand wird an Kieselgel mit Hexan-Essigester 4:1 unter Zusatz von 0,1% Triäthylamin chromatographiert, wonach (all-E)-3,7,11-Trimethyl-10-oxo-2,6,11-dodecatrienylacetat erhalten wird. MS (CI mit NH₃) : 296 (M+NH₄)⁺.

### Beispiel 14

Eine Mischung von 5,29 g (22,4 mMol) (all-E)-12-Hydroxy-2,6,10-trimethyl-1,6,10-dodecatrien-3-on, 1,59 g (22,8 mMol) Hydroxylamin-hydrochlorid, 0,96 g (24,0 mMol) festem Natriumhydroxid, 80 ml Methanol und 10 ml Wasser wird während 20 Stunden bei Raumtemperatur gerührt. Dann wird eingeengt und der Rückstand in einer Mischung von 50 ml Wasser und 50 ml Aether aufgenommen. Nach kräftigem Schütteln in einem Scheidetrichter wird die Aetherphase abgetrennt. Die wässrige Phase wird anschliessend dreimal mit Aether extrahiert. Die vereinigten Aetherphasen werden über Natriumsulfat getrocknet, filtriert und eingedampft. Der Rückstand wird an Kieselgel mit Hexan-Essigester 4:1 unter Zusatz von 0,1% Triäthylamin chromatographiert, und man erhält (6E,10E)-12-Hydroxy-2,6,10-trimethyl-1,6,10-dodecatrien -3-on-oxim. MS: 234 (M-OH)⁺.

### Beispiel 45

Zu einer Grignardlösung, hergestellt aus 350 mg (14,4 mMol) Magnesium und 1,83 g (12 mMol) frisch destilliertem n-Pentylbromid in 10 ml absolutem Tetrahydrofuran werden bei -70° 2,80 g (10 mMol) (all-E)-4,8-Dimethyl-10-[(tetrahydro-2H-pyran-2-yl)oxy] -4,8-decadienal zugetropft. Anschliessend lässt man auf 0° erwärmen und rührt noch während 30 Minuten bei dieser Temperatur weiter. Dann wird die Reaktionsmischung in 250 ml gesättigte Ammoniumchloridlösung von 0° gegossen. Nach dreimaliger Extraktion mit Aether werden die vereinigten organischen Auszüge mit gesättigter Kochsalzlösung gewaschen und dann über Natriumsulfat filtriert. Nach Einengen wird der Rückstand an Kieselgel mit Hexan-Essigester 4:1 chromatographiert, wonach (all-E)-9,13-Dimethyl-15-[(tetrahydro-2H-pyran-2-yl)oxyl] -9,13-pentadecadien-6-ol erhalten wird.

Das obige Material wird zusammen mit 300 mg Pyridinium-toluol-4-sulfonat in 50 ml absolutem Aethanol während 1 Stunde bei 55° gerührt. Dann wird eingedampft und der Rückstand in 300 ml Wasser aufgenommen. Nach dreimaliger Extraktion mit Aether wird die Aetherphase mit gesättigter Kochsalzlösung gewaschen und dann über Natriumsulfat getrocknet. Nach Einengen wird der Rückstand an Kieselgel mit Hexan-Essigester 7:3 chromatographiert. Das erhaltene (all-E)-3,7-Dimethyl-2,6-pentadecadien-1,10-diol wird im Hochvakuum getrocknet. MS: 250 (M-H₂O)⁺.

### Beispiel 46

a) Eine Lösung von 3,92 g (151 mMol) Acetylen in 70 ml absolutem Tetrahydrofuran wird in Argonatmosphäre bei -70° tropfenweise zuerst mit 87,2 ml (140 mMol) einer 1,6M Lösung von Butyllithium in Hexan und dann mit einer Lösung von 13,28 g (47,4 mMol) (aal-E)-4,8-Dimethyl-10-[(tetrahydro-2H-pyran-2-yl)oxy] -4,8-decadienal in 60 ml absolutem Tetrahydrofuran versetzt. Anschliessend wird noch 1 Stunde bei -70° und dann 30 Minuten bei -30° gerührt. Nach Erwärmen auf 0° wird die Reaktionsmischung in eine Mischung von gesättigter Ammoniumchloridlösung und Eis gegossen. Nach Abtrennen der organischen Phase wird die wässrige Phase dreimal mit Aether extrahiert. Die vereinigten organischen Phasen werden mit gesättigter Kochsalzlösung gewaschen, über Natriumsulfat filtriert und dann eingeengt. Der Rückstand wird an Kieselgel mit Hexan-Essigester 4:1 chromatographiert, wonach (all-E)-6,10-Dimethyl-12-[(tetrahydro-2H-pyran-2-yl)oxy] -6,10-dodecadien-1-in-3-ol erhalten wird.
b) Zu einer Suspension von 10 g Dowex 50 W X 8 in 60 ml absolutem Methanol werden bei Raumtemperatur innerhalb von 5 Minuten 6,65 g (21,7 mMol) (all-E)-6,10-Dimethyl-12-[(tetrahydro-2H-pyran-2-yl)oxy] -6,10-dodecadien-1-in-3-ol getropft. Nach 3-stündigem Rühren wird filtriert und eingeengt. Der Rückstand wird an Kieselgel mit Hexan-Essigester 1:1 unter Zusatz von 0,1% Triäthylamin chromatographiert, und man erhält (all-E)-3,7-Dimethyl-2,6-dodecadien-11-in-1,10-diol als farbloses Oel. IR (Film): 3346, 1065, 1004 (OH), 3296, 2113 (-C≡CH), 1667 (-C=C-), 1384 (gem. Dimethyl), 653, 629 (-C≡CH). MS: 204 (M-H₂O)⁺.

### Beispiel 47

a) Zu einer Suspension von 130 g Braunstein (aktiv, gefällt) in 400 ml Methylenchlorid wird bei 0° eine Lösung von 13,07 g (42,65 mMol) (all-E)-6,10-Dimethyl-12-[(tetra hydro-2H-pyran-2-yl)oxy] -6,10-dodecadien-1-in-3-ol in 50 ml Methylenchlorid getropft. Dann wird über Nacht bei +4° weitergerührt. Anschliessend gibt man nochmals 13 g Braunstein zu und rührt während 1 Stunde bei Raumtemperatur. Dann wird über eine Schicht von Natriumsulfat filtriert und eingeengt. Der Rückstand wird an Kieselgel mit Hexan-Essigester 4:1 chromatographiert, wonach (all-E)-6,10-Dimethyl-12-[(tetrahydro-2H-pyran-2-yl)oxy] -6,10-dodecadien-1-in-3-on erhalten wird.
b) Zu einer Lösung von 800 mg Pyridinium-toluol-4-sulfonat in 100 ml absolutem Aethanol wird bei 55° eine Lösung von 7,7 g (25,3 mMol) (all-E)-6,10-Dimethyl-12-[(tetrahydro-2H-pyran-2-yl)oxy] -6,10-dodecadien-1-in-3-on in 10 ml absolutem Aethanol getropft. Nach 1,5 Stunden wird eingeengt und der Rückstand in Aether aufgenommen. Die Aetherphase wird zuerst mit Wasser und dann mit gesättigter Kochsalzlösung gewaschen. Hierauf filtriert man über Natriumsulfat und engt das Filtrat ein. Durch Chromatographie des Rückstandes an Kieselgel mit Hexan-Essigester 1:1 wird als erstes (all-E-10,10-Diäthoxy-3,7-dimethyl-2,6-dodecadien-11-in-1-ol (MS: 249 (M-OEt)⁺) eluiert, gefolgt von (all-E)-12-Hydroxy-6,10-dimethyl-6,10-dodecadien-1-in-3-on (IR (Film): 3358, 1002 (OH), 3258, 2090 (-C≡CH), 1680 (Keton konj.)).

### Beispiel 48

a) Eine Lösung von 0.98 g (12 mMol) 3,3-Dimethyl-1-butin in 25 ml absolutem Tetrahydrofuran wird in Argonatmosphäre bei -70° tropfenweise zuerst mit 7,5 ml (12 mMol) einer 1,6M Lösung von Butyllithium in Hexan und dann mit 2,8 g (10 mMol) (all-E)-4,8-Dimethyl-10-[(tetrahydro-2H-pyran-2-yl)oxy] -4,8-decadienal versetzt. Hierauf wird zuerst noch 1 Stunde bei -70° und dann 15 Stunden bei Raumtemperatur gerührt. Anschliessend wird die Reaktionsmischung in 250 ml eiskalte gesättigte Ammoniumchlorilösung gegossen, worauf man dreimal mit Aether extrahiert. Die vereinigten organi schen Phasen werden mit gesättigter Kochsalzlösung gewaschen, über Natriumsulfat filtriert und dann eingeengt. Der Rückstand wird an Kieselgel mit Hexan-Essigester 4:1 unter Zusatz von 0,1% Triäthylamin chromatographiert, wonach (all-E)-2,2,8,12-Tetramethyl-14-[(tetrahydro-2H-pyran -2-yl))oxy]-8,12-tetradecadien-3-in-5-ol erhalten wird. MS: 278 (M-Dihydropyran, C₅H₈O)⁺, 260 (M-Dihydropyran + H₂O))⁺.
b) Zu einer Suspension von 29,2 g Mangandioxid (aktiviert) in 100 ml Methylenchlorid werden bei 0° innerhalb von 5 Minuten 2,92 g (all-E)-2,2,8,12-Tetramethyl-14-[(tetrahydro-2H-pyran-2-yl)oxy]-8,12-tetradecadien-3-in-5-ol getropft. Anschliessend wird noch 30 Minuten bei Raumtemperatur weitergerührt, worauf man das Reaktionsgemisch über eine Schicht Natriumsulfat abnutscht und das Filtrat einengt. Der Rückstand wird an Kieselgel mit Hexan-Essigester 4:1 chromatographiert, wonach (all-E)-2,2,8,12-Tetramethyl-14-[(tetrahydro-2H-pyran -2-yl)oxy]-8,12-tetradecadien-3-in-5-on erhalten wird.
c) Eine Mischung von 70 ml 90% Aethanol, 0,246 g Pyridinium-toluol-4-sulfonat und 2,45 g (6,8 mMol) (all-E)-2,2,-8,12-Tetramethyl-14-[(tetrahydro-2H-pyran -2-yl)oxy]-8,12-tetradecadien-3-in-5-on wird während 17 Stunden auf 55° erwärmt. Nach Einengen wird der Rückstand in Wasser aufgenommen, worauf man mit Aether extrahiert. Die vereinigten organischen Auszüge werden mit gesättigter Kochsalzlösung gewaschen und dann über eine Schicht Natriumsulfat abgenutscht. Nach Einengen des Filtrats wird der Rückstand an Kieselgel mit Hexan-Essigester 4:1 chromatographiert. Man erhält (all-E)-14-Hydroxy-2,2,8,12-tetramethyl-8,12-tetradecadien -3-in-5-on. MS: 243 (M-(CH₃+H₂O))⁺.

### Beispiel 49

a) Zu 190 ml (0,32 Mol) einer ca. 1,7M Lösung von Propin in Tetrahydrofuran werden bei -70° innerhalb von 25 Minuten 120 ml (0,19 Mol) einer 1,7M Lösung von Butyllithium in Hexan getropft. Nach 30-minütigem Rühren bei -70° wird bei gleicher Temperatur innerhalb von 10 Minuten eine Lösung von 30,81 g (0,11 Mol) (all-E)-4,8-Dimethyl-10-[(tetrahydro-2H-pyran-2-yl)oxy] -4,8-decadienal in 30 ml abs. Tetrahydrofuran zugetropft. Dann wird noch während 1 Stunde bei -70° gerührt. Nach Erwärmen auf Raumtemperatur wird die Mischung in 1,5 l einer gesättigten Lösung von Ammoniumchlorid in Wasser gegossen. Nach dreimaliger Extraktion mit n-Hexan werden die Extrakte vereinigt, mit Natriumsulfat getrocknet, filtriert und eingeengt, wobei rohes (all-E)-7,11-Dimethyl-13-[(tetrahydro-2H-pyran-2-yl)oxy] -7,11-tridecadien-2-in-4-ol erhalten wird.
b) Zu einer Mischung von 10 g stark saurem Ionenaustauscher (Dowex 50 W 8 X) und 60 ml absolutem Methanol werden bei Raumtemperatur 7,9 g (24,65 mMol) (all-E)-7,11-Dimethyl-13-[(tetrahydro-2H-pyran-2-yl)oxy] -7,11-tridecadien-2-in-4-ol getropft. Anschliessend wird noch 2,5 Stunden bei Raumtemperatur gerührt. Dann filtriert man durch eine Glasfritte und dampft ein. Der Rückstand wird an 200 g Kieselgel mit Hexan-Essigester 1:1 unter Zusatz von 0,1% Triäthylamin chromatographiert. Man erhält (all-E)-3,7-dimethyl-2,6-tridecadien-11-in-1,10-diol. MS (CI mit NH₃): 254 (M+NH₄)⁺.

### Beispiel 50

a) Zu einer Suspension von 200 g Mangandioxid (aktiv, gefällt) in 800 ml Methylenchlorid werden bei 0° innerhalb von 15 Minuten 20,5 g (67 mMol) (all-E)-7,11-Dimethyl-13-[(tetrahydro-2H-pyran-2-yl)oxy] -7,11-tridecadien-2-in-4-ol getropft. Nach Erwärmen auf Raumtemperatur wird noch 2,5 Stunden weitergerührt. Dann filtriert man ab und engt das Filtrat ein. Der Rückstand wird an Kieselgel mit Hexan-Essigester 4:1 chromatographiert. Man erhält (all-E)-7,11-Dimethyl-13-[(tetrahydro-2H-pyran-2-yl)oxy] -7,11-trideca dien-2-in-4-on.
b) Zu einer Lösung von 1,5 g Pyridinium-toluol-4-sulfonat in 200 ml absolutem Aethanol werden bei 55° 15,13 g (47,8 mMol) (all-E)-7,11-Dimethyl-13-[(tetrahydro-2H-pyran-2-yl)oxy] -7,11-tridecadien-2-in-4-on getropft. Anschliessend wird noch 2 Stunden bei derselben Temperatur gerührt. Nach Einengen der Reaktionsmischung wird der Rückstand in Aether aufgenommen und mit 500 ml Wasser geschüttelt. Nach Abtrennung der Aetherphase wird die wässrige Phase nochmals mit Aether extrahiert. Die vereinigten organischen Phasen werden mit gesättigter Kochsalzlösung gewaschen, über Natriumsulfat getrocknet und dann eingeengt. Der Rückstand wird an Kieselgel zuerst mit Hexan-Essigester 4:1, dann 1:1, chromatographiert, wonach (all-E)-13-Hydroxy-7,11-dimethyl-7,11-tridecadien-2-in-4-on gewonnen wird. MS: 201 (M-(CH₃+H₂O))⁺; IR (Film): 3412 (OH), 2219 (-C≡C-), 1670 (Keton konj.).

### Beispiel 51

Eine Lösung von 9,6 g (30 mMol) (all-E)-7,11-Dimethyl-13-[(tetrahydro-2H-pyran-2-yl)oxy] -7,11-tridecadien-2-in-4-ol in 50 ml absolutem Tetrahydrofuran wird bei Raumtemperatur tropfenweise mit 30 ml (105 mMol) einer ca. 3,5M Lösung von Natrium-dihydro-bis-(2-methoxyäthoxy)-aluminat in Toluol versetzt. Dann wird 1 Stunde bei 110° gekocht. Anschliessend wird das Reaktionsgemisch sorgfältig auf Eis gegossen, wonach man bis zur leicht sauren Reaktion eiskalte 2N Salzsäure zugibt. Dann extrahiert man dreimal mit Hexan und wäscht die vereinigten Hexanphasen mit gesättigter Kochsalzlösung neutral. Nach Trocknen über Natriumsulfat, Filtration und Eindampfen erhält man (all-E)-7,11-Dimethyl-13-[(tetrahydro-2H-pyran-2-yl)oxy] -2,7,11-tridecatrien-4-ol.

Das obige Material wird zusammen mit 750 mg Pyridinium-toluol-4-sulfonat in 200 ml absolutem Aethanol während 1,5 Stunden bei 55° gekocht. Dann dampft man ein und nimmt den Rückstand in Aether auf. Die organische Phase wird mit Wasser und dann mit gesättigter Kochsalzlösung gewaschen und anschliessend über Natriumsulfat filtriert. Nach Einengen des Filtrats wird der Rückstand an Kieselgel mit Hexan-Essigester 1:1 chromatographiert. Man erhält (all-E)-3,7-Dimethyl-2,6,11-tridecatrien-1,10-diol. MS: 189 (M-(CH₂OH+H₂O))⁺.

### Beispiel 52

a) Eine Mischung von 4,8 g (200 mMol) Magnesium und 200 ml absolutem Tetrahydrofuran wird bei 70-80° mit ca. 200 mg Jodkristallen versetzt. Nach Verschwinden der Jodfarbe werden 24 g (178 mMol) 2-Methyl-1-brompropen zugetropft. Dann wird noch 2 Stunden unter Rückfluss gekocht. Nach Abkühlen auf Raumtemperatur wird die erhaltene Grignardlösung in Argonatmosphäre filtriert, auf ca. -70° abgekühlt und tropfenweise mit einer Lösung von 24 g (85 mMol) (all-E)-4,8-dimethyl-10-[(tetrahydro-2H-pyran-2-yl)oxy] -4,8-decadienal in 50 ml Tetrahydrofuran versetzt. Nach beendeter Zugabe wird noch während 2 Stunden bei einer Temperatur von -60° bis -70° weitergerührt. Dann entfernt man die Kühlung und rührt über Nacht bei Raumtemperatur. Nach Zugabe von 100 ml gesättigter Ammoniumchloridlösung wird die organische Phase abgetrennt. Die wässrige Phase wird zweimal mit Aether extrahiert. Die vereinigten organischen Phasen werden über Magnesiumsulfat getrocknet und eingeengt. Der Rückstand wird an Kieselgel mit Hexan-Essigester 4:1 chromatographiert. Man erhält (all-E)-2,7,11-Trimethyl-13-[(tetrahydro-2H-pyran-2-yl)oxy] -2,7,11-tridecatrien-4-ol. MS: 234 (M-(2-Hydroxy-tetrahydropyran, C₅H₁₀O₂))⁺.
b) Eine Mischung von 3 g (8,91 mMol) (all-E)-2,7,11-trimethyl-13-[(tetrahydro-2H-pyran-2-yl)oxy] -2,7,11-tridecatrien-4-ol, 54 ml Aethanol, 16 ml Wasser und 0,5 g Pyridinium-toluol-4-sulfonat wird während 1 Stunde bei 55° gekocht. Dann wird eingeengt, mit Toluol versetzt und erneut eingedampft. Der Rückstand wird in Aether aufgenommen und durch eine mit Kieselgel beschichtete Nutsche filtriert. Nach Einengen des Filtrats wird an Kieselgel mit Hexan-Essigester 1:1 chromatographiert. Man erhält (all-E)-3,7,12-trimethyl-2,6,11-tridecatrien-1,10-diol. MS: 216 (M-2H₂O)⁺.

### Beispiel 53

a) Zu einer Lösung von 11,72 g tert.-Butyldimethylchlorsilan in 140 ml Dichlormethan werden 12,51 ml 1,8-Diazabicyclo[5.4.0]undec-7-en gegeben. Anschliessend werden innerhalb von 30 Minuten 20,6 g (all-E)-10-Hydroxy-3,7,11-trimethyl-2,6,11 -dodecatriensäureisopropylester zugetropft. Nach 28 Stunden Rühren bei Raumtemperatur giesst man das Reaktionsgemisch auf Eis-Wasser und extrahiert zweimal mit je 400 ml Essigsäureäthylester. Die vereinigten Extrakte werden mit gesättigter Kochsalzlösung gewaschen, über Natriumsulfat getrocknet, filtriert und eingedampft. Das erhaltene Oel wird an 1,5 kg Kieselgel mit Hexan-2% Essigsäureäthylester chromatographiert. Dabei erhält man (6E)-10-(tert.-Butyldimethylsiloxy)-3,7,11-trimethyl -2,6,11-dodecatriensäure-isopropylester als gelbliches Oel.
b) Zu einer Lösung von 32,6 g (6E)-10-(tert.-Butyldimethylsiloxy)-3,7,11-trimethyl -2,6,11-dodecatriensäureisopropylester in 80 ml Hexan werden bei 0° langsam 24,5 ml Natrium-dihydro-bis(2-methoxyäthoxy)-aluminat (70% Lösung in Toluol, "Vitride") zugetropft. Nach 2-stündigem Rühren bei Raumtemperatur werden weitere 2,5 ml "Vitride" zugetropft. Die Reaktionsmischung wird während einer weiteren Stunde gerührt und dann vorsichtig mit 20 ml Wasser versetzt, worauf das Gemisch auf Eis-Wasser gegossen wird. Nach Zugabe von 70 ml 50% Schwefelsäure wird zweimal mit Hexan extrahiert. Die vereinigten Auszüge werden mit 2N Schwefelsäure, Wasser, gesättigter Natriumbicarbonatlösung und gesättigter Kochsalzlösung gewaschen, über Natriumsulfat getrocknet, filtriert und eingedampft. Der Rückstand wird an Kieselgel mit Hexan-Essigsäureäthylester-Triäthylamin 80:20:0,1 chromatographiert, wobei (6E)-10-(tert.-Butyldimethylsiloxy)-3,7,11-trimethyl -2,6,11-dodecatrien-1-ol als farbloses Oel erhalten wird. NMR (80 MHz, CDCl₃): 5,45 (t,J=7, 1 olefin.H); 5,15 (m, 1 olefin.H); 4,95-4,75 (m,2H-C(12)); 4.2 (d,J=7, 2H-C(1)); 4,05 (t,J=6,5, H-C(10)); 2,2-1,3 (m, 4CH₂, 3CH₃); 0,9 (s, SiC(CH₃)₃); 0,05 und 0,0 (2s, Si(CH₃)₂).
c) Zu einer Suspension von 110 g Mangandioxid (gefällt, aktiv) in 220 ml Dichlormethan werden bei 0° 10,58 g (6E)-1-(tert.-Butyldimethylsiloxy)-3,7,11-trimethyl -2,6,11-dodecatrien-1-ol getropft, und die Mischung wird während 2 Stunden bei Raumtemperatur gerührt. Nach Filtration über Hyflo engt man das Filtrat ein und chromatographiert den öligen Rückstand an Kieselgel mit Hexan-Essigsäureäthylester-Triäthylamin 90:10:0,1. Man erhält (6E)-10-(tert.-Butyldimethylsiloxy)- -3,7,11-trimethyl -2,6,11-dodecatrienal als gelbliches Oel. NMR (80 MHz, CDCl₃): 10,1 (d,J=8, H-C(1)); 6,0 (d,J=8, H-C(2)): 5,2 (m, H-C(6)): 5,0-4,8 (m, 2H-C(12)); 4,1 (t,J=6,5, H-C(10)); 2,4-1,4 (m, 4CH₂, 3CH₃); 0,95 (s, SiC(CH₃)₃); 0,1 und 0,05 (2s, Si(CH₃)₂).
d) Zu einer aus 0,56 g Magnesiumspänen und 3,1 g Methyljodid in 80 ml Aether bereiteten Methylmagnesiumjodidlösung tropft man bei -20° eine Lösung von 7,3 g (6E)-10-(tert.-Butyldimethylsiloxy)-3,7,11-trimethyl -2,6,11-dodecatrienal in 10 ml Aether. Nach 2-stündigem Rühren bei -20° giesst man das Reaktionsgemisch auf ein Gemisch von Eis und gesättigter Ammoniumchloridlösung und extrahiert zweimal mit Aether. Die vereinigten organischen Extrakte werden mit gesättigter Kochsalzlösung gewaschen, über Natriumsulfat getrocknet, filtriert und eingedampft. Das als Rückstand verbleibende Oel wird an Kieselgel mit Hexan-Essigsäureäthylester-Triäthylamin 90:10:0,1 chromatographiert, wobei man (7E)-11-(tert.-Butyldimethylsiloxy)-4,8,12-trimethyl -3,7,12-tridecatrien-2-ol als farbloses Oel erhält. NMR (250 MHz, CDCl₃): 5,2 (d,J=8, H-C(3)); 5,1 (t,J=6,5 H-C(7)); 4,84 und 4,75 (2m, 2H-C(13)); 4,57 (m, H-C(2)); 4,0 (t,J=6,5, H-C(11)); 2,25-1,4 (m, 4CH₂, 3CH₃); 1,22 (d,J=6,5, 3H-C(1)); 0,89 (s, SiC(CH₃)₃); 0,04 and 0,0 (2s, Si(CH₃)₂).
e) 5 g (7E)-11-(tert.-Butyldimethylsiloxy)-4,8,12-trimethyl -3,7,12-tridecatrien-2-ol werden bei 0° zu einer Aufschlämmung von 25 Mangandioxid (aktiv, gefällt) in 50 ml Dichlormethan getropft. Nach 5-stündigem Rühren bei Raumtemperatur filtriert man das Reaktionsgemisch über Natriumsulfat und dampft das Filtrat ein. Das erhaltene Material wird noch zweimal demselben Oxidationsprozess mit jeweils frischem Mangandioxid unterworfen, um vollständigen Umsatz zu erzielen. Chromatographie des erhaltenen Rohprodukt an Kieselgel mit Hexan-Essigsäureäthylester 2%→5% liefert (7E)-11-(tert.-Butyldimethylsiloxy)-4,8,12-trimethyl -3,7,12-tridecatrien-2-on als gelbliches Oel, welches gemäss Gaschromatogramm eine 95%ige Reinheit aufweist. NMR (250 MHz, CDCl₃): 6,05 (s, H-C(3)); 5,1 (m, H-C(7)); 4,84 und 4,75 (2m, 2H-C(13)); 4,0 (t,J=6,5, H-C(11)); 2,3-1,4 (m, 4CH₂, 3CH₃); 0,89 (s, SiC(CH₃)₃); 0,35 und 0,0 (2s, Si(CH₃)₂).
f) Zu einer Lösung von 3,2 g (7E)-11-(tert.-Butyldimethylsiloxy)- -4,8,12-trimethyl -3,7,12-tridecatrien-2-on in 70 ml Tetrahydrofuran und 35 ml Methanol werden bei 0° 13 ml 2N HCl gegeben, worauf die Mischung während 5,5 Stunden bei Raumtemperatur gerührt wird. Hierauf giesst man das Reaktionsgemisch auf 300 ml Wasser und extrahiert zweimal mit Hexan. Die vereinigten Extrakte werden mit gesättigter Natriumbicarbonatlösung und gesättigter Kochsalzlösung gewaschen, über Natriumsulfat getrocknet, filtriert und eingedampft. Chromatographie des Rückstandes an Kieselgel mit Hexan-Aether 6:4 liefert (3E/Z,7E)-11-Hydroxy-4,8,12-trimethyl-3,7,12-tridecatrien-2-on als leicht gelbliches Oel. Laut Gaschromatographie handelt es sich um ein (Z/E)-Gemisch im Verhältnis von 1:2. NMR (250 MHz, CDCl₃): 6,06 (s, H-C(3)); 5,13 (m, H-C(7)); 4,94 und 4,84 (2s, 2H-C(13)); 4,05 (t,J=6, H-C(11)); 2,6 (t, 2H-C(5) vom 3Z-Isomeren); 2,17 und 2,16 (2s, CH₃CO vom 3E- und 3Z-Isomeren); 2,12 und 1,87 (2s, CH₃-C(4) vom 3E- und 3Z-Isomeren); 2,0 (m, 2CH₂); 1,75-1,5 (m, 2CH₂,2CH₃). IR (Film): 3434 (breit, OH); 1685 (konj. Keton); 1613 (konj. C=C); 897 (=CH₂). MS: 232 (1, M⁺-H₂O); 217 (2, M⁺-H₂O-CH₃); 189 (5, M⁺-H₂O-COCH₃); 43 (100, COCH₃). Analyse: C₁₆H₂₆O₂ (250,38);
   ber. C 76,75, H 10,47%
   gef. C 76,55, H 10,62%.

### Beispiel 54

a) Eine Suspension von 400 g Mangandioxid (aktiviert) in 1 l Methylenchlorid wird bei Raumtemperatur mit 10 g (30 mMol) (all-E)-2,7,11-Trimethyl-13-[(tetrahydro-2H-pyran-2-yl)oxy] -2,7,11-tridecatrien-4-ol versetzt. Anschliessend wird noch 2 Stunden bei Raumtemperatur gerührt. Dann wird über eine Hyflo-Schicht filtriert. Nach Einengen des Filtrats wird der Rückstand an Kieselgel (0,040-0,063 mm) unter einem Druck von ca. 0,1 bar (Pressluft) mit Hexan-Essigester zuerst im Verhältnis 9:1 und dann im Verhältnis 8:2 chromatographiert. Nach Einengen des Filtrats und Trocknen des Rückstands im Hochvakuum erhält man (all-E)-2,7,11-Trimethyl-13-[(tetrahydro-2H-pyran-2-yl)oxy] -2,7,11-tridecatrien-4-on. MS: 232 (M-(2-Hydroxy-tetrahydropyran, C₅H₁₀O₂))⁺.
b) Eine Mischung von 2 g stark saurem Ionenaustauscher (Dowex 50 W 8 X), 100 ml Methanol und 3,3 g (all-E)-2,7,11-Trimethyl-13-[(tetrahydro-2H-pyran-2-yl)oxy] -2,7,11-tridecatrien-4-on wird während 3 Stunden bei Raumtemperatur gerührt. Nach Filtration und Einengen des Filtrats wird der Rückstand an Kieselgel mit hexan-Essigester 3:2 chromatographiert, wonach (all-E)-13-Hydroxy-2,7,11-trimethyl-2,7,11-tridecatrien-4-on in Form eines leicht gelben Oels erhalten wird. IR (film): 3420, 1009 (OH), 1686 (Keton konj.), 1619 (C=C konj), 1382, 1358 (gem. Dimethyl).

### Beispiel 55

a) Zu einer Suspension von 2 g (83 mMol) Magnesium in 50 ml zuvor über Natrium destilliertem Tetrahydrofuran werden bei 60° 35 ml einer 4,3 molaren Lösung von Vinylbromid (150 mMol) in Tetrahydrofuran getropft. Dann wird noch während 30 Minuten unter Rückfluss gekocht. Nach Abkühlung auf -5° wird tropfenweise mit einer Lösung von 10 g (36 mMol) (all-E)-4,8-dimethyl-10-[(tetrahydro-2H-pyran-2-yl)oxy] -4,8-decadienal in 50 ml absolutem Tetrahydrofuran versetzt. Nach beendeter Zugabe (ca. 20 Minuten) wird noch während 1 Stunde bei 0° weitergerührt. Dann wird die Reaktionsmischung in 500 ml einer gesättigten Lösung von Ammoniumchlorid gegossen. Nach dreimaliger Extraktion mit Aether werden die vereinigten organischen Phasen zuerst mit gesättigter Natriumhydrogencarbonatlösung und dann mit gesättigter Kochsalzlösung gewaschen. Nach Trocknen über Natriumsulfat, Filtration und Einengen erhält man (all-E)-6,10-Dimethyl-12-[(tetrahydro-2H-pyran-2-yl)oxy] -1,6,10-dodecatrien-3-ol in Form eines gelblichen Oels.
b) Eine Mischung von 11,2 g (36 mMol) des erhaltenen rohen (all-E)-6,10-Dimethyl-12-[(tetrahydro-2H-pyran-2-yl)oxy] -1,6,10-dodecatrien-3-ols, 50 g Mangandioxid (aktiv gefällt) und 500 ml Methylenchlorid wird bei Raumtempratur gerührt. Nach jeweils einigen Stunden wird filtriert und mit frischem Mangandioxid versetzt. Nach insgesamt 30 Stunden und viermaligem Versetzen mit frischem Mangandioxid filtriert man über eine Hyflo-Schicht und dampft das Filtrat ein. Als Rückstand verbleibt rohes (all-E)-6,10-Dimethyl-12-[(tetrahydro-2H-pyran-2-yl)oxy] -1,6,10-dodecatrien-3-on in Form eines gelben Oels; man löst dieses in 750 ml 90% Aethanol, gibt 1,2 g Pyridinium-toluol-4-sulfonat zu und rührt während 3,5 Stunden bei 55°. Dann engt man das Gemisch ein, nimmt den Rückstand in Aether auf und wäscht mit Eiswasser. Die organische Phase wird zuerst mit gesättigter Natriumhydrogencarbonatlösung und dann mit gesättigter Kochsalzlösung gewaschen, über Natriumsulfat getrocknet, filtriert und eingeengt. Der Rückstand wird an Kieselgel mit Hexan-Essigester 2:1 chromatographiert, wobei (all-E)-12-Hydroxy-6,10-dimethyl-1,6,10-dodecatrien-3-on als farbloses Oel erhalten wird. MS: 195 (M-C₂H₃)⁺, 189 (M-(H₂O+CH₃))⁺.

### Beispiel 56

a) Eine Mischung von 2,4 g (100 mMol) Magnesium, 100 ml absolutem Tetrahydrofuran und ca. 100 mg Jod wird in Argonatmosphäre auf Rückflusstemperatur erhitzt. Nach Verschwinden der Jod-Farbe werden 12,0 g (89 mMol) (Z)-2-Brom-2-buten zugetropft. Dann wird während 2,5 Stunden am Rückfluss gekocht. Nach Abkühlen auf Raumtemperatur wird die Mischung im Argonstrom durch Glaswolle filtriert. Die erhaltene Grignardlösung wird anschliessend auf -10° abgekühlt und tropfenweise mit 12,0 g (43 mMol) (all-e)-4,8-Dimethyl-10-[(tetrahydro-2H-pyran-2-yl)oxy] -4,8-decadienal versetzt. Nach beendeter Zugabe wird noch während 20 Stunden bei 15° gerührt. Dann versetzt man mit gesättigter Ammoniumchloridlösung und extrahiert anschliessend zweimal mit Aether. Die vereinigten organischen Phasen werden über Magnesiumsulfat getrocknet, filtriert und eingeengt. Der Rückstand wird an Kieselgel mit Hexan-Essigester 9:1 chromatographiert, wobei man (7E,11E)-3,7,11-Trimethyl-13-[(tetrahydro-2H-pyran-2-yl)oxy] -2,7,11-tridecatrien-4-ol in einem 2Z:2E-Verhält nis von 89%:11% (GC und ¹H-NMR) erhält. MS: 234 (M-(2-Hydroxy-tetrahydropyran, C₅H₁₀O₂))⁺.
b) Eine Mischung von 4,5 g (13,3 mMol) des erhaltenen (2(Z/E),7E,11E)-3,7,11-Trimethyl-13-[(tetrahydro-2H-pyran -2-yl)- oxy]-2,7,11-tridecatrien-4-ols, 130 ml Methanol und 7,0 g stark saurem Ionenaustauscher (Dowex 50 W 8 X) wird während 3 Stunden bei Raumtemperatur gerührt. Nach Filtration und Einengen des Filtrats wird der Rückstand an Kieselgel (0,043-0,060 mm) mit Hexan-Essigester 1:1 unter einem Druck von ca. 0,1 bar Pressluft chromatographiert, wobei man (2E,6E)-3,7,11-trimethyl-2,6,11-tridecatrien-1,10-diol in einem 11Z:11E-Verhältnis von 90%:10% (GC und ¹³C-NMR) erhält. MS: 178 (M-C₄H₈+H₂O))⁺.

### Beispiel 57

a) Eine Mischung von 10 g (29,7 mMol) (2(Z/E),7E,11E)-2,7,11- -Trimethyl-13-[(tetrahydro-2H-pyran -2-yl)oxy]-2,7,11-tridecatrien-4-ol, 350 ml Methylenchlorid und 150 g Mangandioxid (aktiv gefällt) wird während 4 Tagen bei Raumtemperatur gerührt. Nach Filtration über eine Hyflo-Schicht und Einengen des Filtrats wird der Rückstand an Kieselgel (0,043-0,060 mm) mit Hexan-Essigester 80:20 unter einem Druck von ca. 0,1 bar Pressluft chromatographiert, wobei man neben nicht umgesetztem Ausgangsmaterial (2(E/Z),7E,11E)-3,7,11-trimethyl-13-[(tetrahydro-2H-pyran -2-yl)oxy]-2,7,11-tridecatrien-4-on erhält. Das 2(E/Z)-Verhältnis beträgt gemäss Gaschromatogramm und ¹³C-NMR ca. 25%: 75%. MS: 249 (M-Tetrahydropyran, C₅H₈O)⁺, 232 (M-(2-Hydroxy-tetrahydropyran, C₅H₁₀O₂))⁺.
b) Zu einer Suspension von 4 g stark saurem Ionenaustauscher (Dowex 50 W 8 X) in 100 ml Methanol wird bei Raumtemperatur eine Lösung von 2,2 g (6,5 mMol) (2(E/Z),7E,11E)-3,7,11-Trimethyl-13-[(tetrahydro-2H-pyran -2-yl)oxy]-2,7,11-tridecatrien-4-on getropft. Anschliessend wird noch 2 Stunden bei Raumtemperatur weitergerührt. Nach Filtration und Eindampfen des Filtrats wird der Rückstand an Kieselgel mit Hexan-Essigester 1:1 unter einem Druck von ca. 0,1 bar Pressluft chromatographiert, wobei man (2(E/Z),7E,11E)-13-Hydroxy-3,7,11-trimethyl-2,7,11 -tridecatrien-4-on als leicht gelbliches Oel erhält. Das 2Z:2E-Verhältnis beträgt gemäss Gaschromatographie und ¹H-NMR 61,5%:38,5%. MS (CI mit NH₃): 233 ((M+H)-H₂O))⁺.

### Beispiel 58

a) Zu 1,2 g (172 mMol) metallischem Lithium in 100 ml absolutem Aether werden bei 0° 10 g (84 mMol) Cyclopropylbromid getropft. Anschliessend wird noch während 90 Minuten bei Raumtemperatur gerührt. Nach Abfiltrieren des überschüssigen Lithiums wird das Filtrat auf -70° abgekühlt und tropfenweise mit 20 g (76,4 mMol) (all-E)-4,8-dimethyl-10-[(tetrahydro-2H-pyran-2-yl)oxy] -4,8-decadienal versetzt. Man lässt langsam auf Raumtemperatur erwärmen und rührt dann noch 20 Minuten weiter. Anschliessend kühlt man auf 0° ab, versetzt mit 100 ml gesättigter Ammoniumchloridlösung und extrahiert zweimal mit Aether. Die vereinigten Aetherphasen werden über Natriumsulfat getrocknet, filtriert und eingeengt. Der Rückstand wird an Kieselgel mit Hexan-Essigester 9:1 chromatographiert, wonach man α-[(all-E)-3,7-Dimethyl-9-[(tetrahydro-2H-pyran-2-yl)oxy] -3,7-nonadienyl]cyclopropanmethanol erhält. MS: 238 (M-Dihydropyran, C₅H₈O)⁺.
b) Eine Mischung von 2,5 g (7,75 mMol) α-[(all-E)-3,7-Dimethyl-9-[(tetrahydro-2H-pyran-2-yl)oxy] -3,7-nonadienyl]cyclopropanmethanol, 50 ml 90% Aethanol und 187 mg Pyridinium-toluol-4-sulfonat wird während 6 Stunden bei 50° gerührt. Man dampft ein, nimmt den Rückstand in Aether auf und wäscht mit Wasser. Nach Extraktion der Wasserphase mit Aether werden die vereinigten Aetherphasen über Natriumsulfat getrocknet, filtriert und eingeengt. Der Rückstand wird an Kieselgel mit Hexan-Essigester 9:1 chromatographiert, wonach man (all-E)-10-Cyclopropyl-3,7-dimethyl-2,6-decadien-1,10-diol erhält. MS: 220 (M-H₂O)⁺.

### Beispiel 59

a) Eine Mischung von 10 g (31 mMol) α-[(all-E)-3,7-Dimethyl-9-[(tetrahydro-2H-pyran-2-yl)oxy] -3,7-nonadienyl]cyclopropanmethanol, 500 ml Methylenchlorid und 200 g Mangandioxid (aktiv gefällt) wird während 6 Stunden bei Raumtemperatur gerührt. Nach Filtration über Natriumsulfat und Eindampfen des Filtrats wird der Rückstand an Kieselgel mit Hexan-Essigester 9:1 chromatographiert, wonach man (all-E)-1-Cyclopropyl-4,7-dimethyl-10-[(tetrahydro-2H-pyran -2-yl)-oxy]-4,8-decadien-1-on erhält. MS: 236 (M-Dihydropyran, C₅H₈O)⁺.
b) Eine Mischung von 3,3 g (10,3 mMol) (all-E)-1-Cyclopropyl-4,7-dimethyl-10-[(tetrahydro-2H-pyran -2-yl)oxy]-4,8-decadien-1-on, 60 ml Aethanol und 241 mg Pyridinium-toluol-4-sulfonat wird während 1 Stunde bei 50° gerührt. Dann engt man die Reaktionsmischung ein, nimmt den Rückstand in Aether auf und wäscht mit Wasser. Nach Extraktion der Wasserphase mit Aether werden die vereinigten Aetherphasen über Natriumsulfat getrocknet, filtriert und eingeengt. Der Rückstand wird an Kieselgel mit Hexan-Essigester 8:2 chromatographiert. Man erhält (all-E)-1-Cyclopropyl-4,8-dimethyl-10-hydroxy-4,8 -decadien-1-on. MS: 218 (M-H₂O)⁺.

### Beispiel 60

Zu einer erwärmten Mischung von 1,7 g (72 mMol) Magnesiumspänen, 50 ml zuvor über Natrium destilliertem Tetrahydrofuran und einem Jodkristall werden 4,2 g (36 mMol) (E/Z)-1-Brom-1-propen in einer solchen Geschwindigkeit zugetropft, dass die Reaktionsmischung gerade am Sieden erhalten wird. Nach beendeter Zugabe wird noch 1 Stunde unter Rückfluss gekocht. Nach Abkühlen wird in Argonatmosphäre von festen Bestandteilen abfiltriert. Die auf 0° abgekühlte Grignardlösung wird anschliessend innerhalb von 15 Minuten tropfenweise mit einer Lösung von 5 g (18 mMol) (all-E)-4,8-Dimethyl-10-[(tetrahydro-2H-pyran-2-yl)oxy] -4,8-decadienal in 5 ml absolutem Tetrahydrofuran versetzt. Anschliessend wird noch 1 Stunde bei 0° und 4 Stunden bei Raumtemperatur gerührt. Dann giesst man die Reaktionsmischung in 150 ml gesättigte Ammoniumchloridlösung und extrahiert dreimal mit Aether. Die vereinigten organischen Phasen werden zuerst mit gesättigter Natriumhydrogencarbonatlösung und dann mit gesättigter Kochsalzlösung gewaschen, anschliessend über Natriumsulfat getrocknet, filtriert und eingedampft.

Das als Rückstand verbleibende (2(E/Z),7E,11E)-7,11-Dimethyl-13-[(tetrahydro-2H-pyran -2-yl]oxy]-2,7,11-tridecatrien-4-ol wird zusammen mit 100 ml Methanol und 10 g stark saurem Ionenaustauscher (Dowex 50 W 8 X) während 3 Stunden bei Raumtemperatur gerührt. Nach Filtration und Einengen wird der Rückstand an Kieselgel mit Hexan-Essigester 1:1 chromatographiert, wonach man (2E,6E,11(E/Z))-3,7-Dimethyl-2,6,11-tridecatrien-1,10-diol in einem E:Z-Ver-hältnis von 83:17 (GC) erhält. MS (CI mit NH₃): 238 ((M+NH₄)-H₂O)⁺, 221 ((M+H)-H₂O)⁺, 220
((M+NH₄)-2H₂O)⁺, 203 ((M+H)-2H₂O)⁺.

### Beispiel 61

a) Eine Mischung von 17,9 g (56 mMol) (2(E/Z),7E,11E)-7,11-Dimethyl-13-[(tetrahydro-2H-pyran -2-yl)oxy]-2,7,11-tridecatrien-4-ol, hergestellt nach den Angaben in Beispiel 61 und an Kieselgel mit Hexan-Essigester 4:1 unter Zusatz von 0,1% Triäthylamin chromatographiert, 400 ml Methylenchlorid und 100 g Mangandioxid (aktiv gefällt) wird bei Raumtemperatur gerührt. Nach einer Reaktionsdauer von jeweils einigen Stunden filtriert man und gibt frisches Mangandioxid zu. Insgesamt wird so bei einer Reaktionsdauer von 5 Tagen vier mal frisches Mangandioxid zugegeben. Dann filtriert man die Reaktionsmischung durch eine Mischung aus Hyflo und Natriumsulfat und dampft das Filtrat ein. Das als Rückstand verbleibende gelbliche Oel wird an Kieselgel mit Hexan-Essigester 4:1 unter Zusatz von 0,1% Triäthylamin chromatographiert, wonach man (all-E)-7,11-Dimethyl-13-[(tetrahydro-2H-pyran-2-yl)oxy] -2,7,11-tridecatrien-4-on als farbloses Oel erhält. IR (film): 1693 (Keton konj.), 1620 (-C=C-, konj.), 974 (-CH=CH-, trans).
b) Eine Mischung von 10,0 g (30 mMol) (all-E)-7,11-Dimethyl-13-[(tetrahydro-2H-pyran-2-yl)oxy] -2,7,11-tridecatrien-4-on, 250 ml Aethanol, 50 ml Wasser und 2,0 g Pyridinium-toluol-4-sulfonat wird während 3 Stunden bei 55° gerührt. Dann dampft man ein, versetzt den Rückstand mit 100 ml Toluol, engt erneut ein, gibt 500 ml Aether zu und filtriert durch eine Schicht Kieselgel. Das Filtrat wird eingeengt, und der in Form eines gelblichen Oels verbleibende Rückstand wird an Kieselgel mit Hexan-Essigester 2:1 chromatographiert. Man erhält (all-E)-13-Hydroxy-7,11-dimethyl-2,7,11-tridecatrien-4-on als farbloses Oel. Ms: 205 (M-CH₂OH)⁺.

### Beispiel 62

Eine Lösung von 2 g (20 mMol, 2.82 ml) Trimethylsilylacetylen in 40 ml absolutem Tetrahydrofuran wird bei -70° tropfenweise mit 12,5 ml (20 mMol) einer 1,6 molaren Lösung von Butyllithium in Hexan versetzt. Nach beendeter Zugabe wird noch 30 Minuten bei -70° gerührt, worauf innerhalb von 15 Minuten eine Lösung von 5 g (17,8 mMol) (all-E)-4,8-Dimethyl-10-[(tetrahydro-2H-pyran-2-yl)oxy] -4,8-decadienal in 40 ml absolutem Tetrahydrofuran zugetropft wird. Anschliessend wird noch 30 Minuten bei -70° gerührt, und dann wird die Reaktionsmischung in 500 ml einer eiskalten, gesättigten Ammoniumchloridlösung gegossen. Nach dreimaliger Extraktion mit Hexan werden die vereinigten organischen Phasen über Natriumsulfat getrocknet, filtriert und eingeengt, wonach man rohes (all-E)-6,10-Dimethyl-12-[(tetrahydro-2H-pyran-2-yl)oxy] -1-(trimethylsilyl)-6,10-dodecadien-1-in-3-ol erhält.

Dieses Material wird bei 0° innerhalb von 15 Minuten zu einer Suspension von 63 g Mangandioxid (aktiv gefällt) getropft. Anschliessend wird noch 4 Stunden bei 0° weitergerührt. Nach Filtration über Natriumsulfat und Einengen erhält man rohes (all-E)-6,10-Dimethyl-12-[(tetrahydro-2H-pyran-2-yl)oxy] -1-(trimethylsilyl)-6,10-dodecadien-1-in-3-on.

3,8 g (10 mMol) dieses Materials werden zusammen mit 800 mg Pyridinium-toluol-4-sulfonat und 100 ml Aethanol während 1 Stunde bei 55° gerührt. Nach Einengen wird der Rückstand an Kieselgel mit Hexan-Essigester 9:1 unter einem Druck von ca. 0,1 bar Pressluft chromatographiert, wonach man (all-E)-12-Hydroxy-6,10-dimethyl-1-(trimethylsilyl) -6,10-dodecadien-1-in-3-on erhält. MS: 261 (M-CH₂OH)⁺, 259 (M-(CH₃+H₂O)⁺.

### Beispiel 63

Zu einer Mischung von 11,4 g (140 mMol) wasserfreiem Natriumacetat, 29 g (0,2 Mol) Natriumsulfat, 10,85 g (0,13 Mol) O-Methylhydroxylamin-hydrochlorid und 110 ml absolutem Methanol werden bei Raumtemperatur innerhalb von 10 Minuten 4,57 g (20,7 mMol) (all-E)-12-Hydroxy-6,10-dimethyl-6,10-dodecadien-1-in-3-on getropft. Dann wird die Reaktionsmischung auf 66-68° erwärmt und während 1 Stunde gerührt. Dann lässt man abkühlen, filtriert, engt das Filtrat ein und nimmt den Rückstand in 500 ml destilliertem Wasser auf. Dann wird zweimal mit Aether extrahiert, worauf die vereinigten Auszüge mit gesättigter Kochsalzlösung gewaschen und anschliessend über Natriumsulfat filtriert werden. Nach Einengen des Filtrats wird der Rückstand an Kieselgel mit Hexan-Essigester 2:1 chromatographiert. Man erhält (6E,10E)-12-Hydroxy-6,10-dimethyl-6,10-dodecadien-1-in -3-on-(E/Z)-O-methyloxim; das E/Z-Verhältnis beträgt gemäss GC und NMR etwa 1:2. MS: 218 (M-OCH₃)⁺.

### Beispiel 64

a)Eine Mischung von 12 g (37,2 mMol) (all-E)-3,7,11-Trimethyl-10-[(tetrahydro-2H-pyran-2-yl)oxy] -2,6,11-dodecatrien-1-ol, 120 g Mangandioxid und 500 ml Hexan wird während 1 Stunde bei Raumtemperatur gerührt. Dann filtriert man über Kieselgur, engt das Filtrat ein und chromatographiert den Rückstand an Kieselgel mit Hexan-Essigester unter Zusatz von 0,1% einer 25% Ammoniaklösung. Man erhält (all-E)-3,7,11-Trimethyl-10-[(tetrahydro-2H-pyran-2-yl)oxy] -2,6,11-dodecatrienal. MS: 218 (M-(2-Hydroxy-tetrahydropyran, C₅H₁₀O₂))⁺.
b) Zu 100 ml (0,15 Mol) einer ca. 1,5M Lösung von Propin in abs. Tetrahydrofuran werden bei -70° innerhalb von 20 Minuten 48,7 ml (78 mMol) einer 1,6M Lösung von Butyllithium in Hexan getropft. Nach 1-stündigem Rühren bei -70° wird bei gleicher Temperatur eine Lösung von 7,25 g (22,6 mMol) von (all-E)-3,7,11-trimethyl-10-[(tetrahydro-2H-pyran-2-yl)oxy] 2,6,11-dodectrienal in 10 ml absolutem Tetrahydrofuran zugetropft, worauf noch während 30 Minuten bei -70° gerührt wird. Dann wird die Reaktionsmischung langsam in 500 ml einer eiskalten gesättigten Ammoniumchloridlösung gegossen, worauf kräftig geschüttelt wird. Nach dreimaliger Extraktion mit Aether werden die vereinigten organischen Phasen über Natriumsulfat getrocknet und eingeengt. Der Rückstand wird an Kieselgel mit Hexan-Essigester 4:1 unter Zusatz von 0,1% Triäthylamin chromatographiert. Man erhält (all-E)-6,10,14-Trimethyl-13-[(tetrahydro-2H-pyran-2-yl)oxy] -5,9,14-pentadecatrien-2-in-4-ol. IR (Film): 3427 (OH), 2232 (-C≡C-), 1135, 1116, 1076 (-C-O-C-).
c) Eine Suspension von 62 g Mangandioxid (aktiv gefällt) in 200 ml Methylenchlorid wird bei 0° mit 6,12 g (17,1 mMol) (all-E)-6,10,14-Trimethyl-13-[(tetrahydro-2H-pyran-2-yl)oxy] 5,9,14-pentadecatrien-2-in-4-ol versetzt. Nach Erwärmen auf Raumtemperatur wird noch während 45 Minuten gerührt. Dann filtriert man über Natriumsulfat und engt das Filtrat ein. Das als Rückstand verbleibende (5(E/Z),9E)-6,10,14-Trimethyl-13-[(tetrahydro-2H-pyran -2-yl)oxy]-5,9,14-pentadecatrien-2-in-4-on wird zusammen mit 450 mg Pyridinium-toluol-4-sulfonat und 80 ml 90% Aethanol während 20 Stunden bei 55° gerührt. Dann engt man ein und nimmt den Rückstand in Aether auf. Nach zweimaligem Waschen mit Wasser wird die organische Phase noch einmal mit gesättigter Kochsalzlösung gewaschen, über Natriumsulfat filtriert und eingeengt. Der Rückstand wird an Kieselgel mit Hexan-Essigester 4:1 chromatographiert, wonach man (5(E/Z),9E)-13-Hydroxy-6,10,14-trimethyl-5,9,14 -pentadecatrien-2-in-4-on in einem (E/Z)-Verhältnis von 2:1 (GC, NMR) erhält. MS: 241 (M-(CH₃+H₂O))⁺.

### Beispiel 65

a) In einem 500 ml Sulfierkolben, versehen mit mechanischem Rührer, Tropftrichter und Thermometer, werden unter Argonatmosphäre 4,86 g Magnesiumspäne, 100 mg Quecksilber(II)-chlorid und 100 ml Aether vorgelegt. Der Tropftrichter wird mit einer Lösung von 23,8 g (15,1 ml) Propargylbromid in 50 ml Aether beschickt, und es wird 1,0 ml dieser Lösung bei Raumtemperatur zum obigen Gemisch getropft. Nach Beginn der Reaktion wird das Gemisch auf -20° gekühlt, und der Rest der Propargylbromidlösung wird innerhalb von 3 Stunden so zugetropft, dass die Reaktionstemperatur -15° nicht übersteigt. Nach beendigter Zugabe wird noch 15 Minuten bei -20° gerührt. Dann wird bei 0° eine Lösung von 28,0 g (E,E)-4,8-Dimethyl-10-[(tetrahydro-2H-pyran-2-yl)oxy] -4,8-decadienal in 50 ml Aether zugetropft. Nach Zugabe der Hälfte dieser Lösung entsteht ein Niederschlag, worauf zur Erleichterung des Rührens 100 ml Aether und 100 ml Tetrahydrofuran zugegeben werden. Der Rest der erwähnten Lösung wird anschliessend bei 5-10° zugetropft, und die resultierende graue Suspension wird über Nacht im schmelzenden Eisbad gerührt und dann auf ein Gemisch von Eis und gesättigter Ammoniumchloridlösung gegossen. Die organische Phase wird abgetrennt, und die wässrige Phase wird zweimal mit Hexan extrahiert. Die vereinigten organischen Phasen werden mit gesättigter Ammoniumchloridlösung, gesättigter Natriumbicarbonatlösung und gesättigter Kochsalzlösung gewaschen, über Natriumsulfat getrocknet, filtriert und eingedampft. Der Rückstand wird an 1,0 kg Kieselgel mit Hexan-Essigsäureäthylester 10%→25% chromatographiert. Man erhält (E,E)-7,11-Dimethyl-13-[(tetrahydro-2H-pyran-2-yl)oxy] 7,11-tridecadien-1-in-4-ol als gelbes Oel. IR (Film): 3450 (OH); 3307, 2118 (-C≡C-H); 1667 (C=C). MS: 236 (1, (M-Dihydropyran, C₅H₈O)⁺); 218 (0,5, (M-(2-Hydroxy-tetrahydropyran, C₅H₁₀O₂))⁺); 85 (100, C₅H₉O⁺).
b) Eine Lösung von 6,40 g (E,E)-7,11-Dimethyl-13-[(tetrahydro-2H-pyran-2-yl)oxy] -7,11-tridecadien-1-in-4-ol und 1,50 g Pyridinium-toluol-4-sulfonat in 200 ml abs. Aethanol wird unter Argon während 2,5 Stunden auf 60° erhitzt. Die Reaktionsmischung wird abgekühlt und eingedampft, und der Rückstand wird in Aether und Wasser aufgenommen. Nach Abtrennen der organischen Phase wird die wässrige Phase noch zweimal mit Aether extrahiert, und die vereinigten organischen Phasen werden mit 2N Salzsäure, gesättigter Natriumbicarbonatlösung und gesättigter Kochsalzlösung gewaschen, über Natriumsulfat getrocknet, filtriert und eingedampft. Das als Rückstand verbleibende gelbe Oel wird an 400 g Kieselgel chromatographiert, wobei mit Hexan-Essigsäureäthylester 25%→40% eluiert wird. Auf diese Weise wird (E,E)-3,7-Dimethyltrideca-2,6-dien-12-in-1,10-diol als gelbliches Oel gewonnen. IR (film); 3350 (OH); 3305, 2118 (-C≡CH); 1667 (C=C); 1003 (OH). MS: 205 (1, (M-CH₂OH)⁺); 203 (1, (M-H₂O-CH₃)⁺); 105 (100); 93 (100).

### Beispiel A

Kristalline Verbindungen der Formel I können als Wirkstoffe zur Herstellung von Hartgelatinekapseln verwendet werden, deren Inhalt pro Kapsel folgende Zusammensetzung aufweist:

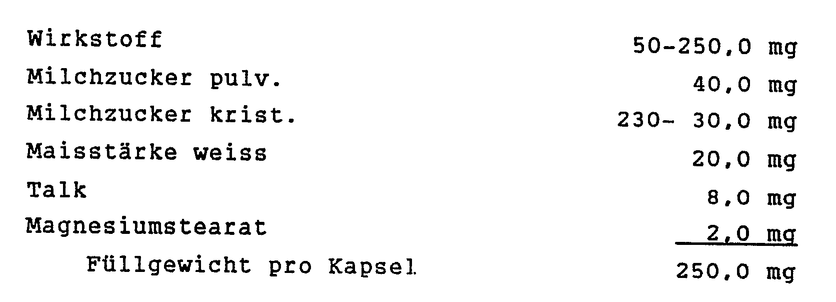

Der Wirkstoff und die Hilfsstoffe werden miteinander vermischt, und das Gemisch wird in Hartgelatinekapseln geeigneter Grösse abgefüllt. Erforderlichenfalls werden die Kapseln anschliessend mit einem magensaftresistenten Lack, bestehend aus Hydroxypropylmethylcellulosephthalat, versehen.

### Beispiel B

Nicht kristalline Verbindungen der Formel I können wie nachstehend beschrieben als Wirkstoffe zur Herstellung von Weichgelatinekapseln verwendet werden; die verwendeten Abkürzungen haben dabei folgende Bedeutung:
BHA = Butyliertes Hydroxyanisol
BHT = Butyliertes Hydroxytoluol
PEG = Polyäthylenglykol
a) 0,2 mg BHA und 1,0 mg Ascorbylpalmitat werden in 400 mg PEG 400 bei Raumtemperatur unter Stickstoffatmosphäre gelöst. Die Lösung wird mit 50-250 mg Wirkstoff bei Raumtemperatur unter Stickstoff versetzt. Nachdem alles gelöst ist, wird das erhaltene Gemisch in flüssiger Form in Weichgelatinekapseln abgefüllt.
b) 300 mg PEG 400 und 100 mg PEG 4000 werden unter Stickstoff erwärmt, bis sich das Gemisch verflüssigt hat. Danach werden unter Stickstoff 0,1 mg BHA, 0,1 mg BHT und 1,0 mg Ascorbylpalmitat zugesetzt. Nachdem sich alles gelöst hat, werden 50-250 mg Wirkstoff unter Stickstoff zugefügt und unter Durchmischung gelöst. Die Flüssigkeit wird dann in Weichgelatinekapseln abgefüllt.
c) 0,2 mg BHA, 0,2 mg BHT und 1,0 mg Ascorbylpalmitat werden in 400 mg Polysorbat-80 bei Raumtemperatur unter Stickstoff gelöst. Das Gemisch wird unter Stickstoff mit 50-250 mg Wirkstoff versetzt. Nachdem sich alles gelöst hat, wird die Flüssigkeit in Weichgelatinekapseln abgefüllt.
d) Ein Gemisch von je 200 mg Polysorbat-60 und Polysorbat-80 wird erwärmt. Das erhaltene flüssige Gemisch wird unter Stickstoff mit 0,2 mg BHA, 1,0 mg α-Tocopherol und 2,0 mg Ascorbylpalmitat versetzt. Nachdem alles gelöst ist, werden 50-250 mg Wirkstoff unter Stickstoff zugesetzt. Nach Durchmischung bis zur vollständigen Lösung wird das erhaltene Gemisch in Weichgelatinekapseln abgefüllt.

## Patentansprüche

1. Verbindungen der allgemeinen Formel 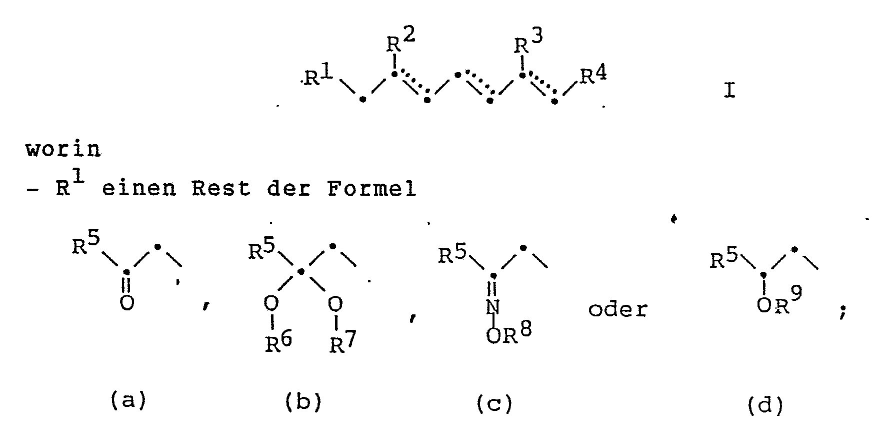 - eines von R² und R³ (C₁-C₈)-Alkyl und das andere Wasserstoff oder (C₁-C₈)-Alkyl;
- R⁴ einen Rest der Formel 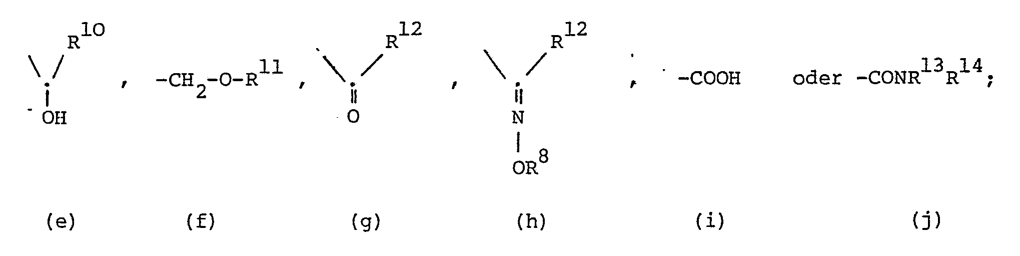 -R⁵ (C₂-C₈)-Alkyl, nicht jedoch Isopropyl, (C₃-C₈)-Cycloalkyl, (C₂-C₈)-Alkenyl, (C₂-C₈)-Alkinyl, Tri-(C₁-C₈)-alkyl-silyläthinyl oder, falls R¹ einen Rest der Formel (a) bedeutet, auch einen Rest der Formel 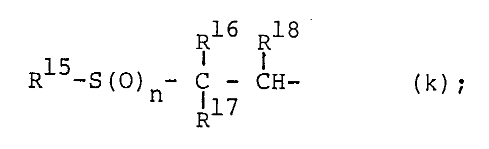 - R⁶ und R⁷ je (C₁-C₈)-Alkyl oder zusammen eine gegebenenfalls durch eine oder zwei (C₁-C₈)-Alkyl-, (C₁-C₈)-Alkoxy-(C₁-C₈)-alkyl- oder (C₁-C₈)-Alkoxycarbonylgruppen substituierte Di- oder Trimethylengruppe;
- R⁸ Wasserstoff oder (C₁-C₈)-Alkyl;
- R⁹ Wasserstoff oder (C₂-C₈)-Alkanoyl;
- R¹⁰ (C₁-C₈)-Alkyl;
- R¹¹ Wasserstoff, (C₁-C₈)-Alkyl, (C₂-C₈)-Alkanoyl oder einen Rest der Formel 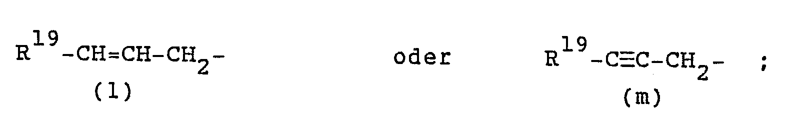 - R¹² Wasserstoff, (C₁-C₈)-Alkyl, (C₂-C₈)-Alkenyl oder (C₂-C₈)-Alkinyl;
- R¹³ und R¹⁴ je Wasserstoff oder (C₁-C₈)-Alkyl oder zusammen mit dem Stickstoffatom 1-Pyrrolidinyl, Piperidino oder Morpholino;
- R¹⁵ (C₁-C₈)-Alkyl, (C₂-C₈)-Alkanoyl, (C₁-C₈)-alkoxycarbonyl-(C₁-C₈)-alkyl, Hydroxy-(C₂-C₈)-alkyl, (C₂-C₈)-Alkanoyloxy-(C₂-C₈)-alkyl, (C₂-C₈)-Alkanoylamino-(C₂-C₈)-alkyl oder durch (C₂-C₈)-Alkanoylamino und (C₁-C₈)-Alkoxycarbonyl oder durch (C₂-C₈)-Alkanoyloxy und (C₁-C₈)-Alkoxycarbonyl disubstituiertes (C₂-C₈)-Alkyl;
- n die Zahl 0, 1 oder 2;
- R¹⁶, R¹⁷ und R¹⁸ je Wasserstoff oder (C₁-C₆)-Alkyl, enthaltend insgesamt höchstens 6 C-Atome;
- R¹⁹ Wasserstoff oder (C₁-C₅)-Alkyl;
- jede der punktierten Linien eine fakultative zusätzliche C-C-Bindung mit E- oder Z-Konfiguration bedeuten; und
- die in den Resten der Formeln (c) und (h) vorhandene Doppelbindung die E- oder Z-Konfiguration aufweist;
sowei pharmazeutisch verwendbare Salze von sauren Verbindungen der Formel I mit Basen,
mit Ausnahme von 10-Hydroxy-3,7,11-trimethyl-2,6,11-dodecatriensäure und 3,7,11-Trimethyl-2,6,11-dodecatrien-1,10-diol.

2. Verbindungen gemäss Anspruch 1, worin R¹ einen Rest der Formel (a), (b), (c) oder (d), R² und R³ je (C₁-C₈)-Alkyl, R⁴ einen Rest der Formel (f), (g), (h), (i) oder (j), R⁵ (C₂-C₈)-Alkenyl, (C₂-C₈)-Alkinyl oder Tri-(C₁-C₈)-alkyl-silyl-äthinyl, R⁶ und R⁷ je (C₁-C₈)-Alkyl oder zusammen die Dimethylengruppe, R⁸ Wasserstoff oder (C₁-C₈)-Alkyl, R⁹ Wasserstoff, R¹¹ Wasserstoff oder (C₂-C₈)-Alkanoyl, R¹², R¹³ und R¹⁴ je Wasserstoff und zwei der punktierten Linien nicht-konjugierte zusätzliche C-C-Bindungen bedeuten.

3. Verbindungen gemäss Anspruch 2, worin R² und R³ je Methyl, R⁵ Vinyl, 1-Methylvinyl, 1-Propenyl, 2-Methyl-1-propenyl, Aethinyl, 1-Propinyl oder Trimethylsilyl-äthinyl, R⁶ und R⁷ je Methyl oder Aethyl oder zusammen die Dimethylengruppe, R⁸ Wasserstoff oder Methyl und R¹¹ Wasserstoff oder Acetyl bedeuten.

4. (all-E)-12-Hydroxy-6,10-dimethyl-6,10-dodecadien-1-in-3-on,
(all-E)-12-Hydroxy-6,10-dimethyl-1,6,10-dodecatrien-3-on,
(all-E)-13-Hydroxy-7,11-dimethyl-7,11-tridecadien-2-in-4-on,
(all-E)-13-Hydroxy-7,11-dimethyl-2,7,11-tridecatrien-4-on und
(all-E)-10,10-Diäthoxy-3,7-dimethyl-2,6-dodecadien-11-in-1-ol.

5. (all-E)-12-Hydroxy-2,6,10-trimethyl-1,6,10-dodecatien-3-ol,
(all-E)-10-Oxo-3,7,11-trimethyl-2,6,11-dodecatrienal,
(6Z,10Z)-12-Hydroxy-2,6,10-trimethyl-1,6,10-dodecatrien-3-on,
(all-E)-3,7-Dimethyl-9-[2-(1-methylvinyl)-1,3-dioxolan-2-yl]-2,6-nonadien-1-ol,
(E-E)-3,7-Trimethyl-10-oxo-2,6,11-dodecatrienamid,
(all-E)-13-Hydroxy-2,7,11-trimethyl-2,7,11-tridecatrien-4-on,
(6E,10Z)-12-Hydroxy-2,6,10-trimethyl-1,6,10-dodecatrien-3-on und
(6Z,10E)-12-Hydroxy-2,6,10-trimethyl-1,6,10-dodecatrien-3-on.

6. (all-E)-10-Oxo-3,7,11-trimethyl-2,6,11-dodecatriensäure;
(all-E)-10,10-Dimethoxy-3,7,11-trimethyl-2,6,11-dodecatrien-1-ol;
(all-E)-10-Hydroxy-3,7,11-trimethyl-2,6,11-dodecatrienal;
(all-E)-12-Hydroxy-6,10-dimethyl-1-(trimethylsilyl)-6,10-dodecadien-1-in-3-on;
(2E,6E)-3,7,11-Trimethyl-10-oxo-2,6,11-dodecatrienal-(E/Z) -oxim;
(6E,10E)-12-Hydroxy-6,10-dimethyl-6,10-dodecadien-1-in-3-on-(E/Z)-O-methyloxim; und
(2E,6E,11(E/Z))-3,7-Dimethyl-2,6,11-tridecatrien-1,10-diol.

7. Verbindungen gemäss einem der Ansprüche 1-6 sowie 10-Hydroxy-3,7,11-trimethyl-2,6,11-dodecatriensäure und 3,7,11-Trimethyl-2,6,11-dodecatrien-1,10-diol zur Anwendung als therapeutische Wirkstoffe, insbesondere bei der Bekämpfung oder Verhütung von Ulcus ventriculi und/oder duodeni.

8. Verfahren zur Herstellung von Verbindungen gemäss einem der Ansprüche 1-6, dadurch gekennzeichnet, dass man
a) von einer Verbindung der allgemeinen Formel 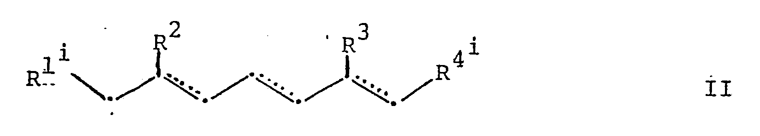 worin R¹ⁱ einen Rest der in Anspruch 1 definierten Formel (a), (b) oder (c) oder einen Rest der Formel 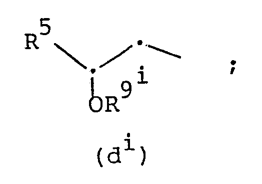 R⁴ⁱ einen Rest der in Anspruch 1 definierten Formel (g), (h), (i) oder (j) oder einen Rest der Formel 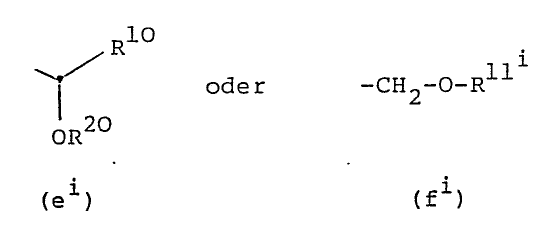 R⁹ⁱ Wasserstoff, (C₂-C₈)-Alkanoyl oder eine Schutzgruppe, R¹¹ⁱ Wasserstoff, (C₁-C₈)-Alkyl, (C₂-C₈)-Alkanoyl, eine Gruppe der in Anspruch 1 definierten Formel (l) oder (m) oder eine Schutzgruppe und R²⁰ Wasserstoff oder eine Schutzgruppe bedeuten und R², R³, R⁵, R¹⁰ und die punktierten Linien die in Anspruch 1 angegebene Bedeutung besitzen, wobei das Molekül mindestens eine Schutzgruppe enthält, die Schutzgruppe(n) abspaltet; oder
b) in einer Verbindung der allgemeinen Formel 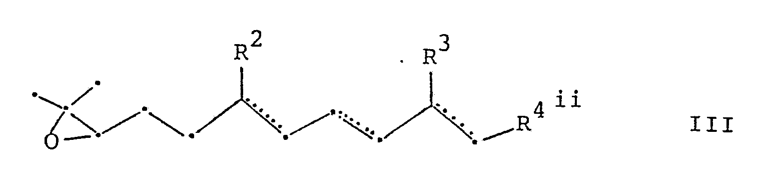 worin R⁴ⁱⁱ einen Rest der in Anspruch 1 definierten Formeln (f), (h), (i) oder (j) bedeutet und R², R³ und die punktierten Linien die in Anspruch 1 angegebene Bedeutung besitzen,
den Epoxidring öffnet; oder
c) einen Carbonsäureester der allgemeinen Formel 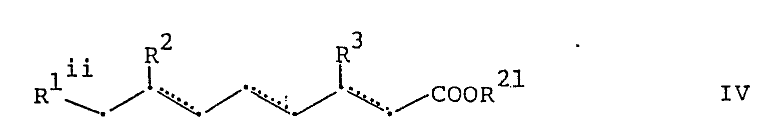 worin R¹ⁱⁱ einen Rest der in Anspruch 1 definierten Formel (b) oder einen Rest der Formel 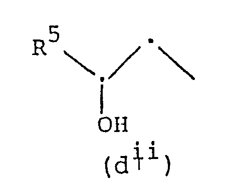 und R²¹ (C₁-C₈)-Alkyl bedeuten und R², R³, R⁵ und die punktierten Linien die in Anspruch 1 angegebene Bedeutung besitzen,
zum entsprechenden primären Alkohol reduziert; oder
d) einen Carbonsäureester der allgemeinen Formel 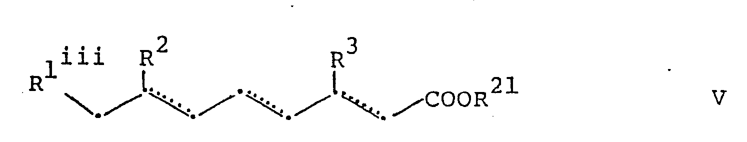 worin R¹ⁱⁱⁱ einen Rest der in Anspruch 1 definierten Formel (b) oder (c) oder der obigen Formel (dⁱⁱ) oder der Formel 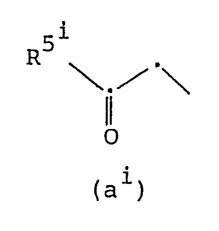 R⁵ⁱ (C₂-C₈)-Alkyl, nicht jedoch Isopropyl, (C₃-C₈)-Cycloalkyl, (C₂-C₈)-Alkenyl, (C₂-C₈)-Alkinyl oder Tri-(C₁-C₈)-alkyl-silyl-äthinyl bedeuten, R²¹ obige Bedeutung besitzt und R², R³ und die punktierten Linien die in Anspruch 1 angegebene Bedeutung besitzen,
zur entsprechenden Carbonsäure hydrolysiert; oder
e) in einer Verbindung der in Anspruch 1 definierten Formel I, worin R¹ einen Rest der in Anspruch 1 definierten Formel (b), (c) oder (d) oder einen Rest der obigen Formel (aⁱ) bedeutet, wobei das Molekül mindestens eine an ein C-Atom gebundene Hydroxygruppe enthält, diese Hydroxygruppe(n) oder eine davon zu (einer) Oxo-gruppe(n) oxydiert; oder
f) eine Verbindung der in Anspruch 1 definierten allgemeinen Formel I, worin R¹ einen Rest der in Anspruch 1 definierten Formel (b) oder (d) und R¹ eine Formylgruppe bedeuten, mit einem eine (C₁-C₈)-Alkylgruppe, eine (C₂-C₈)-Alkenylgruppe oder eine (C₂-C₈)-Alkinylgruppe liefernden Grignard-Reagens behandelt; oder
g) eine Verbindung der in Anspruch 1 definierten Formel I, worin R¹ einen Rest der in Anspruch 1 definierten Formel (b), (c) oder (d) oder einen Rest der obigen Formel (aⁱ) bedeutet, wobei das Molekül mindestens eine Oxogruppe enthält, mit einer Verbindung der allgemeinen Formel
H₂N-OR⁸ VI
worin R⁸ die in Anspruch 1 angegebene Bedeutung besitzt,
umsetzt; oder
h) eine Verbindung der in Anspruch 1 definierten Formel I, worin R¹ einen Rest der Formel 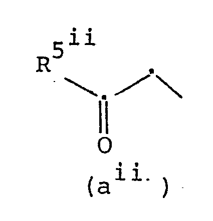 R⁴ einen Rest der in Anspruch 1 definierten Formel (e), (h), (i) oder (j) oder einen Rest der obigen Formel (fⁱ) und R⁵ⁱⁱ (C₂-C₈)-Alk-1-enyl bedeuten,
mit einer Verbindung der allgemeinen Formel
R¹⁵-S-H VII
worin R¹⁵ die in Anspruch 1 angegebene Bedeutung besitzt,
umsetzt; oder
i) in einer Verbindung der in Anspruch 1 definierten Formel I, worin R¹ einen Rest der Formel 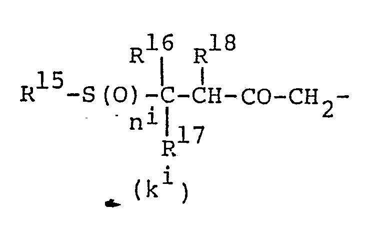 und nⁱ die Zahl 0 oder 1 bedeuten und R¹⁵, R¹⁶, R¹⁷ und R¹⁸ die in Anspruch 1 angegebene Bedeutung besitzen,
die Mercaptogruppe zur Sulfinyl- oder Sulfonylgruppe bzw. die Sulfinyl- zur Sulfonylgruppe oxydiert; oder
j) in einer Verbindung der in Anspruch 1 definierten Formel I, worin R¹ einen Rest der in Anspruch 1 definierten Formel (a), (b) oder (d) oder einen Rest der Formel 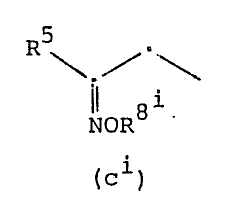 R⁴ einen Rest der in Anspruch 1 definierten Formel (e), (f), (g), (i) oder (j) oder einen Rest der Formel 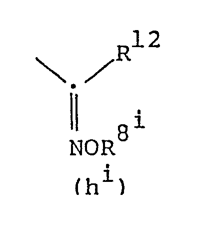 und R⁸ⁱ (C₁-C₈)-Alkyl bedeuten und R⁵ und R¹² die in Anspruch 1 angegebene Bedeutung besitzen,
wobei das Molekül mindestens eine Hydroxygruppe enthält, diese Hydroxygruppe(n) mit einem einen (C₂-C₈)-Alkanoylrest liefernden Mittel acyliert;

worauf man erwünschtenfalls eine erhaltene saure Verbindung der in Anspruch 1 definierten Formel I in ein pharmazeutisch verwendbares Salz mit einer Base überführt.

9. Arzneimittel, insbesondere zur Bekämpfung oder Verhütung von Ulcus ventriculi und/oder duodeni, enthaltend eine Verbindung gemäss einem der Ansprüche 1-6 oder 10-Hydroxy-3,7-11-trimethyl-2,6,11-dodecatriensäure oder 3,7,11-Trimethyl-2,6,11-dodecatrien-1,10-diol und ein therapeutisch inertes Excipiens.

10. Verwendung von Verbindungen gemäss einem der Ansprüche 1-6 sowie von 10-Hydroxy-3,7,11-trimethyl-2,6,11-dodecatriensäure und von 3,7,11-trimethyl-2,6,11-dodecatrien-1,10-diol zur Herstellung von Arzneimitteln gegen Ulcus ventriculi und/oder duodeni.

## Patentansprüche (Patentansprüche für folgende(n) Vertragsstaat(en): GR AT; ES)

1. Verfahren zur Herstellung von Verbindungen der allgemeinen Formel 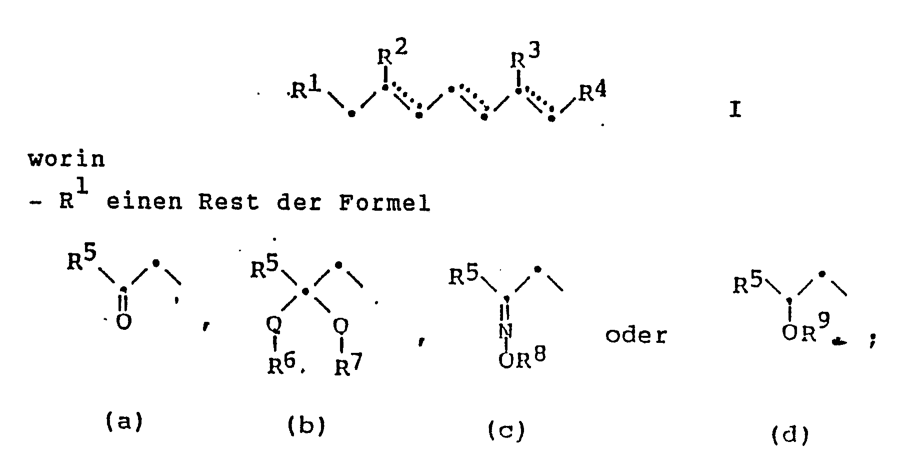 - eines von R² und R³ (C₁-C₈)-Alkyl und das andere Wasserstoff oder (C₁-C₈)-Alkyl;
- R⁴ einen Rest der Formel 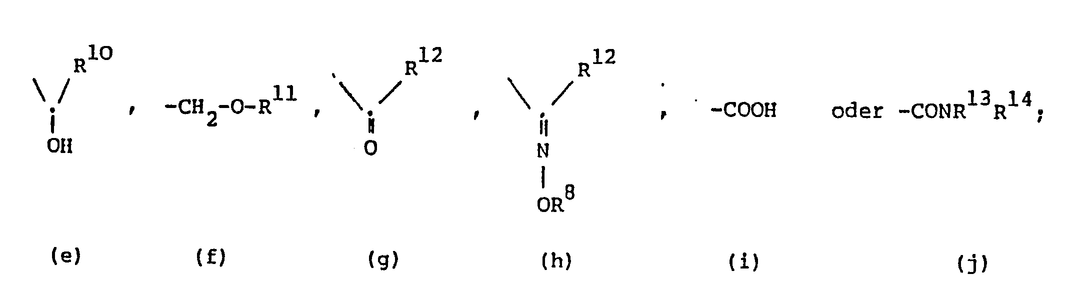 -R⁵ (C₂-C₈)-Alkyl, nicht jedoch Isopropyl, (C₃-C₈)-Cycloalkyl, (C₂-C₈)-Alkenyl, (C₂-C₈)-Alkinyl, Tri-(C₁-C₈)-alkyl-silyläthinyl oder, falls R¹ einen Rest der Formel (a) bedeutet, auch einen Rest der Formel 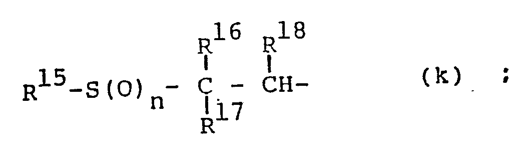 - R⁶ und R⁷ je (C₁-C₈)-Alkyl oder zusammen eine gegebenenfalls durch eine oder zwei (C₁-C₈)-Alkyl-, (C₁-C₈)-Alkoxy-(C₁-C₈)-alkyl- oder (C₁-C₈)-Alkoxycarbonylgruppen substituierte Di- oder Trimethylengruppe;
- R⁸ Wasserstoff oder (C₁-C₈)-Alkyl;
- R⁹ Wasserstoff oder (C₂-C₈)-Alkanoyl;
- R¹⁰ (C₁-C₈)-Alkyl;
- R¹¹ Wasserstoff, (C₁-C₈)-Alkyl, (C₂-C₈)-Alkanoyl oder einen Rest der Formel 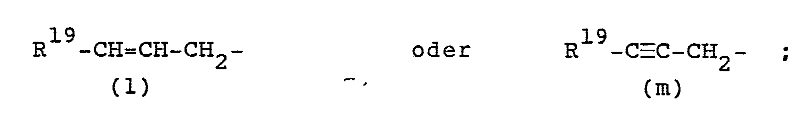 - R¹² Wasserstoff, (C₁-C₈)-Alkyl, (C₂-C₈)-Alkenyl oder (C₂-C₈)-Alkinyl;
- R¹³ und R¹⁴ je Wasserstoff oder (C₁-C₈)-Alkyl oder zusammen mit dem Stickstoffatom 1-Pyrrolidinyl, Piperidino oder Morpholino;
- R¹⁵ (C₁-C₈)-Alkyl, (C₂-C₈)-Alkanoyl, (C₁-C₈)-Alkoxycarbonyl-(C₁-C₈)-alkyl, Hydroxy-(C₂-C₈)-alkyl, (C₂-C₈)-Alkanoyloxy-(C₂-C₈)-alkyl, ((C₂-C₈)-Alkanoylamino-(C₂-C₈)-alkyl oder durch (C₂-C₈)-Alkanoylamino und (C₁-C₈)-Alkoxycarbonyl oder durch (C₂-C₈)-Alkanoyloxy und (C₁-C₈)-Alkoxycarbonyl disubstituiertes (C₂-C₈)-Alkyl;
- n die Zahl 0, 1 oder 2;
- R¹⁶, R¹⁷ und R¹⁸ je Wasserstoff oder (C₁-C₆)-Alkyl, enthaltend insgesamt höchstens 6 C-Atome;
- R¹⁹ Wasserstoff oder (C₁-C₅)-Alkyl;
- jede der punktierten Linien eine fakultative zusätzliche C-C-Bindung mit E- oder Z-Konfiguration bedeuten; und
- die in den Resten der Formeln (c) und (h) vorhandene Doppelbindung die E- oder Z-Konfiguration aufweist;
sowei pharmazeutisch verwendbare Salze von sauren Verbindungen der Formel I mit Basen,
mit Ausnahme von 10-Hydroxy-3,7,11-trimethyl-2,6,11-dodecatriensäure und 3,7,11-Trimethyl-2,6,11-dodecatrien-1,10-diol, dadurch gekennzeichnet, dass man
a) von einer Verbindung der allgemeinen Formel 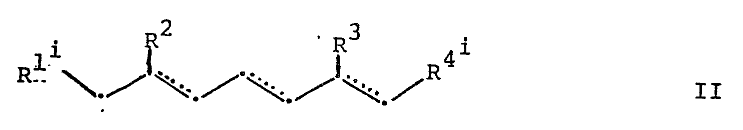 worin R¹ⁱ einen Rest der oben definierten Formel (a), (b) oder (c) oder einen Rest der Formel 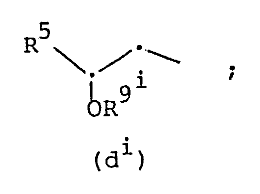 R⁴ⁱ einen Rest der in Anspruch 1 definierten Formel (g), (h), (i) oder (j) oder einen Rest der Formel 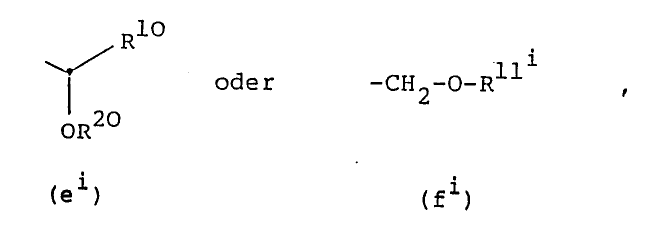 R⁹ⁱ Wasserstoff, (C₂-C₈)-Alkanoyl oder eine Schutzgruppe, R¹¹ⁱ Wasserstoff, (C₁-C₈)-Alkyl, (C₂-C₈)-Alkanoyl, eine Gruppe der oben definierten Formel (l) oder (m) oder eine Schutzgruppe und R²⁰ Wasserstoff oder eine Schutzgruppe bedeuten und R², R³, R⁵, R¹⁰ und die punktierten Linien die oben angegebene Bedeutung besitzen, wobei das Molekül mindestens eine Schutzgruppe enthält, die Schutzgruppe(n) abspaltet; oder
b) in einer Verbindung der allgemeinen Formel 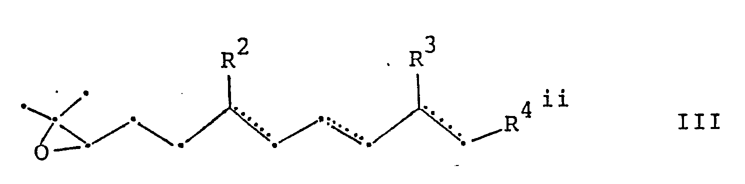 worin R⁴ⁱⁱ einen Rest der oben definierten Formeln (f), (h), (i) oder (j) bedeutet und R², R³ und die punktierten Linien die oben angegebene Bedeutung besitzen,
den Epoxidring öffnet; oder
c) einen Carbonsäureester der allgemeinen Formel 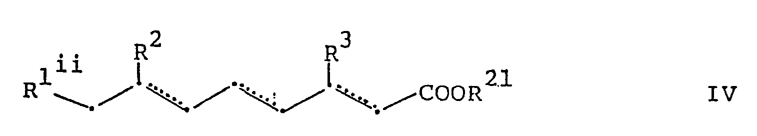 worin R¹ⁱⁱ einen Rest der oben definierten Formel (b) oder einen Rest der Formel 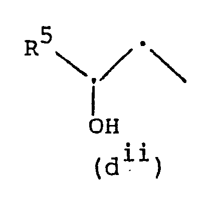 und R²¹ (C₁-C₈)-Alkyl bedeuten und R², R³, R⁵ und die punktierten Linien die oben angegebene Bedeutung besitzen,
zum entsprechenden primären Alkohol reduziert; oder
d) einen Carbonsäureester der allgemeinen Formel 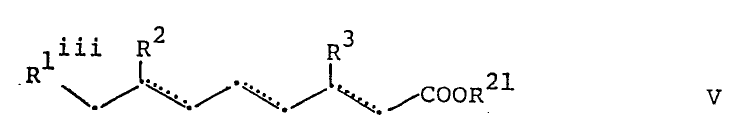 worin R¹ⁱⁱⁱ einen Rest der oben definierten Formel (b) oder (c) oder der obigen Formel (dⁱⁱ) oder der Formel 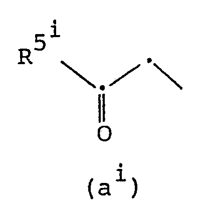 R⁵ⁱ (C₂-C₈)-Alkyl, nicht jedoch Isopropyl, (C₃-C₈)-Cycloalkyl, (C₂-C₈)-Alkenyl, (C₂-C₈)-Alkinyl oder Tri-(C₁-C₈)-alkyl-silyl-äthinyl bedeuten, R²¹ obige Bedeutung besitzt und R², R³ und die punktierten Linien die oben angegebene Bedeutung besitzen,
zur entsprechenden Carbonsäure hydrolysiert; oder
e) in einer Verbindung der oben definierten Formel I, worin R¹ einen Rest der oben definierten Formel (b), (c) oder (d) oder einen Rest der obigen Formel (aⁱ) bedeutet, wobei das Molekül mindestens eine an ein C-Atom gebundene Hydroxygruppe enthält, diese Hydroxygruppe(n) oder eine davon zu (einer) Oxo-gruppe(n) oxydiert; oder
f) eine Verbindung der oben definierten allgemeinen Formel I, worin R¹ einen Rest der oben definierten Formel (b) oder (d) und R⁴ eine Formylgruppe bedeuten, mit einem eine (C₁-C₈)-Alkylgruppe, eine (C₂-C₈)-Alkenylgruppe oder eine (C₂-C₈)-Alkinylgruppe liefernden Grignard-Reagens behandelt; oder
g) eine Verbindung der oben definierten Formel I, worin R¹ einen Rest der oben definierten Formel (b), (c) oder (d) oder einen Rest der obigen Formel (aⁱ) bedeutet, wobei das Molekül mindestens eine Oxogruppe enthält, mit einer Verbindung der allgemeinen Formel
H₂N-OR⁸ VI
worin R⁸ die in Anspruch 1 angegebene Bedeutung besitzt,
umsetzt; oder
h) eine Verbindung der oben definierten Formel I, worin R¹ einen Rest der Formel 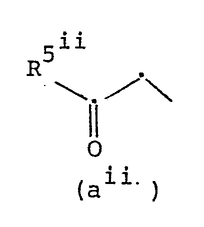 R⁴ einen Rest der oben definierten Formel (e), (h), (i) oder (j) oder einen Rest der obigen Formel (fⁱ) und R⁵ⁱⁱ (C₂-C₈)-Alk-1-enyl bedeuten,
mit einer Verbindung der allgemeinen Formel
R¹⁵-S-H VII
worin R¹⁵ die in Anspruch 1 angegebene Bedeutung besitzt,
umsetzt; oder
i) in einer Verbindung der in Anspruch 1 definierten Formel I, worin R¹ einen Rest der Formel 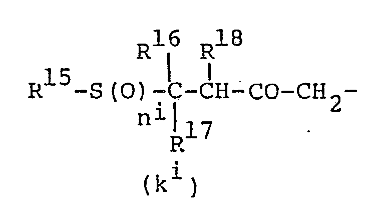 und nⁱ die Zahl 0 oder 1 bedeuten und R¹⁵, R¹⁶, R¹⁷ und R¹⁸ die oben angegebene Bedeutung besitzen,
die Mercaptogruppe zur Sulfinyl- oder Sulfonylgruppe bzw. die Sulfinyl- zur Sulfonylgruppe oxydiert; oder
j) in einer Verbindung der oben definierten Formel I, worin R¹ einen Rest der oben definierten Formel (a), (b) oder (d) oder einen Rest der Formel 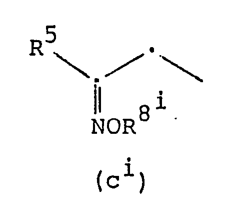 R⁴ einen Rest der oben definierten Formel (e), (f), (g), (i) oder (j) oder einen Rest der Formel 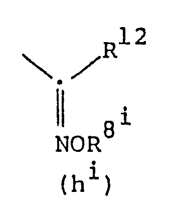 und R⁸ⁱ (C₁-C₈)-Alkyl bedeuten und R⁵ und R¹² die oben angegebene Bedeutung besitzen,
wobei das Molekül mindestens eine Hydroxygruppe enthält, diese Hydroxygruppe(n) mit einem einen (C₂-C₈)-Alkanoylrest liefernden Mittel acyliert;

worauf man erwünschtenfalls eine erhaltene saure Verbindung der in Anspruch 1 definierten Formel I in ein pharmazeutisch verwendbares Salz mit einer Base überführt.

2. Verbindungen gemäss Anspruch 1, worin R¹ einen Rest der Formel (a), (b), (c) oder (d), R² und R³ je (C₁-C₈)-Alkyl, R⁴ einen Rest der Formel (f), (g), (h), (i) oder (j), R⁵ (C₂-C₈)-Alkenyl, (C₂-C₈)-Alkinyl oder Tri-(C₁-C₈)-alkyl-silyl-äthinyl, R⁶ und R⁷ je (C₁-C₈)-Alkyl oder zusammen die Dimethylengruppe, R⁸ Wasserstoff oder (C₁-C₈)-Alkyl, R⁹ Wasserstoff, R¹¹ Wasserstoff oder (C₂-C₈)-Alkanoyl, R¹², R¹³ und R¹⁴ je Wasserstoff und zwei der punktierten Linien nicht-konjugierte zusätzliche C-C-Bindungen bedeuten.

3. Verfahren gemäss Anspruch 2, worin R² und R³ je Methyl, R⁵ Vinyl, 1-Methylvinyl, 1-Propenyl, 2-Methyl-1-propenyl, Aethinyl, 1-Propinyl oder Trimethylsilyl-äthinyl, R⁶ und R⁷ je Methyl oder Aethyl oder zusammen die Dimethylengruppe, R⁸ Wasserstoff oder Methyl und R¹¹ Wasserstoff oder Acetyl bedeuten.

4. Verfahren nach Anspruch 1 zur Herstellung einer Verbindung aus der Gruppe der folgenden:
(all-E)-12-Hydroxy-6,10-dimethyl-6,10-dodecadien-1-in-3-on,
(all-E)-12-Hydroxy-6,10-dimethyl-1,6,10-dodecatrien-3-on,
(all-E)-13-Hydroxy-7,11-dimethyl-7,11-tridecadien-2-in-4-on,
(all-E)-13-Hydroxy-7,11-dimethyl-2,7,11-tridecatrien-4-on und
(all-E)-10,10-Diäthoxy-3,7-dimethyl-2,6-dodecadien-11-in-1-ol,

5. Verfahren nach Anspruch 1 zur Herstellung einer Verbindung aus der Gruppe der folgenden:
(all-E)-12-Hydroxy-2,6,10-trimethyl-1,6,10-dodecatrien-3-ol,
(all-E)-10-Oxo-3,7,11-trimethyl-2,6,11-dodecatrienal,
(6Z,10Z)-12-Hydroxy-2,6,10-trimethyl-1,6,10-dodecatrien-3-on,
(all-E)-3,7-Dimethyl-9-[2-(1-methylvinyl)-1,3-dioxolan-2-yl]-2,6-nonadien-1-ol,
(E-E)-3,7,11-Trimethyl-10-oxo-2,6,11-dodecatrienamid,
(all-E)-13-Hydroxy-2,7,11-trimethyl-2,7,11-tridecatrien-4-on,
(6E,10Z)-12-Hydroxy-2,6,10-trimethyl-1,6,10-dodecatrien-3-on und
(6Z,10E)-12-Hydroxy-2,6,10-trimethyl-1,6,10-dodecatrien-3-on.

6. Verfahren nach Anspruch 1 zur Herstellung einer Verbindung aus der Gruppe der folgenden:
(all-E)-10-Oxo-3,7,11-trimethyl-2,6,11-dodecatriensäure;
(all-E)-10,10-Dimethoxy-3,7,11-trimethyl-2,6,11-dodecatrien-1-ol;
(all-E)-10-Hydroxy-3,7,11-trimethyl-2,6,11-dodecatrienal;
(all-E)-12-Hydroxy-6,10-dimethyl-1-(trimethylsilyl)-6,10-dodecadien-1-in-3-on;
(2E,6E)-3,7,11-Trimethyl-10-oxo-2,6,11-dodecatrienal-(E/Z) -oxim;
(6E,10E)-12-Hydroxy-6,10-dimethyl-6,10-dodecadien-1-in-3-on-(E/Z)-O-methyloxim; und
(2E,6E,11(E/Z))-3,7-Dimethyl-2,6,11-tridecatrien-1,10-diol.

7. Verfahren zur Herstellung von Arzneimitteln, insbesondere von solchen zur Bekämpfung oder Verhütung von Ulcus ventriculi und/oder duodeni, dadurch gekennzeichnet, dass man eine oder mehrere Verbindungen gemäss einem der Ansprüche 1-6 und/oder 10-Hydroxy-3,7,11-trimethyl-2,6,11-dodecatriensäure und/oder 3,7,11-Trimethyl-2,6,11-dodecatrien-1,10-diol sowie erwünschtenfalls einen oder mehrere andere therapeutisch wertvolle Stoffe zusammen mit einem oder mehreren therapeuztisch inerten Excipientien in eine galenische Darreichungsform bringt.

8. Verwendung von Verbindungen gemäss einem der Ansprüche 1-6 sowie von 10-Hydroxy-3,7,11-trimethyl-2,6,11-dodecatriensäure und von 3,7,11-Trimethyl-2,6,11-dodecatrien-1,10-diol zur Herstellung von Arzneimitteln gegen Ulcus ventriculi und/oder duodeni.
